(19) Europäisches Patentamt
European Patent Office
Office européen des brevets

(11) EP 1 608 687 B1

(12) **FASCICULE DE BREVET EUROPEEN**

(45) Date de publication et mention de la délivrance du brevet:
**20.01.2010 Bulletin 2010/03**

(21) Numéro de dépôt: **04742303.3**

(22) Date de dépôt: **22.03.2004**

(51) Int Cl.:
***C08B 37/16*** *(2006.01)* ***A61K 47/48*** *(2006.01)*
***A61K 31/724*** *(2006.01)*

(86) Numéro de dépôt international:
**PCT/FR2004/000691**

(87) Numéro de publication internationale:
**WO 2004/087768 (14.10.2004 Gazette 2004/42)**

(54) **NOUVEAUX DERIVES DE CYCLODEXTRINES, LEUR PROCEDE DE PREPARATION ET LEUR UTILISATION NOTAMMENT POUR LA SOLUBILISATION DE SUBSTANCES PHARMACOLOGIQUEMENT ACTIVES**

NEUE CYCLODEXTRINDERIVATE, HERSTELLUNGSVERFAHREN DAFÜR UND VERWENDUNG DAVON ZUR SOLUBILISIERUNG VON PHARMAKOLOGISCH WIRKSAMEN SUBSTANZEN

NOVEL CYCLODEXTRIN DERIVATIVES, METHOD FOR THE PREPARATION THEREOF AND USE THEREOF FOR THE SOLUBILIZATION OF PHARMACOLOGICALLY ACTIVE SUBSTANCES

(84) Etats contractants désignés:
**AT BE BG CH CY CZ DE DK EE ES FI FR GB GR HU IE IT LI LU MC NL PL PT RO SE SI SK TR**

(30) Priorité: **28.03.2003 FR 0303899**

(43) Date de publication de la demande:
**28.12.2005 Bulletin 2005/52**

(73) Titulaires:
- **Centre National de La Recherche Scientifique-CNRS**
  **75794 Paris Cedex 16 (FR)**
- **UNIVERSITE JOSEPH FOURIER - Grenoble 1**
  **38400 St. Martin d'Hères (FR)**
- **CONSEJO SUPERIOR DE INVESTIGACIONES CIENTIFICAS**
  **28006 Madrid (ES)**
- **UNIVERSIDAD DE SEVILLA**
  **41013 Sevilla (ES)**

(72) Inventeurs:
- **DEFAYE, Jacques**
  **F-38330 Saint-Ismier (FR)**
- **ORTIZ-MELLET, Carmen**
  **E-41011 Seville (ES)**
- **GARCIA-FERNANDEZ, José Manuel**
  **E-41011 Seville (ES)**
- **GOMEZ-GARCIA, Maria**
  **41003 Sevilla (ES)**
- **CHMURSKI, Kazimierz**
  **P-01-822 Varsovie (PL)**
- **YU, Jian-Xin**
  **Dallas, TX 75205-2292 (US)**

(74) Mandataire: **Grosset-Fournier, Chantal Catherine**
**Grosset-Fournier & Demachy**
**54, Rue Saint-Lazare**
**75009 Paris (FR)**

(56) Documents cités:
**EP-A- 0 403 366 WO-A-95/19994**
**WO-A-97/33919 WO-A-99/45032**

- **NAGATA Y. ET AL.: "pH-Responsive Guest Binding of Polypeptide Containing a Cyclodextrin at the Terminal" BULL. CHEM. SOC. JPN, vol. 67, 1994, pages 495-499, XP0001161089**
- **NOBUYOSHI AOKI ET AL.: "Gas chromatographic-mass spectrometric study of reactions of halodeoxycelluloses with thiols in aqueous solutions" CARBOHYDRATE POLYMERS, vol. 27, 1995, pages 13-21, XP004034456 GREAT BRITAIN**
- **JUAN J. GARCIA-LOPEZ ET AL.: "Synthesis of cluster N-Glycosides based on a beta-cyclodextrin core" CHEM. EUR. JOURNAL, vol. 5, no. 6, 1999, pages 1775-1784, XP000828759 WEINHEIM**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne de nouveaux dérivés de cyclodextrines, ainsi que leur procédé de préparation. La présente invention concerne également l'utilisation de ces nouveaux dérivés pour la solubilisation de substances pharmacologiquement actives dans un milieu aqueux.

Les cyclodextrines, ou cyclomaltooligosaccharides, sont des oligosaccharides cycliques qui sont connus pour leur aptitude à inclure dans leur cavité des molécules diverses, de taille adaptée à celle de la structure hôte. Le caractère généralement polaire de ces associations conduit à inclure préférentiellement des structures moléculaires de type hydrophobe, permettant notamment la solubilisation dans l'eau et les milieux biologiques de composés peu ou pas solubles dans ces milieux et éventuellement d'améliorer leur stabilisation. Ces propriétés sont actuellement utilisées en particulier pour le transport de médicaments.

La solubilité relativement faible dans l'eau des cyclodextrines, et notamment de la plus accessible d'entre elles sur le plan économique, la β-cyclodextrine (18 g/l, soit 15 mmol/l, à 25 °C) limite cependant leur utilisation dans ce but. D'un autre côté, les cyclodextrines ne possédant pas de capacité de reconnaissance vis-à-vis de récepteurs biologiques dans l'organisme, ces entités ne peuvent pas être utilisées pour le ciblage et la vectorisation de principes actifs.

Pour remédier à cet état de fait, les cyclodextrines ont été modifiées chimiquement pour améliorer leur solubilité dans l'eau d'une part et, d'autre part, pour incorporer dans leur structure des signaux de reconnaissance cellulaire. Ainsi, les demandes internationales WO 95/19994, WO 95/21870 et WO 97/33919 et la demande de brevet européen EP 0 403 366 décrivent des dérivés de cyclodextrines dont une ou plusieurs fonctions alcool primaire sont substituées par des groupes monosaccharidiques
ou oligosaccharidiques via un atome d'oxygène ou de soufre ou bien via un groupement thiourée, ainsi que leur utilisation. Ces cyclodextrines ramifiées sont en particulier susceptibles de servir d'hôte pour le taxol et ses dérivés, en particulier le Taxotère®, qui sont des agents antitumoraux et antiparasitaires, comme il est décrit par P. Potier dans Chem. Soc. Rev., 21, 1992, pp. 113-119. On obtient ainsi des complexes d'inclusion, ce qui permet de solubiliser dans l'eau ces agents antitumoraux. A titre d'exemple, la solubilité dans l'eau du Taxotère® qui est 0,004 g/l, peut être augmentée jusqu'à 6,5 g/L par addition de $6^{I}$-S-α-maltosyl-$6^{I}$-thiocyclomaltoheptaose à sa suspension aqueuse, comme cela est décrit dans le document WO 95/19994.

Le document EP-A-0 605 753 décrit des complexes d'inclusion du taxol utilisant des cyclodextrines ramifiées telles que les maltosyl-cyclodextrines, pour augmenter la solubilité de ce composé dans l'eau.

Des dérivés de cyclodextrines comportant un ou plusieurs substituants glycosyle ou maltosyle liés à la cyclodextrine par un atome de soufre sont également décrits par V. Lainé et al. dans J. Chem. Soc., Perkin Trans., 2, 1995, pp. 1479-1487. Ces dérivés ont été utilisés pour solubiliser un antiinflammatoire tel que la prédnisolone.

Le document WO 97/33919 décrit les procédés de préparation des thiouréido-cyclodextrines par couplage des $6^{I}$-amino-$6^{I}$-désoxycyclodextrines ou encore des dérivés peraminés correspondants avec des isothiocyanates d'alkyle ou des mono- ou oligosacharides.

L'incorporation de substituants glucidiques sur les cyclodextrines conduit à des dérivés dotés d'une solubilité dans l'eau beaucoup plus élevée si on la compare à la cyclodextrine de départ. En même temps, cette approche permet de conférer à la cyclodextrine une affinité particulière pour certains sites biologiques, car les substituants glucidiques sont bien connus comme marqueurs de reconnaissance cellulaire. Ainsi, ce type de modification de la cyclodextrine peut permettre le ciblage et la vectorisation d'une substance active incluse dans la cyclodextrine.

L'affinité d'un marqueur glucidique pour un récepteur spécifique de membrane cellulaire (lectine) est en règle générale faible. Pour atteindre des affinités utiles pour le ciblage et la vectorisation, il faut envisager une présentation multiple et simultanée du ligand. Dans le cas de dérivés des cyclodextrines monosubstitués en position alcool primaire (c'est-à-dire des cyclodextrines dans lesquelles l'un des groupes OH de l'alcool primaire est substitué), ce problème peut être partiellement résolu par l'incorporation de structures glycérophtaliques, comme décrit par I. Baussanne et al. dans Chem. Commun, 2000, pp. 1489-1490. Cependant, le procédé de préparation de tels composés est compliqué.

Par ailleurs, les résultats récents décrits par I. Baussanne et al. dans ChemBioChem 2001, pp. 777-783 montrent que les dérivés de la β-cyclodextrine comportant des substituants de type glycosylthiouréido, obtenues à partir de la per-(C-6)-amine correspondante, ne montrent pas une affinité suffisante vis-à-vis des lectines complémentaires.

A ce jour, il n'existe aucun dérivé de cyclodextrine, mono ou polysubstitué, obtenu par un procédé simple, permettant d'augmenter la solubilisation de substances pharmacologiquement actives et présentant également une affinité suffisante vis-à-vis des lectines complémentaires.

Un des buts de la présente invention est de fournir de nouveaux dérivés de cyclodextrines, polysubstitués mais également monosubstitués, en position alcool primaire, présentant non seulement un intérêt pour la solubilisation des substances actives, en particulier des antitumoraux de la famille du taxol comme le Taxotere®, mais également une forte affinité vis à vis de récepteurs membranaires spécifiques, ce qui permet d'envisager par leur intermédiaire un transport efficace et sélectif de la substance active vers des organes cibles.

Un des buts de l'invention consiste à fournir un procédé de préparation simple à mettre en oeuvre, et permettant

d'obtenir de nouveaux dérivés de cyclodextrines avec un bon rendement de l'ordre d'au moins 50%, et de préférence 70%, sans avoir à effectuer de purifications longues et compliquées.

La présente invention concerne l'utilisation d'un procédé comprenant une étape de réaction d'un composé sélectivement ou totalement halogéné en position alcool primaire, de formule (VII) suivante :

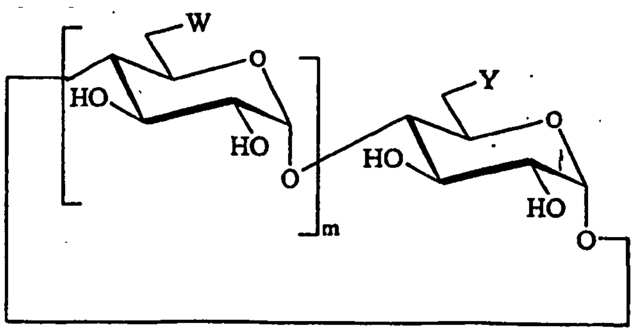

(VII)

m représentant un nombre entier égal à 5, 6 ou 7,
W représentant un groupe OH ou un groupe Y, les groupes W étant tous identiques,
et Y représentant un atome d'halogène choisi dans le groupe constitué du chlore, du brome, de l'iode, et étant de préférence le brome ou l'iode,
avec un ω-aminoalcanethiol de formule (VIII) suivante :

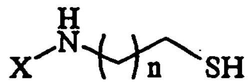

(VIII)

ledit ω-aminoalcanethiol étant éventuellement N-alkylé,
ou le sel correspondant de formule (VIII-a) suivante :

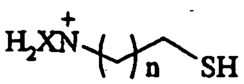

(VIII-a)

ou un sel de tétraalkylammonium de formule (VIII-b) suivante :

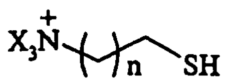

(VIII-b)

ledit sel étant associé à un contre-ion halogénure, de préférence l'ion chlorure,
n représentant un nombre entier compris de 1 à 6,
X représentant un atome d'hydrogène ou un groupe alkyle comprenant de 1 à 6 atomes de carbone, et étant notamment un groupe méthyle, éthyle, propyle ou butyle, X étant de préférence un atome d'hydrogène,
le composé de formule (VIII) étant de préférence la cystéamine de formule $H_2N-CH_2-CH_2-SH$,
afin d'obtenir un composé répondant aux formules suivantes (A-a) ou (A-b) :

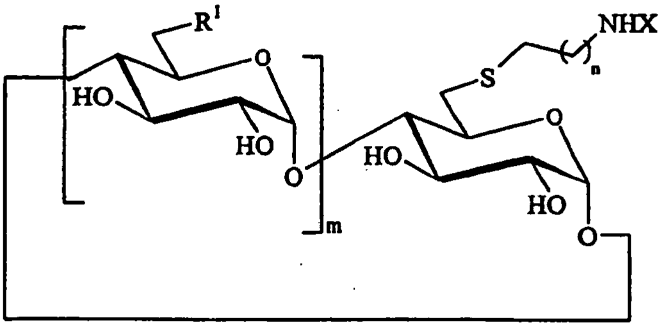

(A-a)

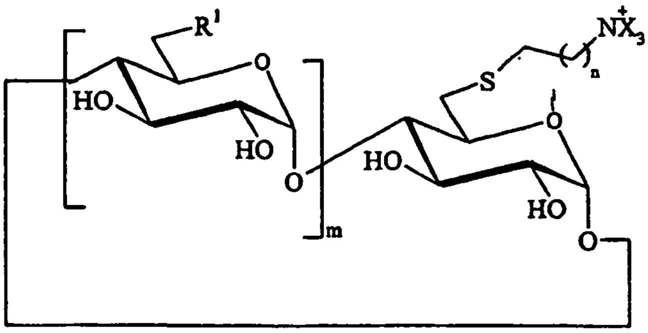

(A-b)

$R_1$ représentant soit un groupe OH soit un groupe $-S-CH_2-(CH_2)_n-Z$, tous les $R^1$ étant identiques, Z représentant un groupe NHX ou un groupe ammonium quaternaire de la forme $^+NX_3$, m, n et X étant tels que définis ci-dessus, pour la préparation de composés de formule (I) suivante :

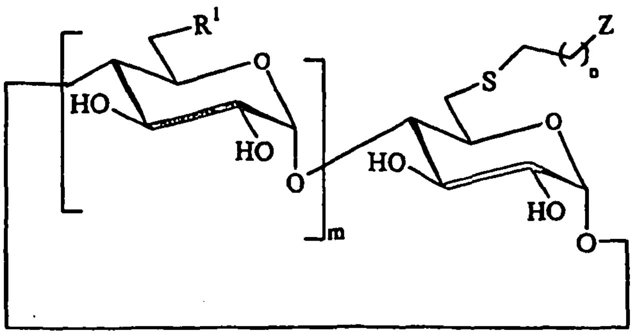

(I)

dans laquelle :

- m, n et $R_1$ sont tels que définis ci-dessus, et
- Z représente soit :
    * un groupe NHX,
    * un groupe ammonium quaternaire de la forme $^+NX_3$,
    * un groupe

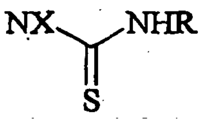

,

X étant tel que défini ci-dessus, et

R représentant un atome d'hydrogène, un substituant alkyle de 1 à 12 atomes de carbone linéaire ou ramifié, ou un groupe aromatique tel que le groupe phényle, benzyle ou naphtyle, ou des dérivés de ces groupements portant des substituants sur le cycle aromatique tels que les substituants méthyle, éthyle, chlore, brome, iode, nitro, hydroxyle, méthoxyle ou acétamido,

ou R représentant un élément de bioreconnaissance tel qu'un dérivé d'acide aminé, un peptide, un monosaccharide, un oligosaccharide, un élément de multiplication à plusieurs ramifications comportant des groupements glucidiques qui peuvent être identiques ou différents, ou une sonde de visualisation ou de détection fluorescente ou radioactive.

La présente invention concerne un composé répondant à la formule générale suivante :

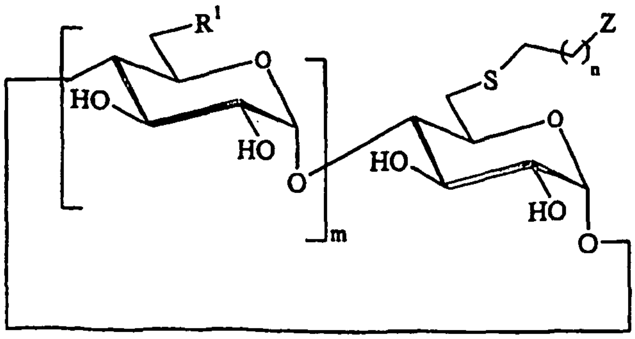

(I)

dans laquelle :

- n représente un nombre entier compris de 1 à 6 ;
- m représente un nombre entier égal à 5, 6 ou 7 ;
- $R^1$ représente soit un groupe OH soit un groupe $-S-CH_2-(CH_2)_n-Z$, tous les $R^1$ étant identiques ;
- Z représente soit :

  * un groupe NHX,
  * un groupe ammonium quaternaire de la forme $^+NX_3$,
  * un groupe

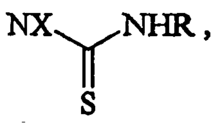

X représentant un atome d'hydrogène ou un groupe alkyle comprenant de 1 à 6 atomes de carbone, et étant notamment un groupe méthyle, éthyle, propyle ou butyle, et

R représentant un atome d'hydrogène, un substituant alkyle de 1 à 12 atomes de carbone linéaire ou ramifié, ou un groupe aromatique tel que le groupe phényle, benzyle ou naphtyle, ou des dérivés de ces groupements portant des substituants sur le cycle aromatique tels que les substituants méthyle, éthyle, chlore, brome, iode, nitro, hydroxyle, méthoxyle ou acétamido,

ou R représentant un élément de bioreconnaissance tel qu'un dérivé d'acide aminé, un peptide, un monosaccharide, un oligosaccharide, un élément de multiplication à plusieurs ramifications, lesquelles ramifications comportent des groupements glucidiques qui peuvent être identiques ou différents, ou une sonde de visualisation ou de détection fluorescente ou radioactive,

sous réserve que le composé dans lequel n =1, m = 6, Z = $NH_2$ et $R_1$ = OH soit exclu.

L'expression "élément de bioreconnaissance" désigne une structure moléculaire complémentaire d'un récepteur biologique, susceptible d'être reconnue par ce dernier et de conduire à une réponse spécifique : induction et régulation de la biosynthèse d'une enzyme, inhibition de l'activité d'une enzyme par fixation sur son site actif, induction d'une

réponse immunitaire suite à une affection bactérienne, inhibition d'un processus inflammatoire par blocage du site actif d'une sélectine, etc.

L'expression "élément de multiplication à plusieurs ramifications" désigne notamment une chaîne carbonée ramifiée comprenant un carbone quaternaire tétrasubstitué comme les dérivés du tris(2-aminométhyl)méthylamine (TRIS) et du pentaérythritol.

L'expression "sonde de visualisation ou de détection fluorescente ou radioactive" désigne une structure moléculaire permettant la détection d'un système par une technique physicochimique, telle que la fluorescence ou la radioactivité. Parmi les sondes fluorescentes, on peut notamment citer les dérivés de la fluorescéine, du dansyle (5-(diméthylamino)-1-naphtalènesulfonyle) ou de la coumarine. Parmi les sondes radioactives, on peut citer les produits marqués par un isotope radioactif.

La formule (I) susmentionnée concerne à la fois les composés poly- et monosubstitués sur le cycle cyclodextrine. Les composés monosubstitués correspondent à la formule (I) dans laquelle $R^1$ représente OH et les composés polysubstitués correspondent à la formule (I) dans laquelle $R^1$ représente $-S-CH_2-(CH_2)_n-Z$.

Dans ces nouveaux dérivés, on a constaté que la présence d'un groupe espaceur du type cystéaminyle ou, plus généralement, d'un groupe espaceur dérivé d'un ω-aminoalcanethiol, entre la position alcool primaire de la cyclodextrine et le groupe Z, est intéressante, notamment d'une part pour augmenter la réactivité des groupements amine dans le cas des composés répondant à la formule (I), Z représentant NHX, et d'autre part pour garantir l'efficacité du phénomène de reconnaissance cellulaire dans le cas des dérivés répondant à la formule (I), Z représentant un groupe thiourée. Il est à noter par ailleurs que ce groupe espaceur est introduit d'une façon simple en utilisant la cystéamine commerciale ou l'ω-aminoalcanethiol homologue correspondant comme réactif, ce qui évite notamment l'étape de réduction nécessaire lorsque les groupements amine sont préparés à partir d'un précurseur de type azide comme c'est le cas dans les exemples décrits dans le document WO 97/33919 susmentionné.

Il faut noter que le produit de formule (I) dans lequel m est égal à 6, $R_1$ représente OH et Z représente $NH_2$ a été préparé précédemment par réaction du $6^I$-O-tosylcyclomaltoheptaose dans la *N,N*-diméthylformamide avec la cystéamine à température ambiante avec un rendement non précisé (Y. Nagata et al., Bull Chem. Soc. Jpn. 1994, 67, 495-499) ou encore par réaction de ce même tosylate avec le chlorhydrate de cystéamine dans un mélange eau-diméthylformamide à 60°C en présence d'hydrogénocarbonate de sodium avec un rendement de 43% (B. Ekberg et al., Carbohydr. Res., 1992, 192, 111-117). Dans le cadre de la présente invention, ce produit a été utilisé comme intermédiaire de synthèse pour l'obtention des thiouréidocystéaminyl cyclodextrines correspondantes. Il a été trouvé avantageux de le préparer par réaction d'un précurseur monohalogéné en C-6 de la β-cyclodextrine, de préférence le bromure, avec le chlorhydrate de cystéamine dans la N,N-diméthylformamide en présence de triéthylamine (rendement 85%). éventuellement, le groupement aminé de la cystéamine ou de l'ω-aminoalcanethiol peut porter un substituant alkyle tel qu'un groupe méthyle, éthyle, propyle ou butyle. Ce groupement espaceur préfonctionalisé permet d'associer la cyclodextrine à un motif hydrophile et de reconnaissance cellulaire tel qu'un dérivé glucidique, ou encore un acide aminé ou un peptide, par des liaisons de type thiourée, amide et thioéther qui sont très stables et donnent lieu à des structures bien définies. La liaison thiourée est créée dans une dernière étape et permet de coupler la cyclodextrine à de nombreux substituants, en particulier des substituants comportant un élément de multiplication à plusieurs ramifications, lesdites ramifications portant divers motifs glucidiques ou même une sonde de visualisation ou de détection fluorescente ou radioactive.

Les cystéaminyl-cyclodextrines répondant à la formule (I) donnée ci-dessus dans laquelle Z représente un groupement amine du type NHX (X = H ou substituant alkyle) peuvent être isolées sous forme de sel d'ammonium (cas où Z représente $^+NX_3$) ou de base libre (cas où Z représente NHX). Dans le cas du sel, le contre-ion est un anion monovalent, en particulier un halogénure tel que le chlorure, le bromure ou l'iodure. C'est en particulier le cas pour les cystéaminyl-cyclodextrines répondant à la formule (I) dans laquelle Z représente un groupement, ammonium quaternaire, chargé positivement, du type $^+NX_3$ (X = substituant alkyle). Les composés de formule (I), dans laquelle Z représente NHX, peuvent être utilisés comme précurseurs dans la préparation des thiouréidocystéaminyl-cyclodextrines, notamment des dérivés hyperramifiés. Lorsque Z dans la formule (I) représente un groupement $NH_2$, les thiourées obtenues sont N,N'-disubstituées, alors que lorsque Z représente un groupement NHX, X représentant un substituant alkyle, tel que méthyle, éthyle, propyle ou butyle, les thiourées obtenues sont *N,N,N'*-trisubstituées.

Dans le cas des thiouréidocystéaminyl-cyclodextrines de formule (I), dans laquelle Z représente un groupe thiourée, les substituants R peuvent être de divers types. Ainsi, R peut représenter un atome d'hydrogène, un substituant allyle de 1 à 12 atomes de carbone linéaire ou ramifié, ou un groupe aromatique tel que phényle, benzyle naphtyle ou des dérivés de ces groupements portant des substituants sur le cycle aromatique, lesdits substituants étant tels que définis précédemment. R peut représenter aussi, en particulier, des groupes dérivés d'acides aminés, de peptides, de monosaccharides ou d'oligosaccharides éventuellement substitués. A titre d'exemple de groupes dérivés de monosaccharides, on peut citer ceux dérivés du glucose, du mannose et du galactose, en configuration anomérique α ou β. Dans le cas où le groupe dérivé du monosaccharide est substitué, un ou plusieurs des groupes hydroxyle du monosaccharide peuvent être remplacés par des groupes alcoxy de 1 à 16 atomes de carbone, des groupes acyloxy comme le groupe

acétoxy, des groupes amine et amide. Les groupes dérivés d'oligosaccharides peuvent être les groupes maltosyle, maltotriosyle, lactosyle, ou encore des tri- ou tétrasaccharides marqueurs d'affinité cellulaire du type Lewis X ou sialyl Lewis X, ou encore des oligosaccharides dérivés de l'héparine. Ils peuvent également être substitués par des groupes alcoxy, acyloxy, des groupements aminés, sulfatés ou phosphatés.

Selon l'invention, R peut également représenter un groupe comportant un élément de multiplication ramifié, par exemple un groupe dérivé du tris(2-hydroxyméthyl)méthylamine (TRIS) ou du pentaérythritol, comportant dans les ramifications des groupes dérivés de mono- ou d'oligosaccharides qui peuvent être identiques ou différents. A titre d'exemples, on peut citer les groupes dérivés de mono-
ou oligosaccharides déjà cités dans le paragraphe précédent, qui peuvent également comporter des substituants oxygénés ou aminés. Ces groupes glucidiques peuvent être liés à l'élément de multiplication par une liaison oxygénée, soufrée ou aminée. Dans le cas où R comprend un élément de ramification, une des ramifications peut aussi porter une sonde de type fluorescent, en particulier un dérivé de la fluorescéine ou encore une sonde radioactive.

Selon un mode de réalisation avantageux, les composés de l'invention répondent à l'une des formules suivantes :

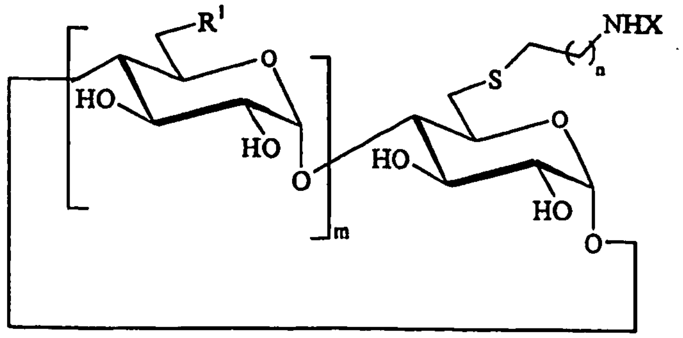

**(A-a)**

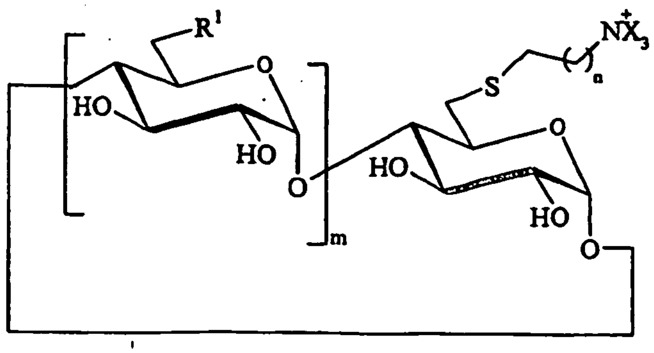

**(A-b)**

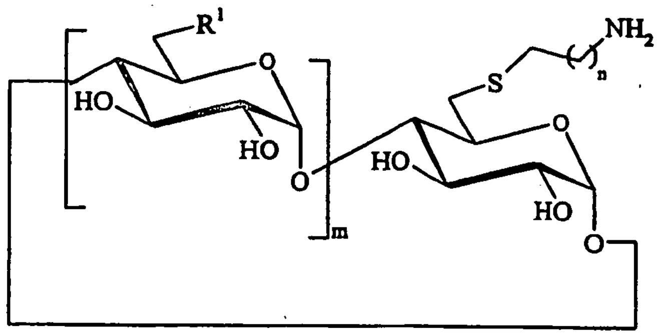

**(A-c)**

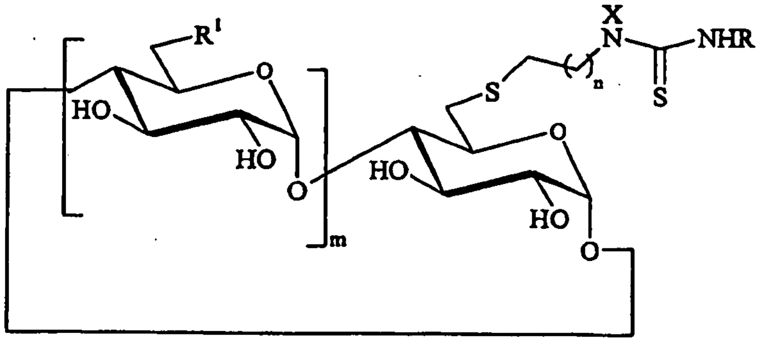

(B)

dans lesquelles m, n, $R^1$, X et R sont tels que définis précédemment.

**[0027]** , Un composé avantageux de la présente invention est caractérisé en ce que $R^1$ représente OH, et répond à la formule générale suivante :

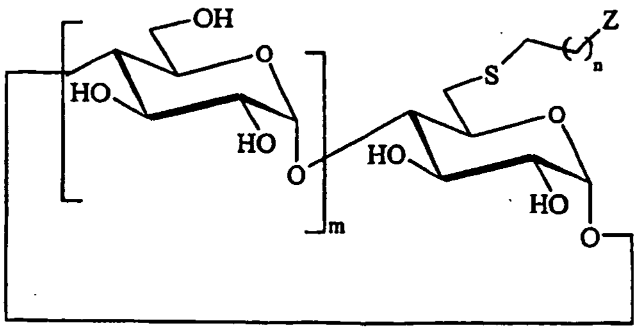

(I-a)

dans laquelle :

- m, n et Z sont tels que définis ci-dessus.

Les composés susmentionnés sont des composés monosubstitués sur le cycle cyclodextrine.

Dans ce type de composés, l'accès à la cavité de la cyclodextrine est moins encombré que dans le cas des composés de l'art antérieur, ce qui peut résulter en de meilleures propriétés de complexation pour certaines molécules invitées.

Un composé avantageux de la présente invention, répondant à la formule (I-a), est caractérisé en ce que Z représente un groupe NHX, X étant tel que défini ci-dessus, et étant notamment un atome d'hydrogène.

De tels composés répondent à la formule suivante :

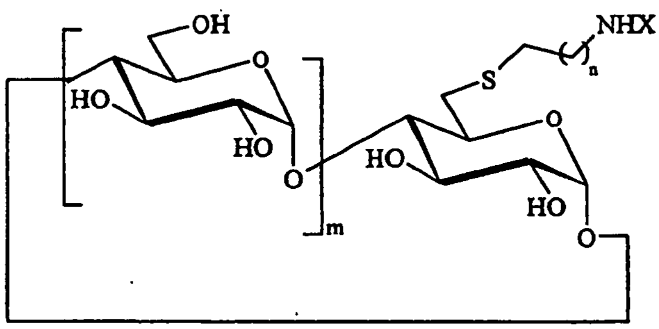

(I-f)

Les composés susmentionnés peuvent servir d'intermédiaire de réaction lorsque X représente un atome d'hydrogène, afin d'obtenir les composés de formule (I-a) dans laquelle Z représente un groupe thiourée.

Un composé avantageux de la présente invention, répondant à la formule (I-a), est caractérisé en ce que Z représente un groupe $NH_2$ ou un groupe $NX_3^+$ et répond à l'une des formules (I-f-bis) ou (I-g) suivantes :

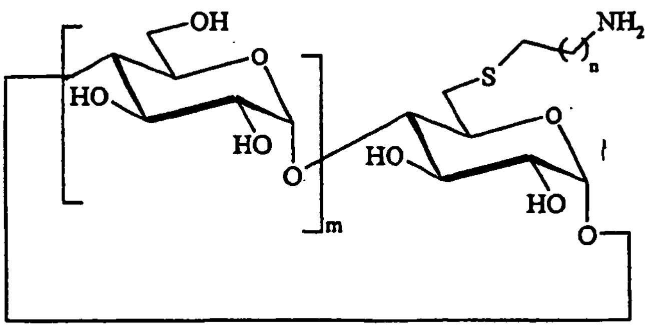

(I-f-bis)

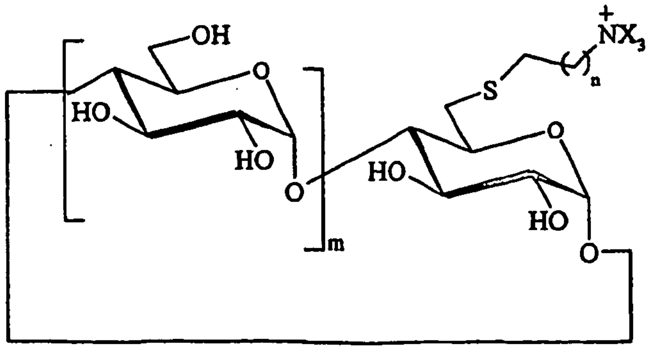

(I-g)

dans lesquelles m, n et X sont tels que définis précédemment.

Un composé avantageux selon la présente invention est un composé tel que défini ci-dessus, répondant à la formule (I-a) et caractérisé en ce que Z représente un groupe

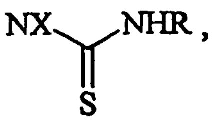

R étant tel que défini ci-dessus, et X étant tel que défini ci-dessus, et étant notamment un atome d'hydrogène.

De tels composés répondent à la formule suivante :

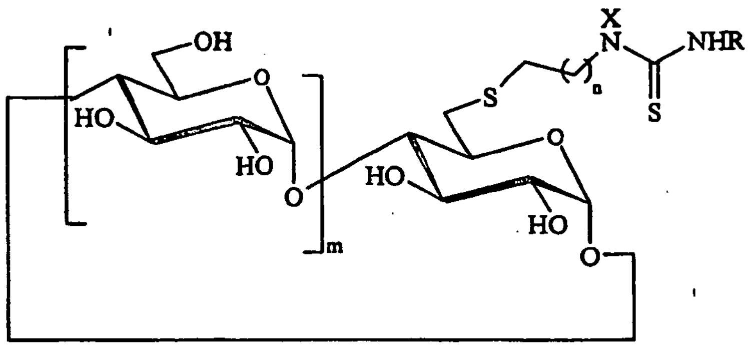

(I-h)

Un composé avantageux selon la présente invention est un composé de formule (I) tel que défini ci-dessus, caractérisé en ce que $R^1$ représente un groupe $-S-CH_2-(CH_2)_n-Z$, et répondant à la formule générale suivante (I-b) :

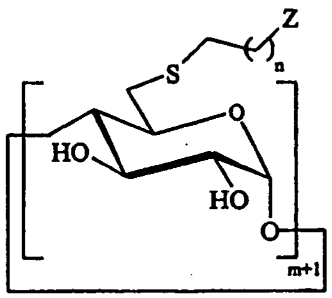

(I-b)

dans laquelle m, n et Z sont tels que définis ci-dessus.

Les composés susmentionnés sont des composés polysubstitués sur le cycle cyclodextrine.

Dans de telles structures, les éléments de bioreconnaissance incorporés sont multipliés par rapport aux dérivés monosubstitués, ce qui, dans le cas des composés de l'invention, peut se traduire par de meilleures affinités vis-à-vis des récepteurs biologiques. Pour les dérivés hyperramifiés, de nouvelles propriétés supramoléculaires (donc de transport) sont envisageables du fait de la possibilité d'extension de la cavité de la cyclodextrine par les substituants en C-6.

Un composé avantageux selon la présente invention est un composé tel que défini ci-dessus, de formule (I-b), répondant à la formule suivante :

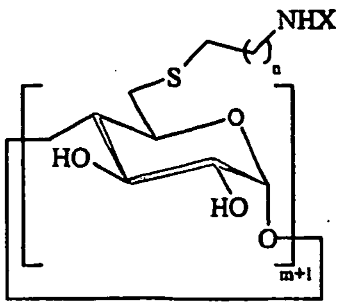

(I-c)

X, n et m étant tels que définis ci-dessus.

Les composés de formule (I-c) sont des composés dérivés de la cyclodextrine, nommés composés cystéaminylcyclodextrines, et peuvent servir d'intermédiaire de réaction, lorsque X représente un atome d'hydrogène, afin d'obtenir les composés de formule (I-b) dans laquelle Z représente un groupe thiourée.

Un composé avantageux selon la présente invention est un composé tel que défini ci-dessus, de formule (I-b), caractérisé en ce que X représente un atome d'hydrogène et en ce que n est égal à 1, et répondant à la formule suivante :

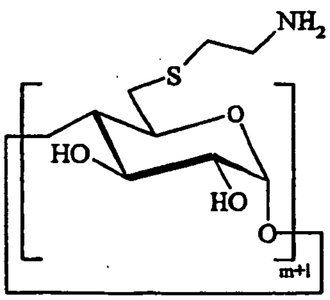

(I-d)

m étant tel que défini ci-dessus.

Un composé avantageux selon la présente invention est un composé tel que défini ci-dessus, de formule (I-b), répondant à la formule suivante :

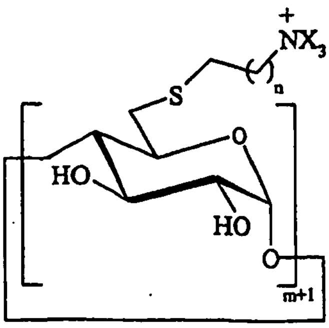

(I-e)

X, n et m étant tels que définis ci-dessus.

Un composé avantageux selon la présente invention est un composé tel que défini ci-dessus, répondant à la formule suivante (II) :

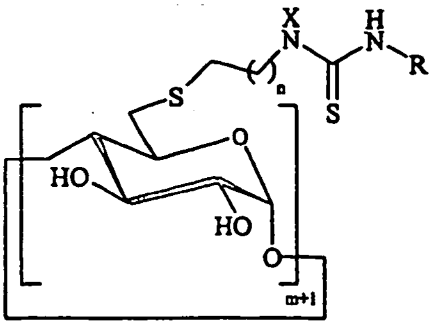

(II)

X, n, m et R étant tels que définis ci-dessus, et R étant identique pour chaque groupe

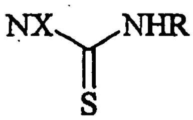

tel que défini ci-dessus.

Les composés de formule (II) sont des composés dérivés de la cyclodextrine, nommés composés thiouréidocystéaminylcyclodextrines.

Un composé avantageux selon la présente invention est un composé tel que défini ci-dessus, de formule (II), caractérisé en ce que X représente un atome d'hydrogène et en ce que n est égal à 1, et répondant à la formule suivante :

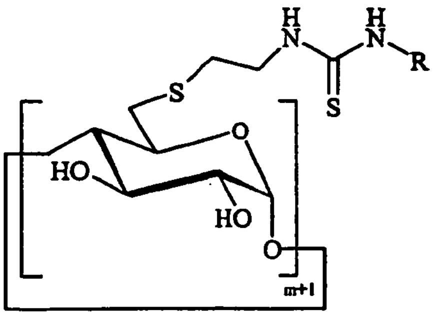

(II-a)

R et m étant tels que définis ci-dessus.

Un composé avantageux selon la présente invention est un composé tel que défini ci-dessus, caractérisé en ce que l'un au moins des groupes NHX tels que définis ci-dessus est protoné et associé à un anion monovalent choisi

notamment parmi l'ion chlorure, bromure ou iodure.

La présente invention concerne également un composé tel que défini ci-dessus, caractérisé en ce que n est égal à 1 et en ce que le groupe Z représente le groupe ammonium quaternaire $^+NX_3$, et en ce qu'il peut être associé à un anion monovalent choisi notamment parmi l'ion chlorure, bromure ou iodure, et répondant à la formule suivante :

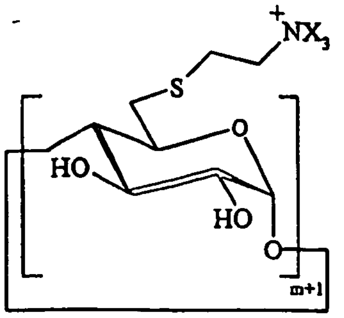

(I-e-bis)

Ces produits chargés positivement peuvent présenter des interactions électrostatiques favorables avec des molécules chargées négativement telles que des polynucléotides. De ce point de vue, on peut envisager une application de ces dérivés comme vecteurs pour le transfert de gènes. Par ailleurs, comme cela est décrit par J. Defaye et coll. (J. Incl. Phen. Mol. Recogn. Chem. 29 (1997) 57-63), on sait que la présence d'un groupement chargé positivement en C-6 des cyclodextrines décroît le caractère hémolytique de ces entités, et donc leur toxicité.

La présente invention concerne également un composé de formule (II-a) tel que défini ci-dessus, caractérisé en ce que le groupement R est choisi parmi les groupes suivants :

- le groupe α-D-mannopyranosyle, de formule suivante (III) :

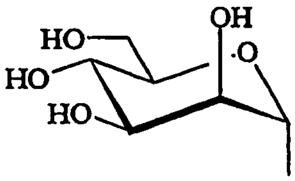

(III)

- le groupe β-lactosyle, de formule suivante (III-a) :

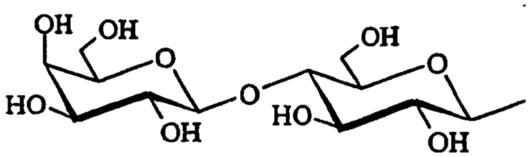

(III-a)

- le groupe dérivé du trisaccharide Lewis X ou du tétrasaccharide sialyl Lewis X, respectivement de formule suivante (III-b) et (III-c) :

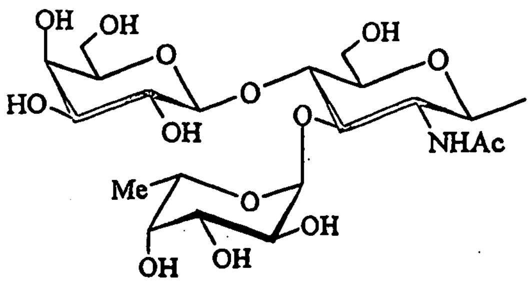

(III-b)

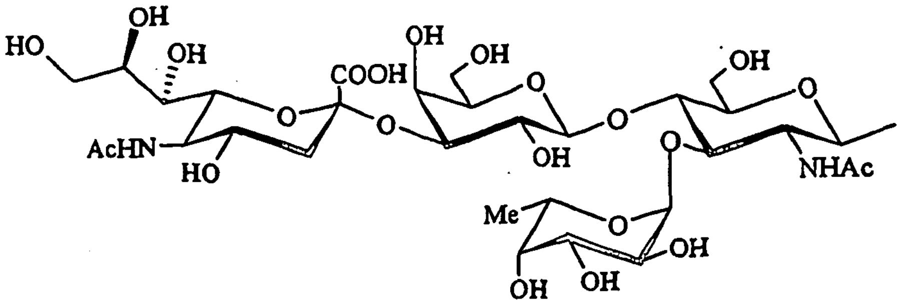

(III-c)

- un oligosaccharide dérivé de l'héparine, de formule suivante (III-d) :

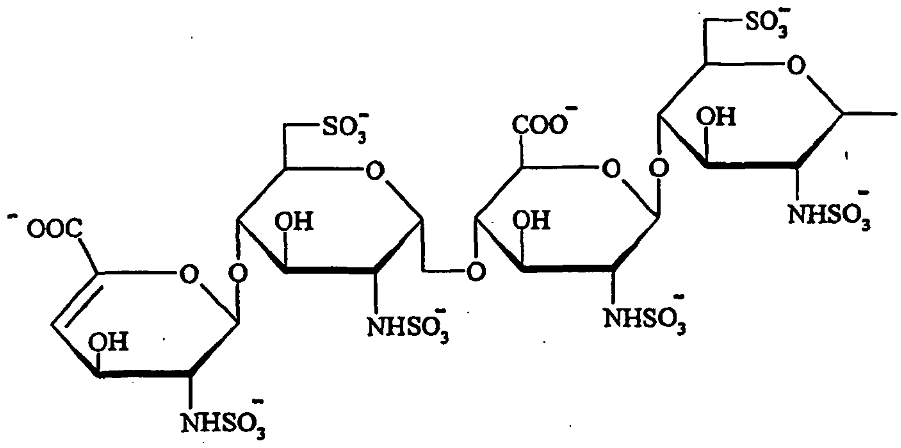

(III-d)

Ainsi, la présente invention concerne les composés répondant à l'une des formules suivantes :

- composé de formule (II-a) lorsque R représente le groupe α-D-mannopyranosyle de formule (III) :

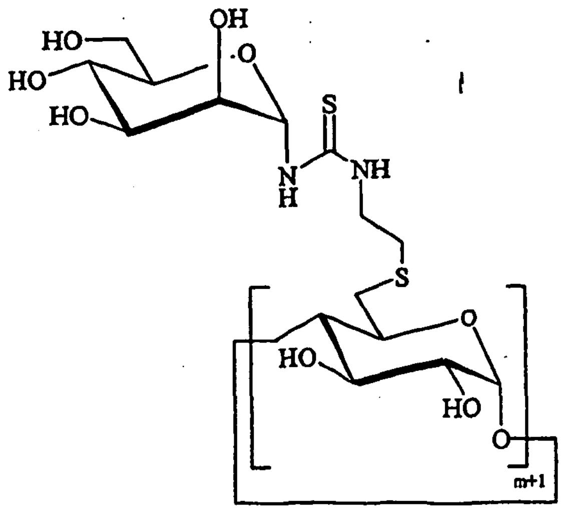

- composé de formule (II-a) lorsque R représente le groupe β-lactosyle de formule (III-a) :

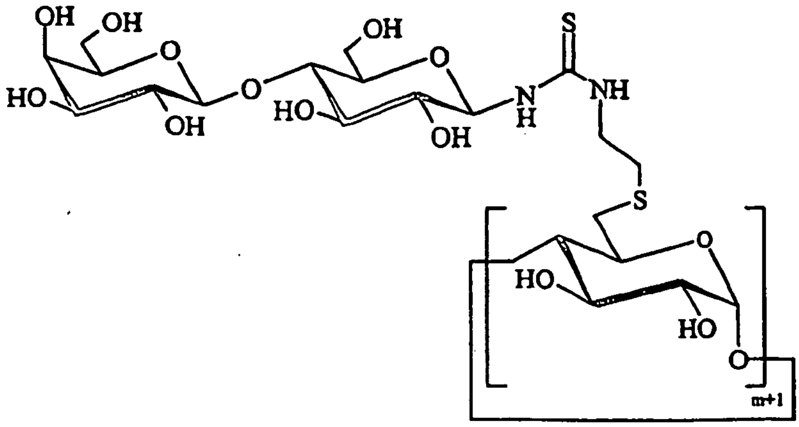

- composé de formule (II-a) lorsque R représente le groupe dérivé du trisaccharide Lewis X de formule (III-b) :

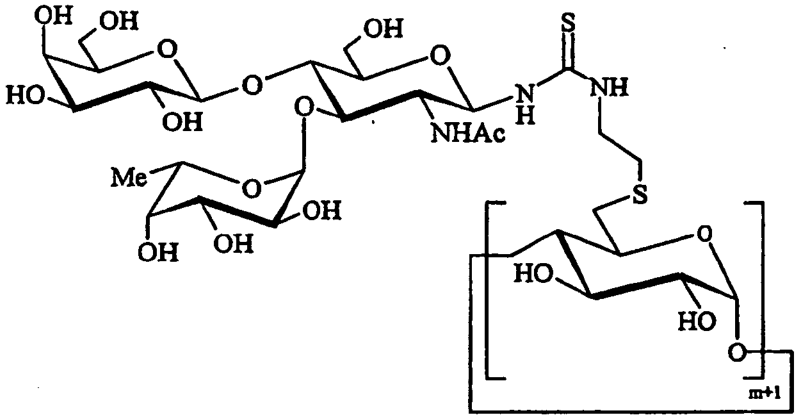

- composé de formule (II-a) lorsque R représente le groupe dérivé du tétrasaccharide sialyl Lewis X de formule (III-c) :

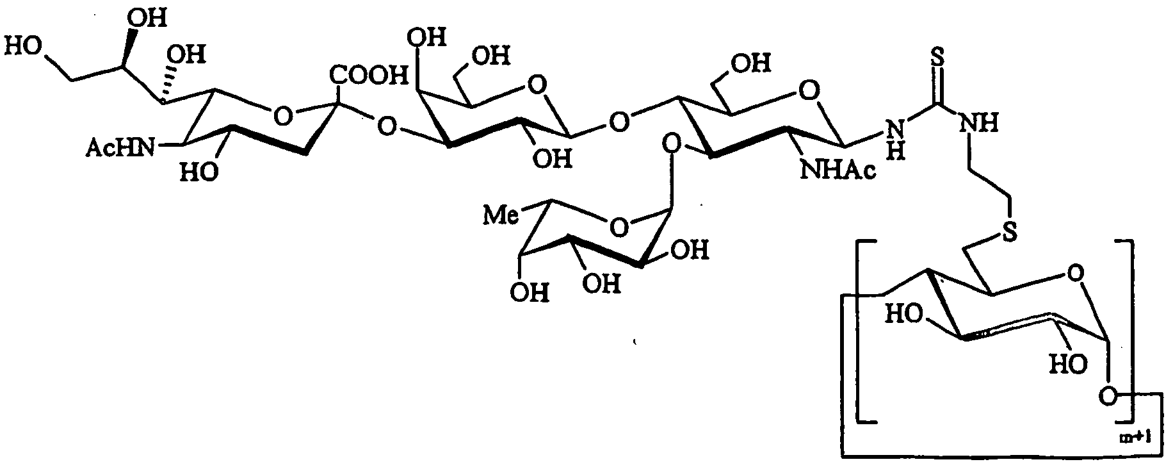

- composé de formule (II-a) lorsque R représente un oligosaccharide dérivé de l'héparine, de formule (III-d) :

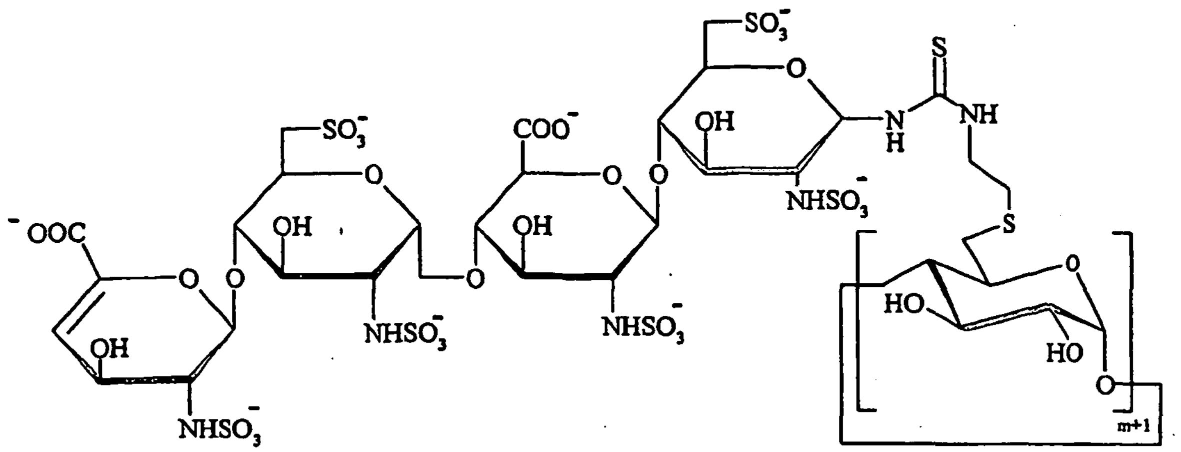

La présente invention concerne également un composé de formule (II-a) tel que défini ci-dessus, caractérisé en ce que :

R comporte un élément de ramification dérivé du tris(2-hydroxyméthyl)méthylamine, ou
R représente l'un des groupes suivants :

- le groupe tris(α-D-mannopyranosyloxyméthyl)méthyle, de formule suivante (IV) :

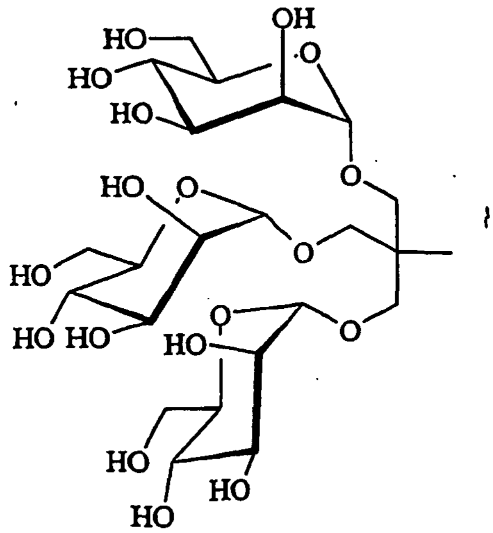

(IV)

- le-groupe tris(β-lactosyloxyméthyl)méthyle, de formule suivante (IV-a) :

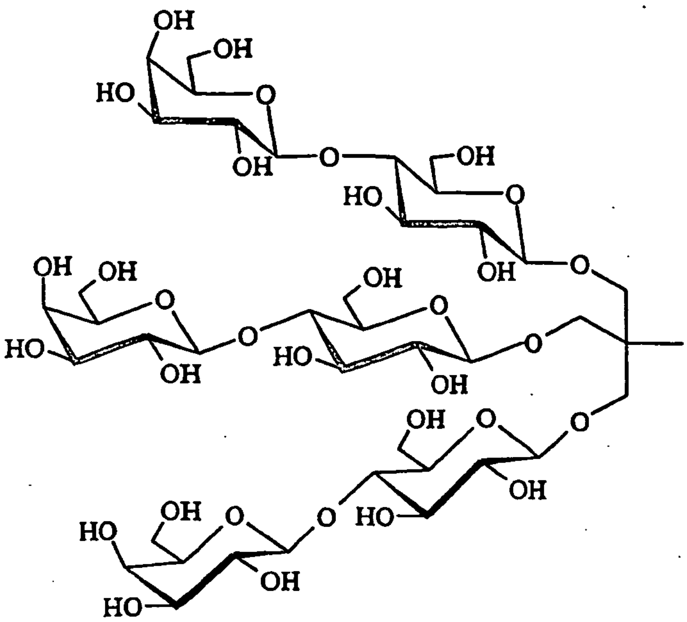

(IV-a)

La présente invention concerne également les composés répondant à l'une des formules suivantes :

- composé de formule (II-a) lorsque R représente le groupe tris(α-D-mannopyranosyloxyméthyl)méthyle, de formule (IV) :

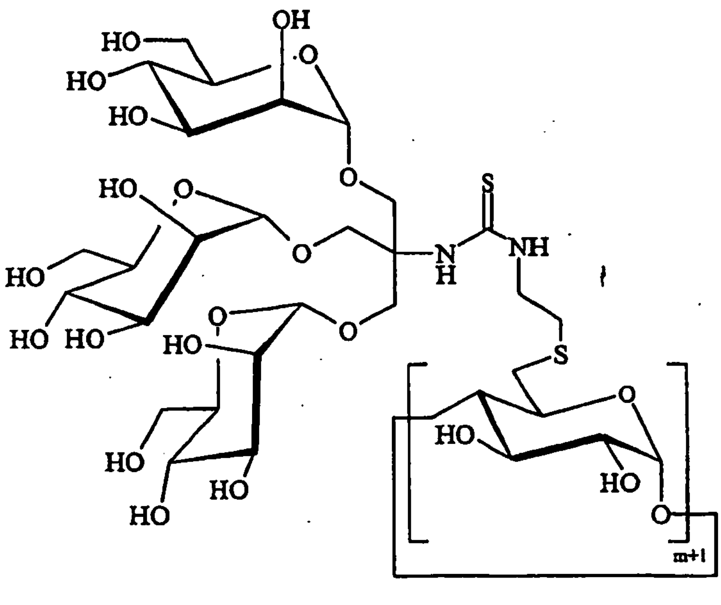

- composé de formule (II-a) lorsque R représente le groupe tris(β-lactosyloxyméthyl)méthyle, de formule (IV-a) :

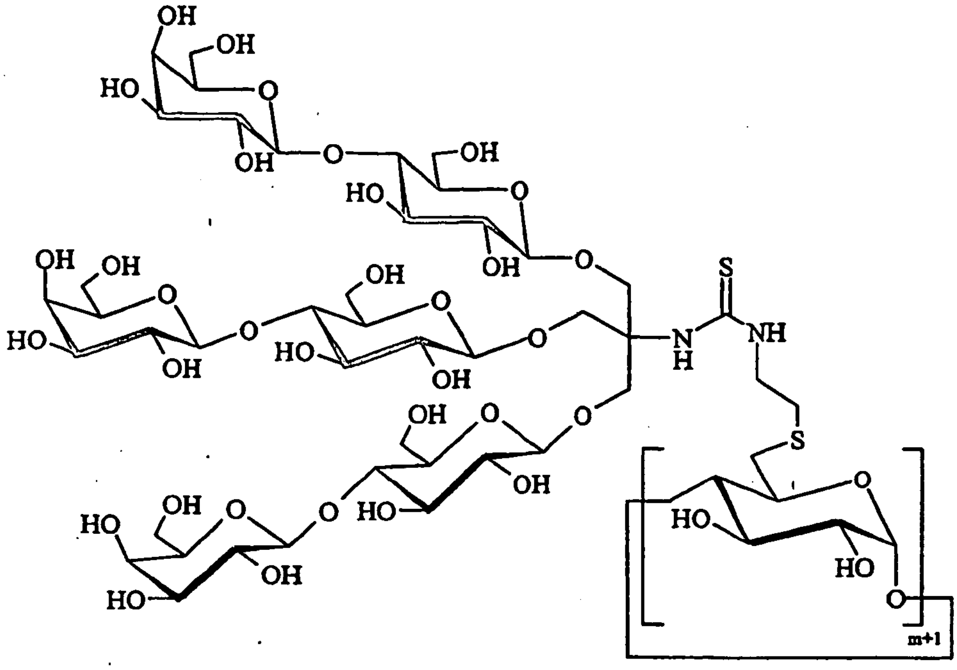

La présente invention concerne également un composé de formule (I-a) tel que défini ci-dessus, caractérisé en ce que R comporte un élément de ramification dérivé du pentaérythritol, ledit composé répondant à la formule suivante :

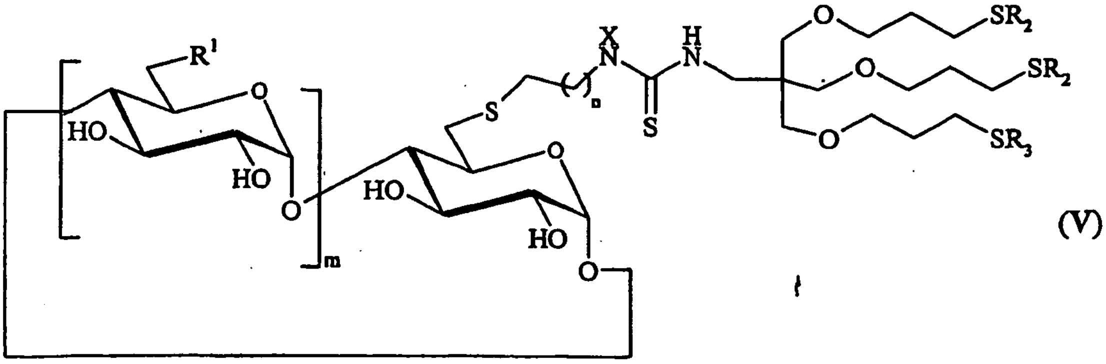

(V)

dans laquelle m, n, $R^1$ et X sont tels que définis ci-dessus, et
$R^2$ et $R^3$ représentent des dérivés glucidiques qui peuvent être différents ou identiques ou encore une sonde fluorescente ou radioactive.

Un composé avantageux selon la présente invention est un composé tel que défini ci-dessus, de formule (V), caractérisé en ce que $R^1$ représente OH.

Un tel composé répond à la formule suivante :

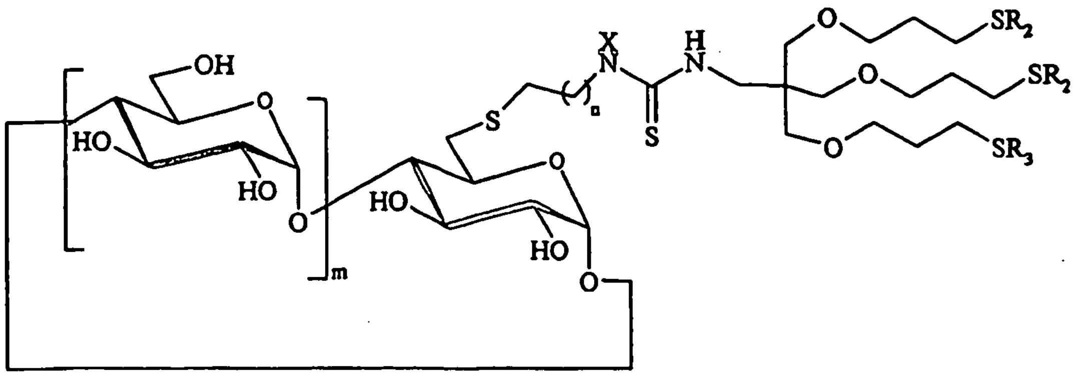

Les composés susmentionnés représentent des composés monosubstitués sur le cycle cyclodextrine.

Un composé avantageux selon la présente invention est un composé tel que défini ci-dessus, de formule (V), caractérisé en ce que $R^1$ représente le groupe de formule :

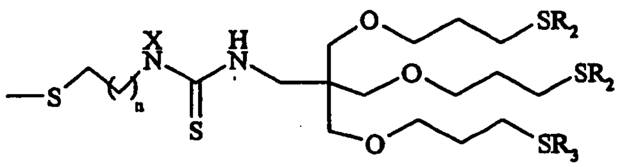

Un tel composé répond à la formule suivante :

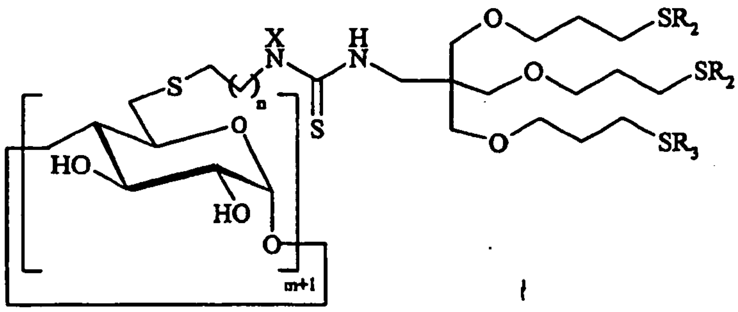

Les composés susmentionnés représentent des composés polysubstitués sur le cycle cyclodextrine.

La présente invention concerne également un composé tel que défini précédemment, de formule (V), caractérisé en ce que n est égal à 1, en ce que X représente un atome d'hydrogène et en ce que $R^2$ et $R^3$ représentent l'un des groupes suivants :

- le groupe α-D-mannopyranosyle, de formule (III), tel que défini ci-dessus, ou
- le groupe β-lactosyle, de formule (III-a), tel que défini ci-dessus, ou
- le groupe β-D-glucopyranosyle, de formule (VI) suivante:

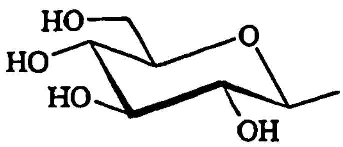

(VI)

$R^2$ et $R^3$ pouvant être identiques ou différents.

De tels composés répondent à l'une des formules suivantes :

a) Composés monosubstitués (cas où $R_1$ représente OH)

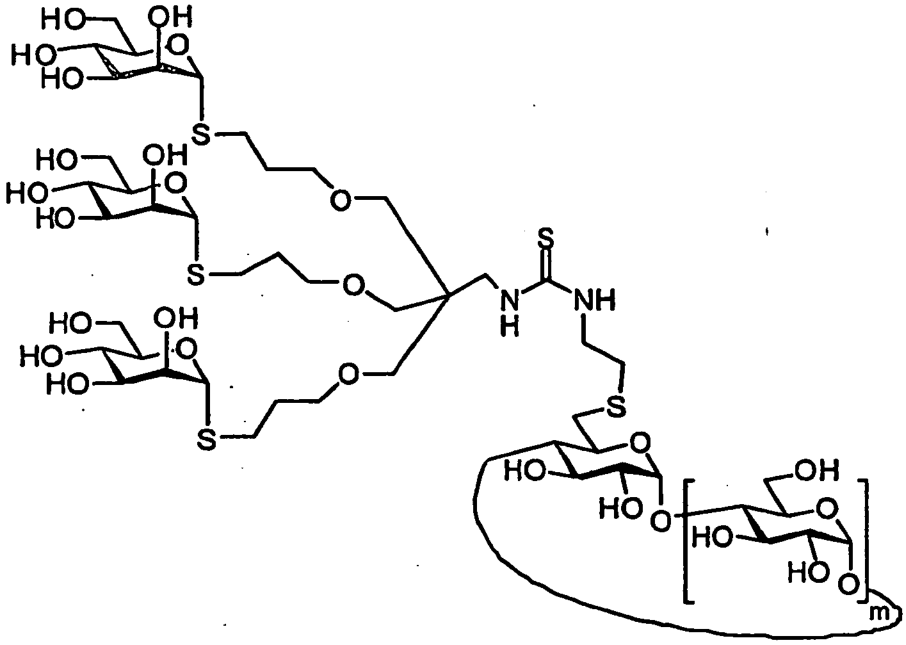

(V ; $R_2$ = $R_3$ = α-D-mannopyranosyle)

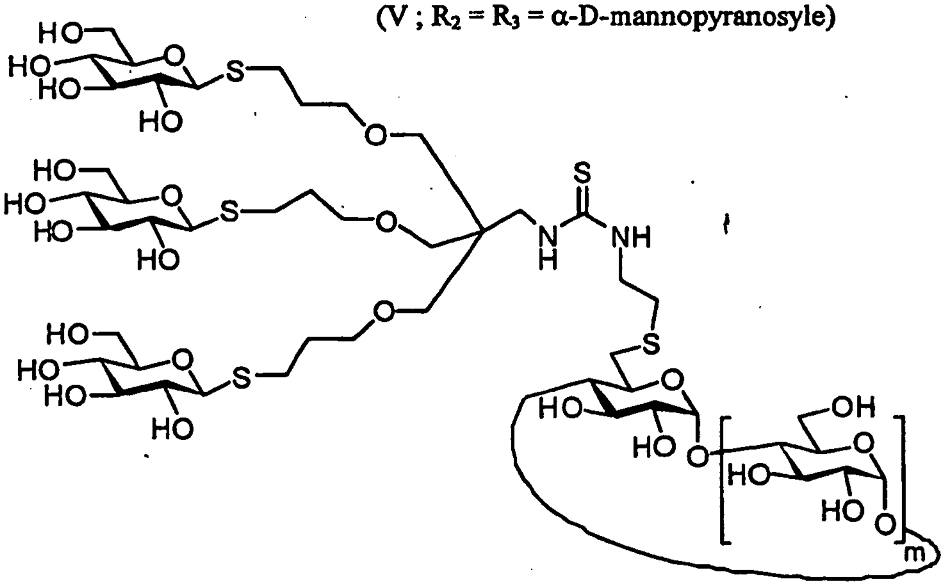

(V ; $R_2$ = $R_3$ = β-D-glucopyranosyle)

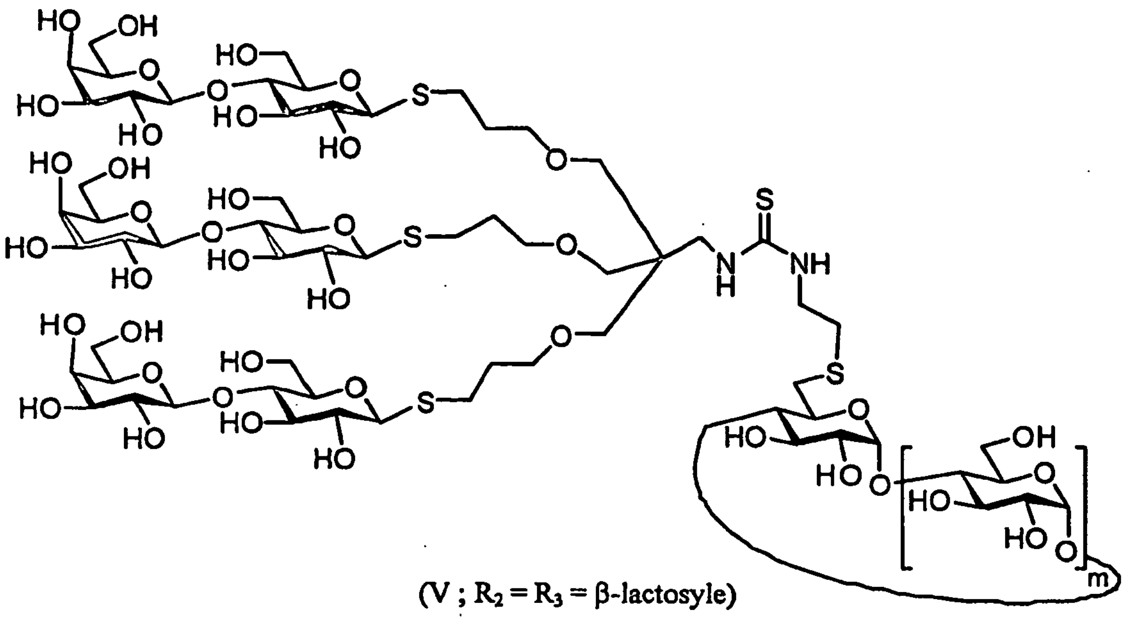

(V ; $R_2 = R_3$ = β-lactosyle)

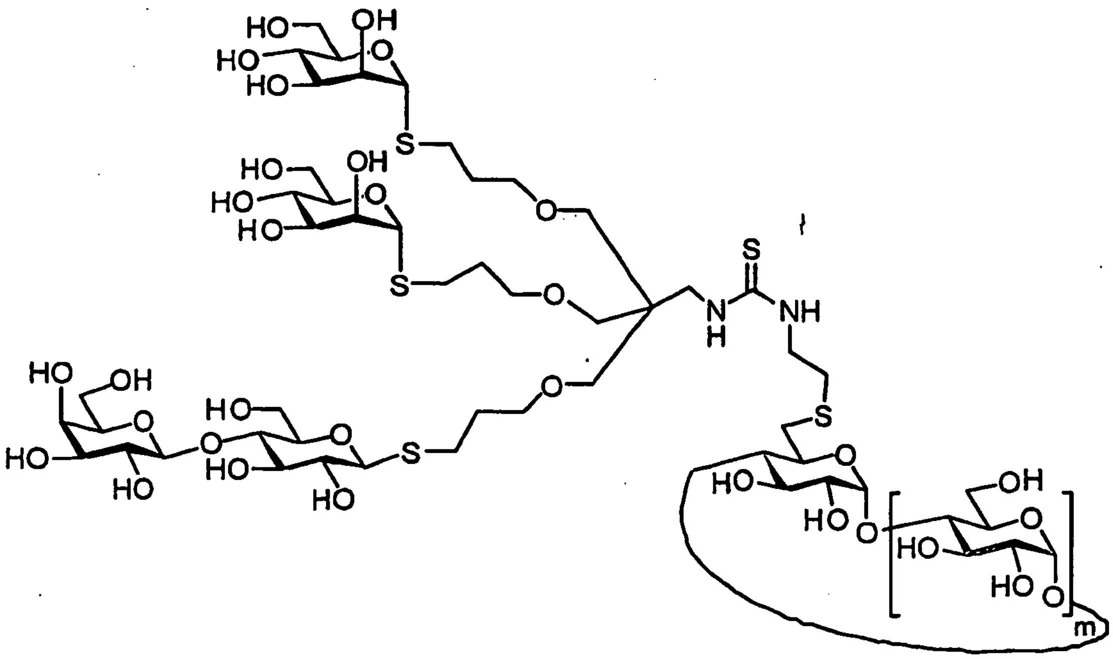

(V ; $R_2$ = α-D-mannopyranosyle ; $R_3$ = β-lactosyle)

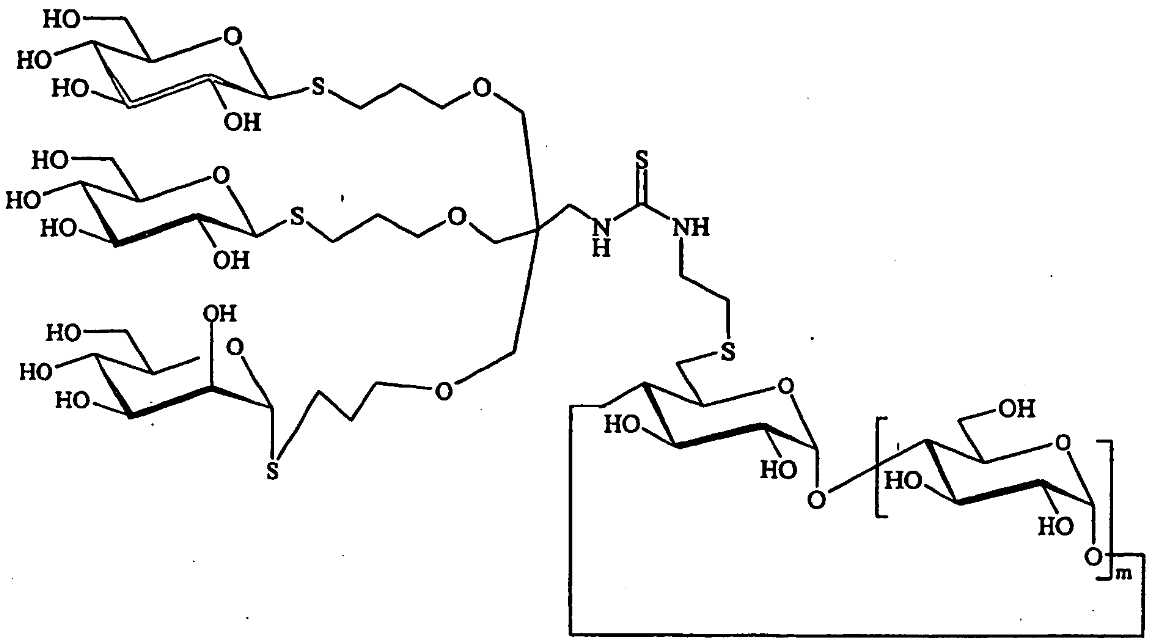

(V ; $R_2$ = β-D-glucopyranosyle ; $R_3$ = α-D-mannopyranosyle)

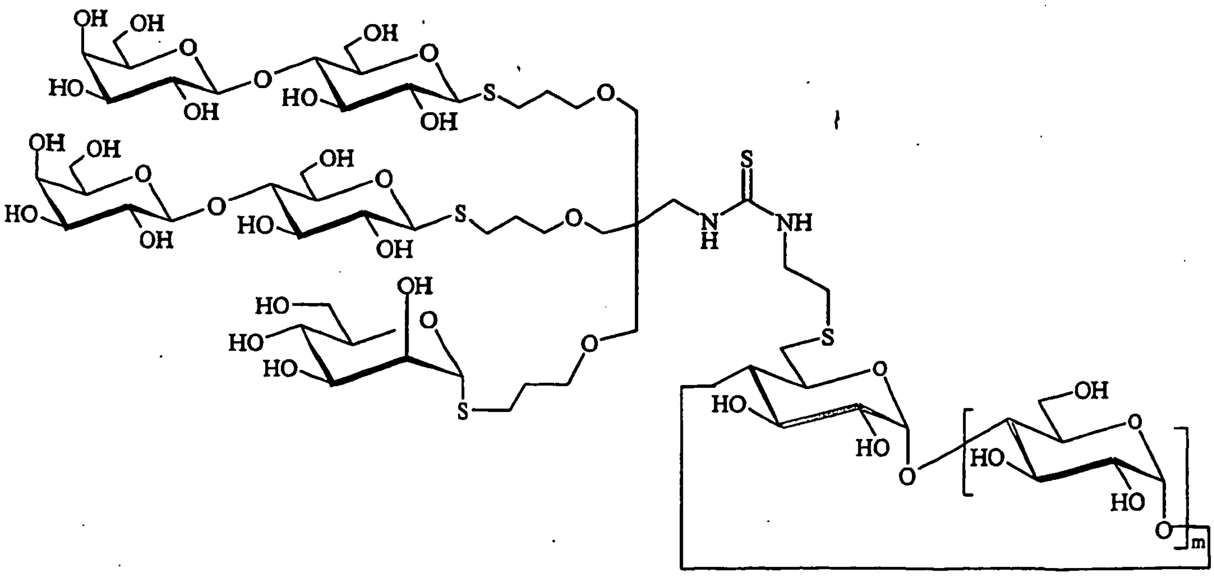

(V ; $R_2$ = β-lactosyle ; $R_3$ = α-D-mannopyranosyle)

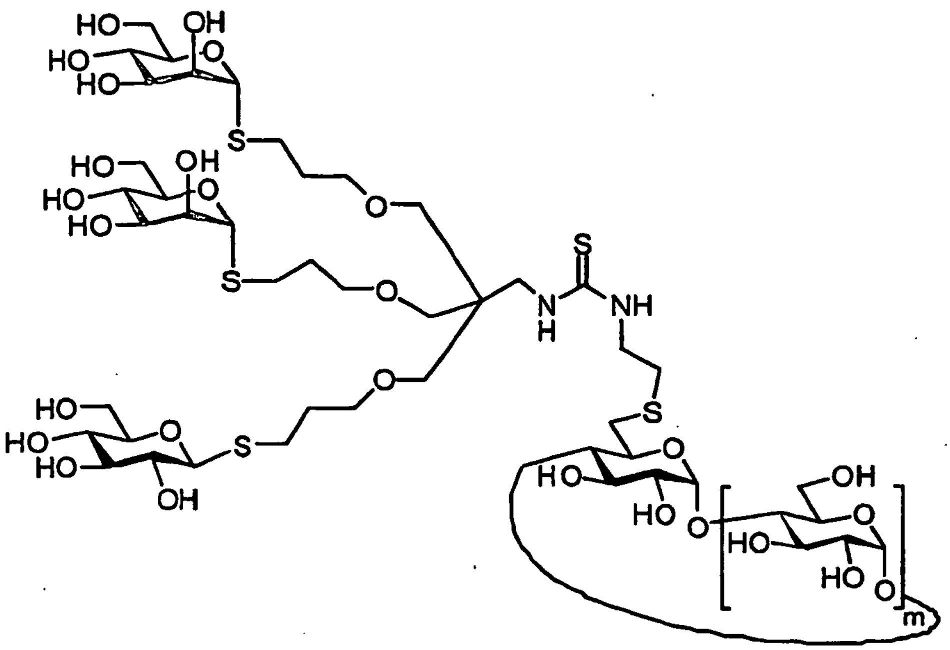

(V ; $R_2$ = α-D-mannopyranosyle ; $R_3$ = β-D-glucopyranosyle)

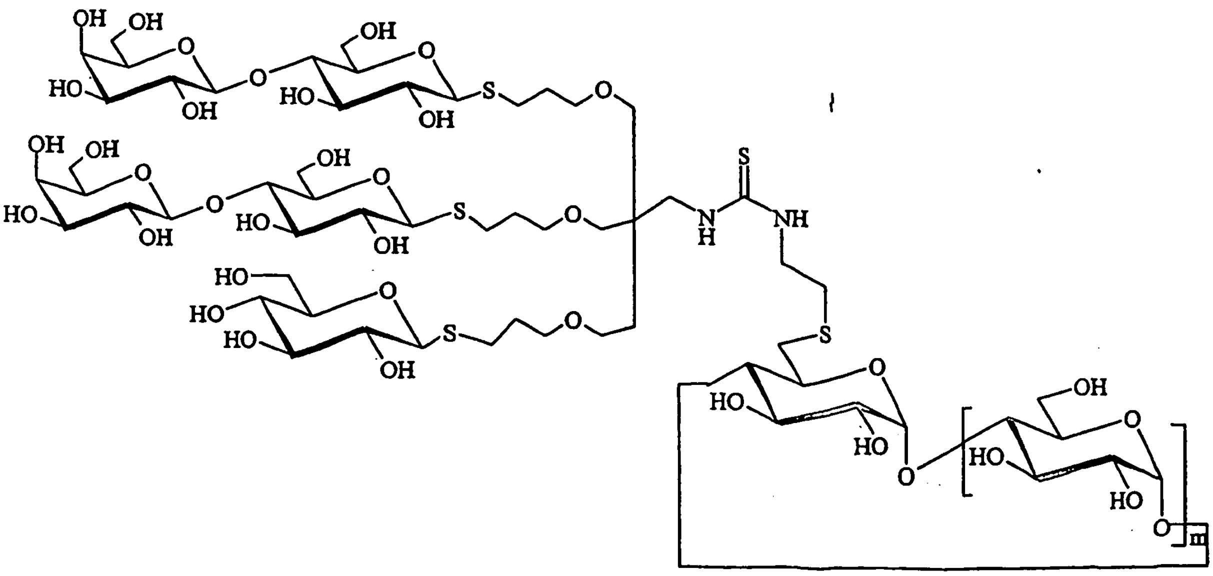

(V ; $R_2$ = β-lactosyle ; $R_3$ = β-D-glucopyranosyle)

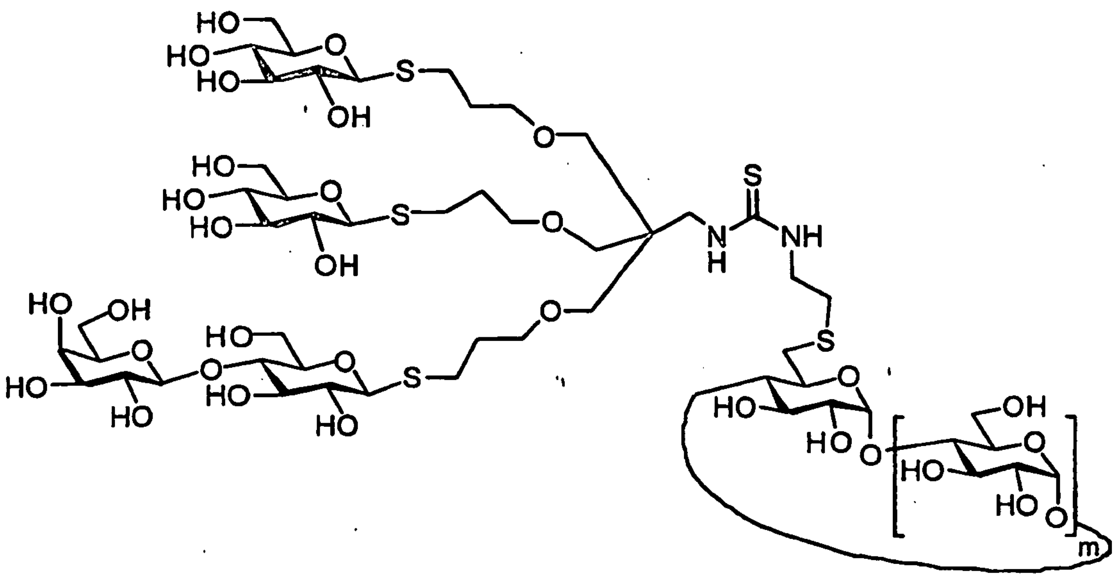

(V ; $R_2$ = β-D-glucopyranosyle ; $R_3$ = β-lactosyle)

b) Composés polysubstitués

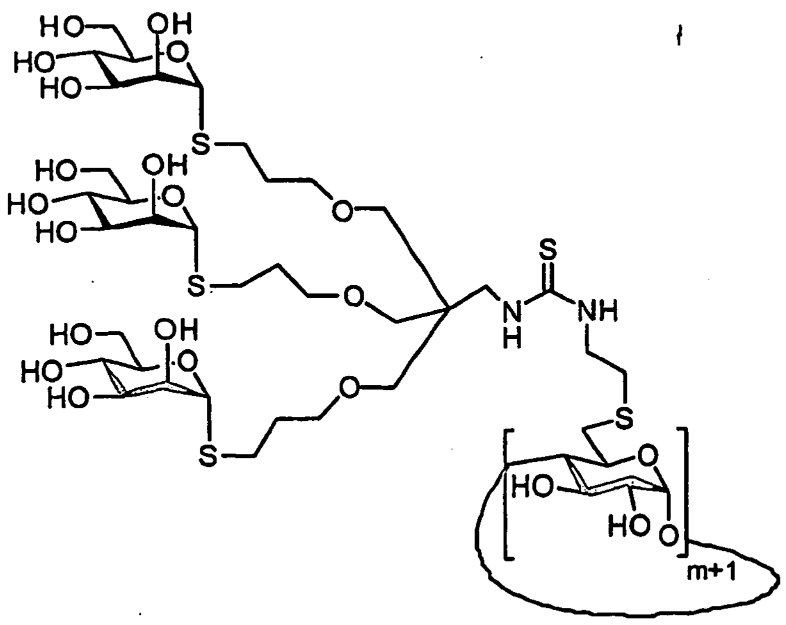

(V ; $R_2$ = $R_3$ = α-D-mannopyranosyle)

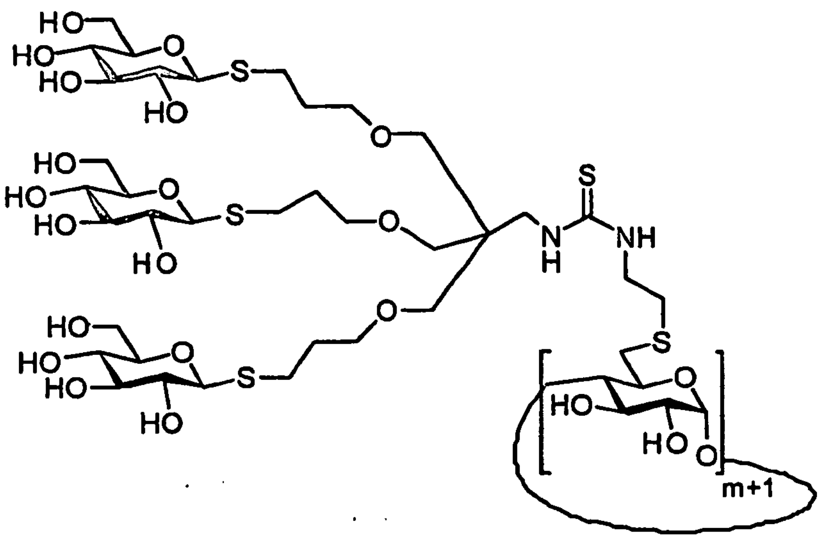

(V ; $R_2 = R_3$ = β-D-glucopyranosyle)

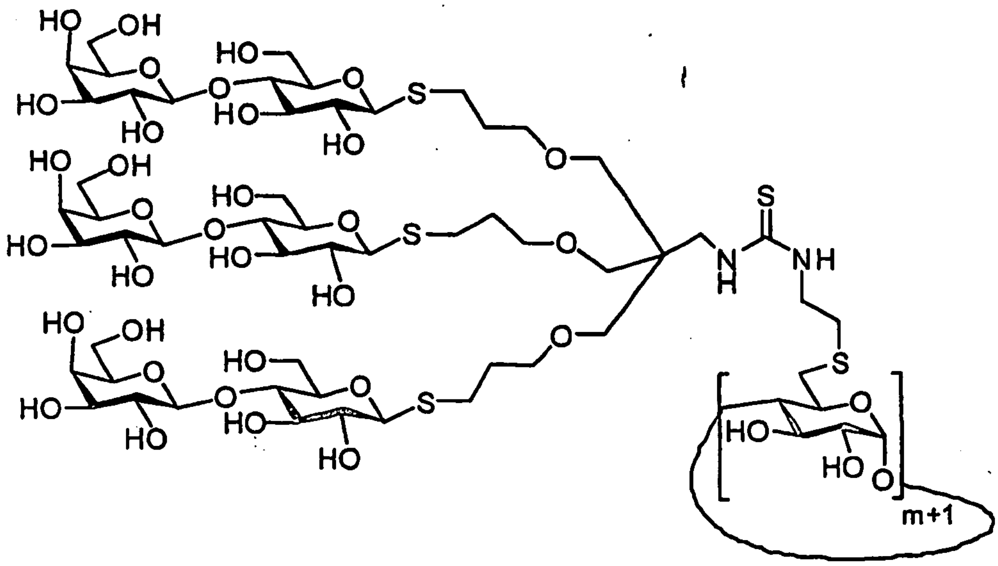

(V ; $R_2 = R_3$ = β-lactosyle)

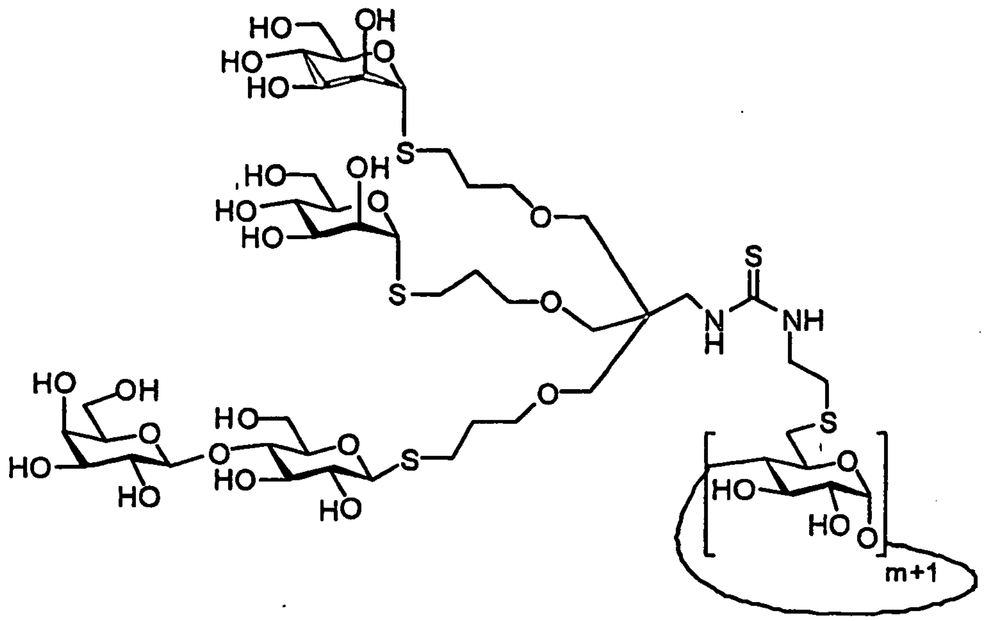

(V ; $R^2$ = α-D-mannopyranosyle ; $R^3$ = β-lactosyle)

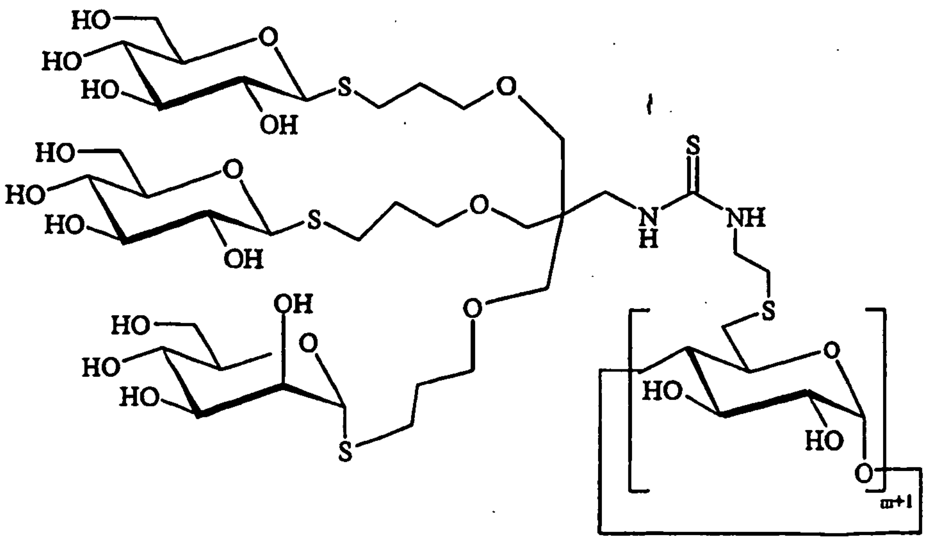

(V ; $R_2$ = β-D-glucopyranosyle ; $R_3$ = α-D-mannopyranosyle)

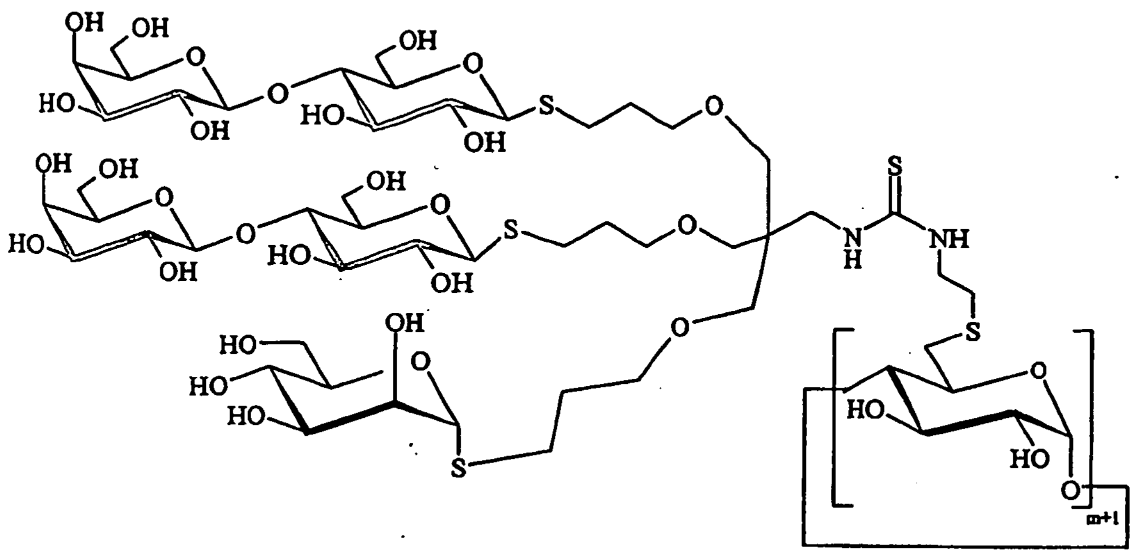

(V ; $R_2$ = β-lactosyle ; $R_3$ = α-D-mannopyranosyle)

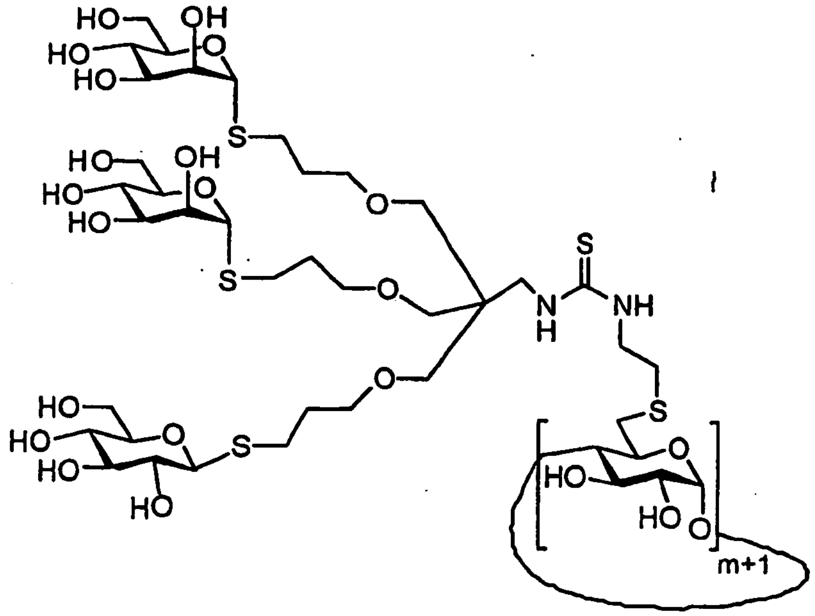

(V ; $R_2$ = α-D-mannopyranosyle ; $R_3$ = β-D-glucopyranosyle)

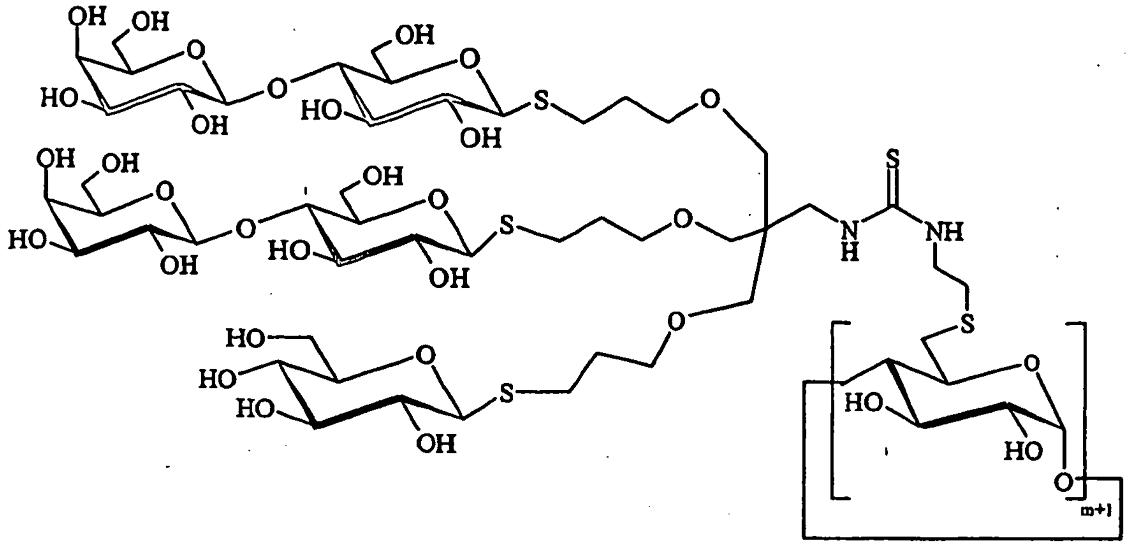

(V ; $R_2$ = β-lactosyle ; $R_3$ = β-D-glucopyranosyle)

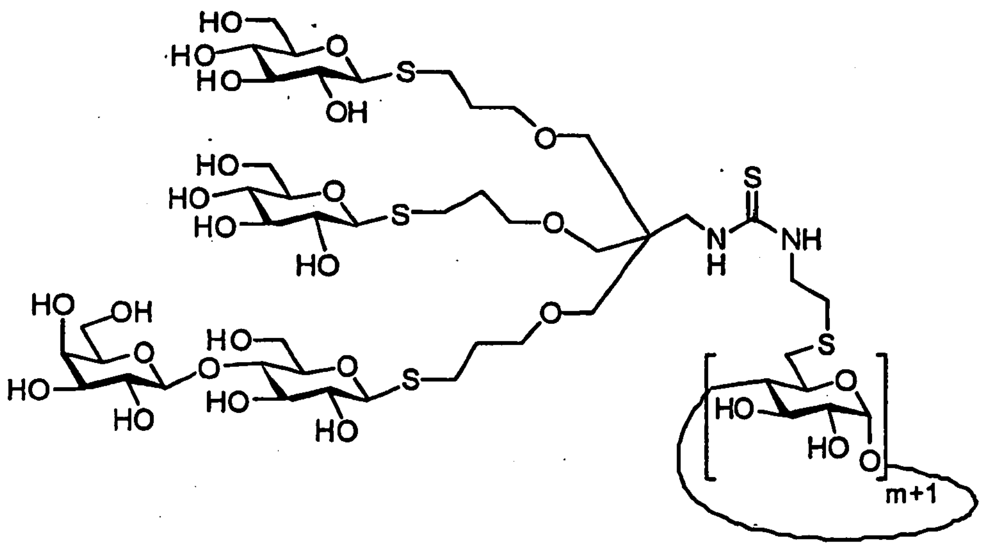

(V ; $R_2$ = β-D-glucopyranosyle ; $R_3$ = β-lactosyle)

La présente invention concerne également un composé tel que défini ci-dessus, caractérisé en ce que m est égal à 6.

L'utilisation des cystéaminyl-cyclodextrines de l'invention, répondant à la formule (I) où Z représente un groupement amine du type NHX (X = H ou substituant alkyle), comme précurseurs pour la préparation de dérivés persubstitués en position alcool primaire par des substituants glucidiques présente des avantages en comparaison à d'autres méthodes préalablement décrites. Notamment, la formation d'une liaison thiourée présente des avantages du point de vue de la simplicité opératoire, des rendements et de la purification du produit final qui ne nécessite le plus souvent pas de séparation chromatographique. De plus, la présence du bras espaceur de type cystéaminyle permet de diminuer l'encombrement stérique et permet l'incorporation d'un élément de multiplication pour accéder à des dérivés hyperramifiés,

susceptibles d'être mieux reconnus par des récepteurs cellulaires spécifiques du fait de la multiplication du motif de reconnaissance, ce qui n'est pas le cas des procédés de l'art antérieur, notamment de la demande internationale WO 97/33919.

En effet, dans l'art antérieur, du fait probablement d'un encombrement stérique, les per(6-amino-6-désoxy) cyclodextrines utilisées comme matière de départ dans la préparation des thiouréido-cyclodextrines persubstitués en position alcool primaire et décrites dans le document WO 97/33919, ont une réactivité nucléophile relativement faible, si bien que la réaction avec des isothiocyanates pour créer la liaison thiourée ne se fait correctement qu'avec des isothiocyanates activés, tels que les glycosylisothiocyanates, ce qui complique la réaction et empêche l'incorporation d'éléments de multiplication. Par ailleurs, le document ChemBioChem 2001 déjà cité montre que les glycosylthioureido-cyclodextrines résultantes sont mal reconnues par des lectines spécifiques complémentaires.

La présente invention concerne également un procédé de préparation d'un composé tel que défini ci-dessus, de formule (I), caractérisé en ce qu'il comprend les étapes suivantes :

- la réaction d'un composé sélectivement ou totalement halogéné en position alcool primaire, de formule (VII) suivante :

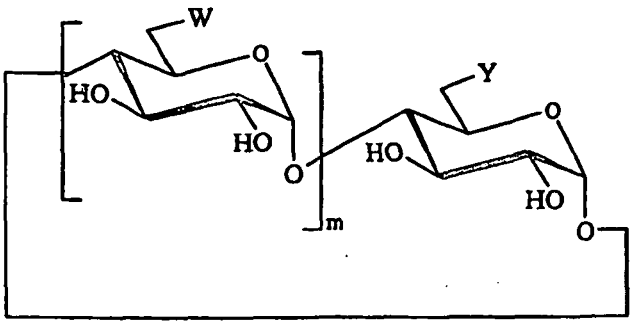

(VII)

m étant tel que défini ci-dessus,
W représentant un groupe OH ou un groupe Y, les groupes W étant tous identiques,
et Y représentant un atome d'halogène choisi dans le groupe constitué du chlore, du brome, de l'iode, et étant de préférence le brome ou l'iode,
avec un ω-aminoalcanethiol de formule (VIII) suivante :

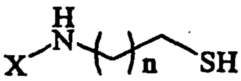

(VIII)

ledit ω-aminoalcanethiol étant éventuellement N-alkylé,

ou le sel correspondant de formule (VIII-a) suivante :

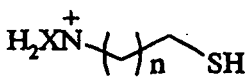

(VIII-a)

ou un sel de tétraalkylammonium de formule (VIII-b) suivante :

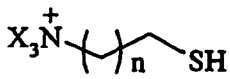

(VIII-b)

ledit sel étant associé à un contre-ion halogénure, de préférence l'ion chlorure,
n et X étant tels que définis ci-dessus, et X étant de préférence un atome d'hydrogène,
le composé de formule (VIII) étant de préférence la cystéamine de formule $H_2N\text{-}CH_2\text{-}CH_2\text{-}SH$,

afin d'obtenir un composé tel que défini ci-dessus et répondant aux formules suivantes (A-a) ou (A-b) :

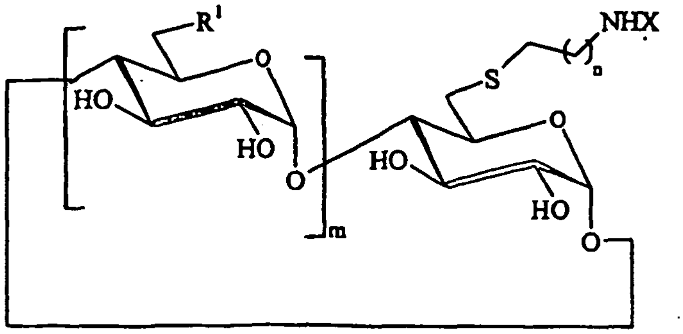

(A-a)

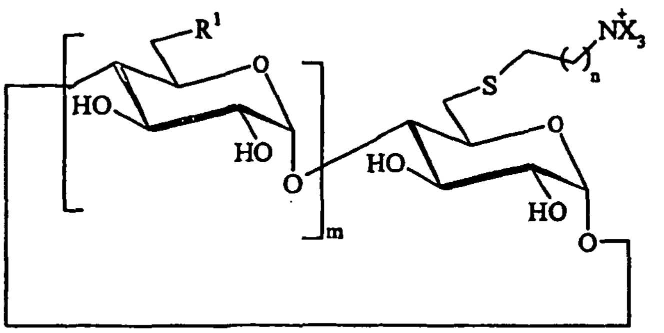

(A-b)

et éventuellement

- la réaction du composé de formule (A-a) tel qu'obtenu à l'étape précédente avec un isothiocyanate de formule (IX) suivante :

  R-N=C=S (IX)

  dans laquelle R est tel que défini ci-dessus, afin d'obtenir un composé tel que défini ci-dessus, et répondant à la formule suivante :

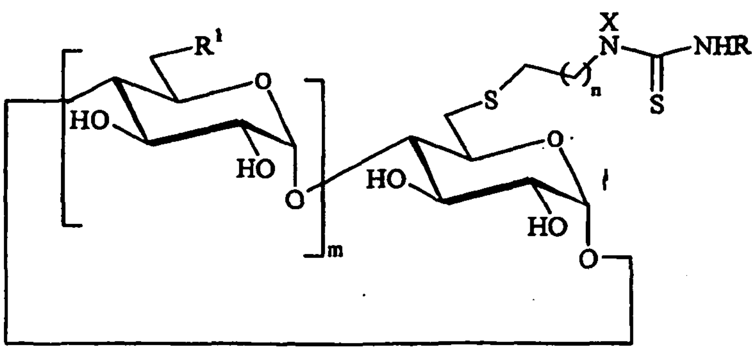

(B)

Ce procédé permet d'obtenir des composés mono- et polysubstitués sur le cycle cyclodextrine.

La première étape du procédé susmentionné est effectuée dans la diméthylformamide en présence de triéthylamine (proportion DMF/triethylamine 4:1), et l'ensemble est agité pendant 48 heures à température ambiante. Le rendement de cette étape est compris entre environ 80 et 95%.

La deuxième étape de ce procédé est un couplage effectué dans un mélange eau-acétone (1:1 à 1:2) à pH 8-9 (bicarbonate de sodium) à température ambiante pendant 1 à 6 heures. Le rendement de cette étape est compris entre environ 55 et 95%.

La présente invention concerne également un procédé de préparation d'un composé tel que défini ci-dessus, répondant à la formule générale (I-b) suivante :

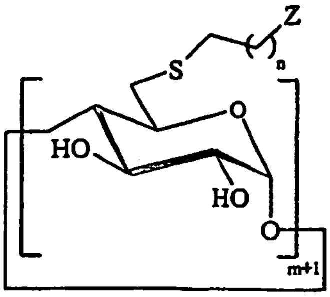

**(I-b)**

ledit procédé étant caractérisé en ce qu'il comprend les étapes suivantes :

- la réaction d'un composé per(6-désoxy-6-halo) cyclodextrine, de formule (VII-a) suivante :

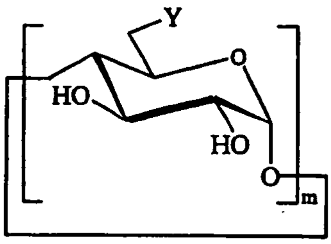

**(VII-a)**

m étant tel que défini ci-dessus, et Y représentant un atome d'halogène choisi dans le groupe constitué du chlore, du brome, de l'iode, et étant de préférence le brome ou l'iode, avec un ω-aminoalcanethiol de formule (VIII) suivante :

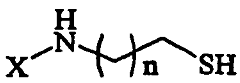

**(VIII)**

ledit ω-aminoalcanethiol étant éventuellement N-akylé, ou le sel correspondant de formule (VIII-a) suivante:

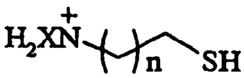

**(VIII-a)**

ou un sel de tétraalkylammonium de formule (VIII-b) suivante :

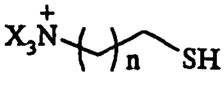

**(VIII-b)**

ledit sel étant associé à un contre-ion halogénure, de préférence l'ion chlorure, n et X étant tels que définis ci-dessus, et X étant de préférence un atome d'hydrogène, le composé de formule (VIII) étant de préférence la cystéamine de formule $H_2N\text{-}CH_2\text{-}CH_2\text{-}SH$, afin d'obtenir un composé de formule (I-c), (I-d) ou (I-e), tel que défini ci-dessus,

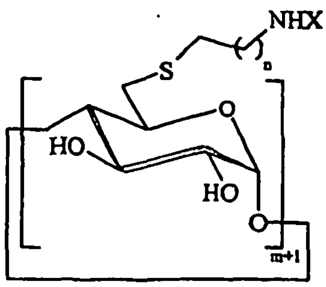

(I-c)

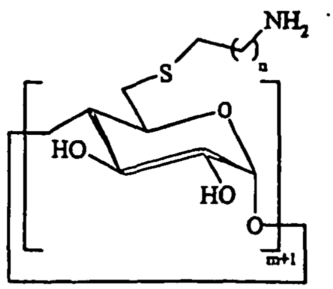

(I-d)

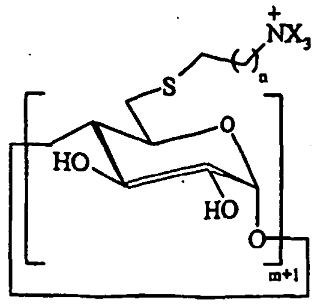

(I-e)

et éventuellement

- la réaction du composé de formule (I-c) tel qu'obtenu à l'étape précédente avec un isothiocyanate de formule (IX) suivante :

R-N=C=S (IX)

dans laquelle R est tel que défini ci-dessus,
afin d'obtenir un composé de formule (II) ou (II-a), tel que défini ci-dessus.

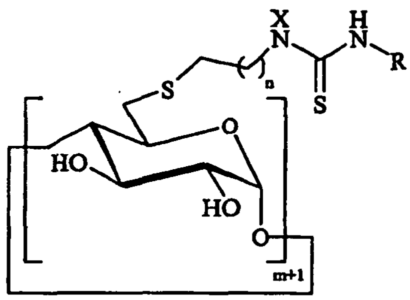

(II)

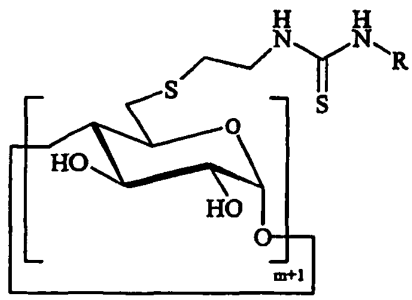

(II-a)

Ce procédé permet d'obtenir des composés polysubstitués sur le cycle cyclodextrine.

La première étape du procédé susmentionné est effectuée dans la diméthylformamide en présence de triéthylamine (proportion DMF/triethylamine 4:1), et l'ensemble est agité pendant 48 heures à température ambiante. Le rendement de cette étape est compris entre environ 80 et 95%.

La deuxième étape de ce procédé est un couplage effectué dans un mélange eau-acétone (1:1 à 1:2) à pH 8-9 (bicarbonate de sodium) à température ambiante pendant 1 à 6 heures. Le rendement de cette étape est compris entre environ 55 et 90%.

La présente invention concerne également un procédé de préparation d'un composé de formule (I-f-bis)

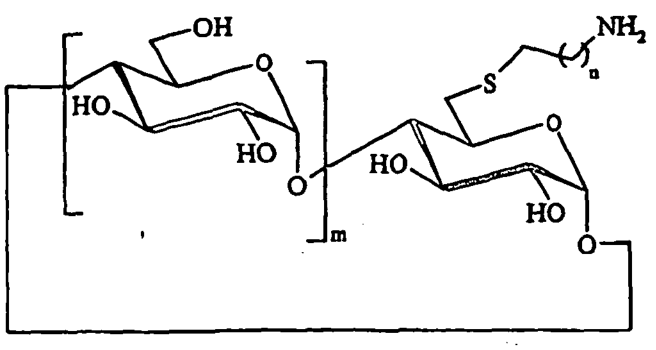

(I-f-bis)

dans laquelle m et n sont tels que définis ci-dessus, n étant de préférence égal à 1, ledit procédé étant caractérisé en ce qu'il comprend la réaction d'un composé sélectivement halogéné en position alcool primaire, de formule (VII) suivante :

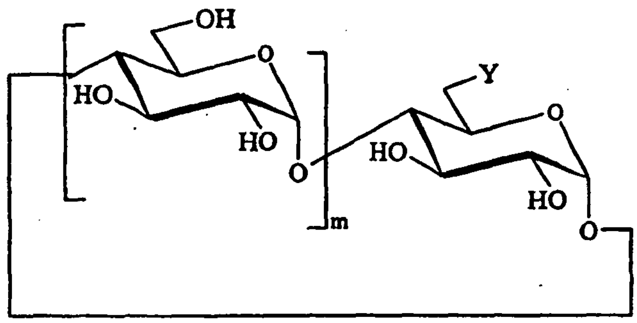

(VII)

m étant tel que défini ci-dessus, et
Y représentant un atome d'halogène choisi dans le groupe constitué du chlore, du brome, de l'iode, et étant de préférence le brome ou l'iode,
avec un ω-aminoalcanethiol de formule suivante :

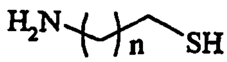

n étant tel que définis ci-dessus,
ou de préférence avec la cystéamine de formule $H_2N\text{-}CH_2\text{-}CH_2\text{-}SH$.

La présente invention concerne également un procédé de préparation de composés tels que définis ci-dessus, répondant à la formule suivante :

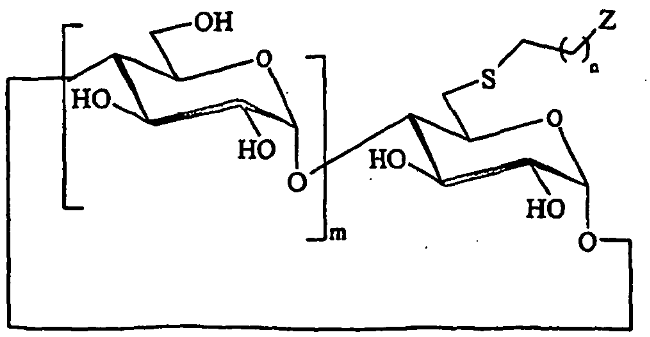

(I-a)

ledit procédé étant caractérisé en ce qu'il comprend les étapes suivantes :

- la réaction d'un composé sélectivement halogéné en position alcool primaire, de formule (VII) suivante :

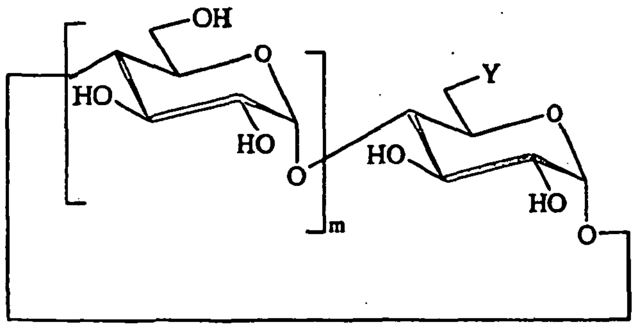

(VII)

m étant tel que défini ci-dessus, et

Y représentant un atome d'halogène choisi dans le groupe constitué du chlore, du brome, de l'iode, et étant de préférence le brome ou l'iode,

avec un ω-aminoalcanethiol de formule (VIII) suivante :

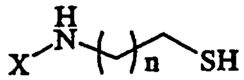

(VIII)

ledit ω-aminoalcanethiol étant éventuellement N-alkylé,

ou le sel correspondant de formule (VIII-a) suivante :

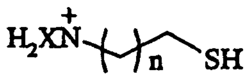

(VIII-a)

ou un sel de tétraalkylammonium de formule (VIII-b) suivante :

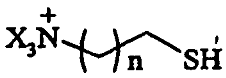

(VIII-b)

ledit sel étant associé à halogénure comme contre-ion, et étant de préférence l'ion chlorure,

n et X étant tels que définis ci-dessus, et X étant de préférence un atome d'hydrogène,

le composé de formule (VIII) étant de préférence la cystéamine de formule $H_2N-CH_2-CH_2-SH$,

afin d'obtenir un composé de formule (I-f) ou (I-g) tel que défini ci-dessus, de formule suivante :

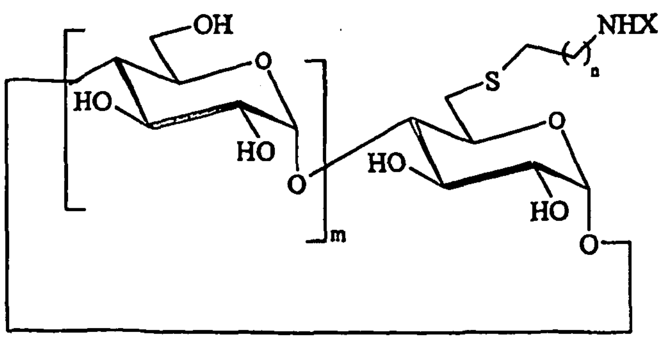

(I-f)

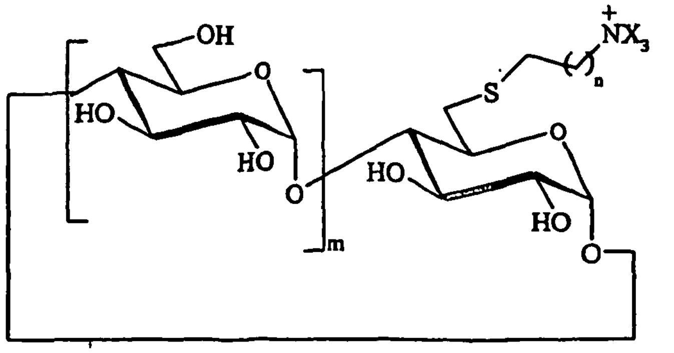

(I-g)

dans lesquelles m, n et X sont tels que définis ci-dessus,
et éventuellement

- la réaction du composé de formule (I-f) tel qu'obtenu à l'étape précédente avec un isothiocyanate de formule (IX) suivante :

R-N=C=S **(IX)**

dans laquelle R est tel que défini ci-dessus,
afin d'obtenir un composé tel que défini ci-dessus, de formule (I-h) :

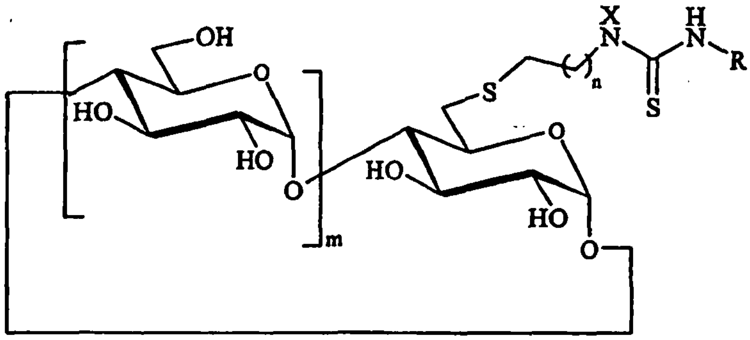

(I-h)

Ce procédé permet d'obtenir des composés monosubstitués sur le cycle cyclodextrine.

La première étape du procédé susmentionné est effectuée dans la diméthylformamide en présence de triéthylamine (proportion DMF/triethylamine 4:1), et l'ensemble est agité pendant 48 heures à température ambiante. Le rendement de cette étape est compris entre environ 85 et 95%.

La deuxième étape de ce procédé est un couplage effectué dans un mélange eau-acétone (1:1) à pH 8-9 (bicarbonate de sodium) à température ambiante pendant 1 à 3 heures. Le rendement de cette étape est compris entre environ 80 et 95%.

Les dérivés de type cystéaminyl-cyclodextrine de l'invention répondant à la formule (I), dans laquelle tous les $R^1$ sont identiques et représentent $S-CH_2-(CH_2)_n-Z$, peuvent être préparés par un procédé qui consiste à faire réagir un dérivé de cyclodextrine persubstitué en position alcool primaire par des groupements halogénés, en particulier les per(6-bromo ou 6-iodo-6-désoxy)cyclodextrines, avec le chlorhydrate de cystéamine commercial ou, en général, avec un halohydrate d'un ω-aminoalcanethiol de 2 à 6 atomes de carbone, éventuellement *N*-alkylé, dans la *N,N*-diméthylformamide en présence d'une base telle qu'une amine comme la triéthylamine ou la *N,N*-diméthylaminopyridine, ou un hydrure métallique tel que les hydrures de sodium ou de lithium. Les dérivés de cyclodextrines persubstitués en position alcool primaire par des groupements halogénés, utilisés comme produits de départ dans ce procédé, peuvent être préparés à partir de la cyclodextrine commerciale en une seule étape et un bon rendement par réaction avec divers réactifs d'halogénation sélective. On peut utiliser pour cela les procédés décrits par J. Defaye et col. dans les documents Supramol. Chem, 2000, 12, pp. 221-224, Polish J. Chem. 1999, 73, pp. 967-971, Tetrahedron Lett. 1997, 38, pp. 7365-7368, Carbohydr. Res. 1992, 228, pp. 307-314, et Angew. Chem., Int. Ed. Engl. 1991, 30, pp. 78-80.

Les dérivés de type cystéaminyl-cyclodextrine de l'invention répondant à la formule (I) dans laquelle tous les

$R^1$ sont identiques et représentent OH peuvent être préparés par un procédé qui consiste à faire réagir un dérivé de cyclodextrine monosubstitué en position alcool primaire par un groupement halogéné, en particulier les mono($6^I$-bromo ou $6^I$-iodo-$6^I$-désoxy)cyclodextrines, avec le chlorhydrate de cystéamine commercial ou, en général, avec un halohydrate d'un ω-aminoalcanethiol de 2 à 6 atomes de carbone, éventuellement N-alkylé, suivant la méthodologie décrite ci-dessus pour la préparation des dérivés persubstitués en position alcool primaire. Les dérivés de cyclodextrines monosubstitués en position alcool primaire par des groupements halogénés, utilisés comme produits de départ dans ce procédé, peuvent être préparés à partir du dérivé $6^I$-*O*-p-toluénesulfonyl de la cyclodextrine en une étape et un bon rendement par déplacement nucléophile du groupe tosylate par un anion halogénure dans la *N*,*N*-diméthylformamide. Pour la préparation des derivés de cyclodextrines comportant un groupement p-toluènesulfonate sur une seule des positions alcool primaire, on peut utiliser le procédé décrit par J. Defaye et col. dans le document WO/9961483 ou les procédés décrits dans l'article de revue par L. Jicsinszky et col. dans Comprehensive Supramolecular Chemistry, Vol. 3 (Editeurs J. Szejtli et T. Osa), Pergamon, Oxford, 1996, pp. 57-198.

L'invention concerne aussi des dérivés de type cystéaminyl-cyclodextrine répondant à la formule (I) dans laquelle les $R^1$ sont différents et représentent OH ou S-$CH_2$-$(CH_2)_n$-Z, avec différents schémas de substitution. On peut accéder à ce type de dérivés en partant des dérivés de cyclodextrine sélectivement substitués par des groupements de type sulfonate en position alcool primaire suivant la séquence des réactions décrite ci-dessus pour la préparation des dérivés de type cystéaminyl-cyclodextrine monosubstitués en position alcool primaire. Pour la préparation des dérivés de cyclodextrine sélectivement polysubstitués en positions alcool primaire par des groupements de type sulfonate, on peut utiliser les procédés décrits dans l'article de revue par L. Jicsinszky et col. dans Comprehensive Supramolecular Chemistry, Vol. 3 (Editeurs J. Szejtli et T. Osa), Pergarnon, Oxford, 1996, pp. 57-198.

Le procédé de préparation des thiouréidocystéaminyl-cyclodextrines de formule (II) de l'invention consiste à faire réagir une cystéaminyl-cyclodextrine de formule (I) où Z représente un groupement amine du type NHX (X = H ou substituant alkyle) avec un isothiocyanate de formule R-NCS dans laquelle R a la signification donnée ci-dessus. Cette réaction peut être faite dans un solvant organique tel que la pyridine ou encore dans un mélange d'eau avec un solvant organique miscible tel que l'acétone.

Lorsque R est un groupe dérivé d'un monosaccharide ou d'un oligosaccharide, l'isothiocyanate de formule R-NCS peut être préparé par réaction du thiophosgène sur un aminodésoxyglycose ou une glycosylamine. On peut suivre pour cette réaction les procédés décrits par J. M. Garcia Fernandez et C. Ortiz Mellet dans Adv. Carbohydr. Chem. Biochem. 1999, 55, pp. 35-135.

Lorsque R comporte un élément de multiplication ramifié dérivé du tris(2-hydroxymethyl)méthylamine (TRIS), on peut préparer l'isothiocyanate correspondant par réaction du thiophosgène sur le dérivé aminé portant des substituants glucidiques sur les positions alcool primaire, comme décrit dans le document Chem. Commun., 2000, pp. 1489-1490. Le glycodendron trivalent aminé précurseur peut être obtenu par réaction de glycosidation d'un dérivé du TRIS avec la fonction amine convenablement protégée sous forme de dérivé carbobenzoxy, comme décrit par P. R. Ashton et al. dans J. Org. Chem. 1998, 63, pp. 3429-3437.

Lorsque R comporte un élément de multiplication ramifié dérivé du pentaérythritol, les glycodendrons convenablement fonctionnalisés avec un groupement isothiocyanate peuvent être préparés à partir du pentaérythritol commercial par une séquence de réactions qui implique :

(i) une triallylation sélective par traitement avec le bromure d'allyle, ce qui laisse un seul groupe hydroxyle libre ;

(ii) l'addition radicalaire d'un 1-thiosucre à la double liaison des groupements allyle. Cette réaction peut se faire, soit par activation par la lumière ultraviolette, soit en présence d'un initiateur de radicaux libres tel que l'azobis (isobutyronitrile) ou l'acide p-nitroperbenzoïque. A titre d'exemple, on peut adapter les conditions réactionnelles décrites par D. A. Fulton et J. F. Stoddart dans Org. Lett. 2000, 2, pp. 1113-1116 ou par X.-B. Meng et al. dans Carbohydr. Res. 2002, 337, pp. 977-981. Nous avons découvert notamment que cette réaction permet l'addition séquentielle des différentes ramifications glucidiques. On peut ainsi accéder à des glycodendrons homogènes aussi bien qu'hétérogènes dans lesquels les substituants glucidiques répondent aux structures mentionnées ci-dessus. Cette approche permet également l'incorporation d'un substituant autre qu'un dérivé glucidique à la structure, en particulier une sonde de type fluorescent telle qu'un dérivé de la fluorescéine. Les 1-thiosucres précurseurs peuvent être préparés, soit à partir des halogénures de glycosyle correspondants par réaction avec la thiourée suivie d'hydrolyse du sel d'isothiouronium résultant, soit à partir de glycals par addition radicalaire de l'acide thioacétique à la double liaison. On peut suivre pour ces réactions les procédés décrits dans les articles de revue publiés par J. Defaye et J. Gelas dans Studies in Natural Products Chemistry, Vol. 8 (Editeur Atta.ur Rahman), Elsevier, Amsterdam, 1991, pp. 315-357 et par H. Driguez dans Top. Curr. Chem., 1997, 187, pp. 85-116.

(iii) la transformation du groupement alcool primaire restant en groupement isothiocyanate. Cette transformation peut se faire par exemple par transformation du groupe hydroxyle en un bon groupe partant tel que le p-toluènesulfonate ou le trifluorométhanesulfonate, suivi de déplacement nucléophile par l'anion azidure et isothiocyanation de l'azide résultante par réaction avec la triphénylphosphine et le bisulfure de carbone. Pour cette transformation, on

peut suivre le procédé décrit dans le document Chem. Commun., 2000, pp. 1489-1490.

Les procédés décrits ci-dessus pour l'obtention des cystéaminyl- et thiouréidocystéaminyl-cyclodextrines de l'invention sont très intéressants car ils permettent d'obtenir les dérivés souhaités dans un nombre réduit d'étapes et avec des rendements élevés. En plus, ces procédés permettent d'accéder à des dérivés hyperramifiés homogènes et hétérogènes non accessibles commodément par d'autres voies. Ces dérivés sont reconnus de façon très efficace par des lectines membranaires spécifiques, en fonction des substituants glucidiques incorporés.

La présente invention concerne également un complexe d'inclusion d'un composé tel que défini précédemment, avec une molécule pharmacologiquement active, le rapport molaire entre le composé et la molécule pharmacologiquement active étant avantageusement d'environ 50:1 à environ 1:1.

L'expression "complexe d'inclusion" désigne l'entité supramoléculaire composée par le composé selon l'invention (molécule hôte) et la molécule pharmacologiquement active (molécule invitée), dans laquelle la molécule invitée est maintenue dans la cavité hydrophobe de la molécule hôte par l'intermédiaire d'interactions non covalentes.

L'expression "molécule pharmacologiquement active" désigne un principe actif doté d'activité thérapeutique, tel qu'un agent anticoagulant, antidiabétique, antiinflammatoire, antibiotique, antitumoral, etc.

La présente invention concerne également un complexe tel que défini ci-dessus, caractérisé en ce que la molécule pharmacologiquement active est un agent antitumoral, appartenant notamment à la famille du Taxol.

On peut notamment citer comme agents antitumoraux, les agents antitumoraux non biologiques approuvés par la FDA (Food and Drug Administration) tels que :

- des agents alkylants : nitrosourées telles que : lomustine, carmustine et streptozocine ; huiles de moutarde telles que : méchloréthamine, melphalan, uracile moutarde à l'azote, chlorambucile, cyclophosphamide et iphosphamide ; autres agents d'alkylation tels que : cisplatine, carboplatine, mitomycine, thiotepane, decarbazine, procarbazine, hexaméthyl mélamine, triéthylène mélamine, bisulfane, pipobromane, et mitotane ;
- des antimétabolites : méthotrexate, trimétrexate, pentostatine, cytarabine, ara-CMP, fludarabine phosphate, hydroxyurée, fluorouracile, floxuridine, chlorodésoxyadénosine, gemcitabine, thioguanine et 6-mercaptopurine ;
- des agents de coupure de l'ADN : bléomycine ; poisons de la topoisomérase I tels que : topotecan, irinotecan, sel de sodium de la camptothécine et des analogues de topotecan et irinotecan ; poisons de la topoisomérase II tels que :daunorubicine, doxorubicine, idarubicine, mitoxantrone, téniposide et étoposide ;
- des agents d'intercalation de l'ADN : dactinomycine et mithramycine ;
- des inhibiteurs de la mitose : vinblastine, vincristine, navelbine, paclitaxel et docétaxel.

Un agent antitumoral préféré, appartenant à la famille du Taxol, est le docétaxel (Taxotère®).

On a évalué l'affinité des thiouréidocystéaminyl-cyclodextrines de l'invention pour des lectines membranaire spécifiques *in vitro* suivant le protocole ELLA (de l'anglais Enzyme-Linked Lectin Assay). On peut trouver de nombreux exemples d'application de ce protocole dans l'article de revue de J. J. Lundquist et E. J. Toon dans Chem. Rev. 2002, 102, pp. 555-578. Cette technique mesure la capacité d'un ligand mono- ou oligosaccharidique soluble d'inhiber l'association entre une lectine complémentaire et un autre ligand de référence fixé sur une microplaque de support plastique. Ainsi, les thiouréidocystéaminyl-cyclodextrines portant des substituants $\alpha$-D-mannopyranosyle sont reconnus par la concanavaline A ou par la lectine spécifique du mannose des macrophages, les dérivés avec des substituants $\beta$-lactosyle sont reconnus par la lectine spécifique du lactose *d'Arachys hypogaea* ou des hépatocytes, et les dérivés comportant le substituant tétrasaccharidique sialyl-Lewis X sont reconnus par les sélectines de l'endothélium impliquées dans le processus de l'inflammation.

On observe, de façon générale, une augmentation importante de l'affinité des dérivés de type thiouréidocystéaminyl-cyclodextrine vis-à-vis des lectines spécifiques en comparaison avec les conjugués de type thiouréido-cyclodextrine décrits dans le document ChemBioChem 2001.

Les thiouréidocystéaminyl-cyclodextrines de l'invention comportant des éléments de reconnaissance cellulaire sont utilisables en particulier pour masquer des récepteurs de membrane complémentaires. Ainsi, les thiouréidocystéaminyl-cyclodextrines polymannosylés permettent de bloquer le récepteur spécifique du mannose des macrophages, les dérivés polylactosylés le récepteur spécifique de lactose des hépatocytes et les dérivés incorporant des substituants dérivés du tétrasaccharide sialyl Lewis X les sélectines de l'endothélium. Dans ce contexte, ces dérivés sont utilisables en tant que molécules actives pour la prévention et le traitement des processus d'infection et de cancérisation impliquant des phénomènes d'adhésion et, également, pour la prévention et le traitement des maladies liées au dérèglement du processus de l'inflammation.

Ces composés d'inclusion peuvent être préparés par des procédés classiques, par exemple par dispersion de la molécule active en solution ou à l'état pur dans une solution du dérivé de cyclodextrine, en présence ou non d'un cosolvant, comme décrit dans le document WO 97/33919.

Ces complexes d'inclusion peuvent être préparés par exemple en ajoutant à une solution ou à une suspension

du composé de l'invention de formule (I) la molécule pharmacologiquement active en solution ou à l'état pur. Le complexe d'inclusion ainsi formé peut être isolé par lyophilisation.

Dans le cas où on ajoute la molécule pharmacologiquement active en solution, par exemple un agent antitumoral de la famille du Taxol, on utilise une solution concentrée de la molécule dans un solvant organique miscible à l'eau, par exemple l'acétone, et on soumet le mélange obtenu à une agitation et à un barbotage de gaz inerte tel que l'azote, pour éliminer le solvant organique.

Dans le cas des composés de la famille du Taxol, tels que le Taxotère®, il est aussi possible de disperser ce produit à l'état pur dans une solution stérile d'un composé selon l'invention.

La présente invention concerne également une composition pharmaceutique comprenant un composé tel que défini ci-dessus, ou un complexe d'inclusion tel que défini ci-dessus, avec un véhicule pharmacologiquement acceptable.

La présente invention concerne également une composition pharmaceutique telle que définie ci-dessus, sous forme de solution aqueuse.

La présente invention concerne également une composition pharmaceutique telle que définie ci-dessus, caractérisée en ce qu'elle contient par dose unitaire d'environ 50 mg à environ 500 mg de l'un des composés tels que définis précédemment, ou en ce qu'elle contient par dose unitaire d'environ 100 mg à environ 750 mg de l'un des complexes tels que définis ci-dessus.

Ces compositions pharmaceutiques, qui peuvent être administrées par voie orale ou parentérale, sont par exemple des solutions, des poudres, des suspensions, etc., en particulier des solutions injectables.

## LÉGENDE DES FIGURES

La Figure 1 représente la variation de l'inhibition de l'association entre la lectine ConA et le mannane de levure en présence des composés n°2, n°3, n°4, n°6 et n°7, ainsi que pour l'heptakis(6-désoxy-6-α-D-mannopyranosylthitiuréido)cyclomalto-heptaose (dérivé de la per-(C-6)-amine β-cyclodextrine), en fonction de la concentration de ligand en μM.

La courbe en trait plein avec les ronds noirs correspond au dérivé heptavalent de la per-(C-6)-amine β-cyclodextrine; la courbe en trait pointillé avec les croix correspond au composé n°2 ; la courbe en trait plein avec les croix correspond au composé n°3 ; la courbe en trait plein avec les triangles blancs correspond au composé n°4 ; la courbe en trait plein avec les losanges blancs correspond au composé n°6 et la courbe en trait pointillé avec les carrés noirs correspond au composé n°7.

## PARTIE EXPÉRIMENTALE

### Exemple 1: Préparation de l'heptachlorhydrate d'heptakis[6-*S*-(2-aminoéthyl-6-thio)]cyclomaltoheptaose (composé no. 1).

Ce composé répond à la formule (I) donnée ci-dessus avec n = 1, m = 6, dans laquelle tous les $R^1$ sont identiques et représentent $S-CH_2-(CH_2)_n-Z$, Z représentant un groupement $NH_2$, et a été isolé sous forme de son heptachlorhydrate.

A une solution de chlorhydrate de 2-aminoéthanethiol (999 mg, 8,8 mmol) dans la DMF distillée (10 mL), sous argon, on ajoute la triéthylamine (2,5 mL) en utilisant une seringue. On observe que la suspension devient violette. A cette suspension, on ajoute goutte à goutte une solution de l'heptakis[6-bromo-6-désoxy]cyclomaltoheptaose (1 g) dans la DMF (5 mL). Le mélange réactionnel est agité pendant 48 h à température ambiante. On observe alors l'apparition d'un précipité blanc. Le solide est filtré et solubilisé dans l'eau (20 mL), le pH de la solution est ajusté à 4 par addition d'acide chlorhydrique dilué et finalement la solution est lyophilisée. Le résidu solide est mis en suspension dans l'éthanol à 96 % et la suspension est agitée pendant 30 min, puis filtrée et séchée. On obtient ainsi le composé n° 1 (976 mg, 86%) ayant les caractéristiques suivantes :

- $[a]_D$ + 81,4° (c 1,0 ; eau)
- spectre de masse (électrospray) : m/z 1549 (100%, $[M + Na]^+$)
- solubilité dans l'eau : 500 $g.L^{-1}$ (323 $mmol.L^{-1}$)
- donnés de $^1H$ RMN (500 MHz, $D_2O$) : δ 5,03 (7 H, d, $J_{1,2}$ 3,5 Hz, H-1), 3,93 (7 H, td, $J_{4,5}$ 9,2 Hz, $J_{5,6b}$ 7,0 Hz, $J_{5,6a}$ 2,0 Hz, H-5), 3,82 (7 H, t, $J_{2,3} = J_{3,4}$ 9,4 Hz, H-3), 3,56 (7 H, dd, H-2), 3,55 (7 H, t, H-4), 3,17 (14 H, t, $^3J_{H,H}$ 6,8 Hz, $CH_2N$), 3,08 (7 H, dd, $J_{6a,6b}$ 13,5 Hz, H-6a), 2.91 (14 H, t, $^3J_{H,H}$ 6,8 Hz, $CH_2S$), 2,90 (7 H, dd, H-6b)
- donnés de $^{13}C$ RMN (125,7 MHz, $D_2O$) : δ 101,6 (C-1) ; 83,6 (C-4) ; 72,7 (C-3) ; 72,0 (C-2) ; 71,6 (C-5) ; 38,6 ($CH_2N$) ; 32,7 (C-6) ; 30,0 ($CH_2S$).

**Exemple 2 : Préparation de l'heptakis[6-S-[2-[*N'*-(α-D-mannopyranosyl) thioureidoléthyl-6-thio]cycloraaltoheptaose (composé no. 2).**

Ce composé répond à la formule (II) donnée ci-dessus avec n = 1, m = 6, X représentant un atome d'hydrogène et R répondant à la formule :

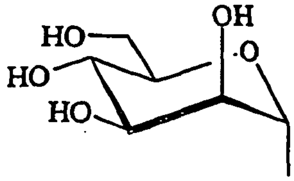

(III)

Ce composé est obtenu par condensation du composé no. 1 (voir exemple 1) avec l'isothiocyanate de 2,3,4,6-tétra-*O*-acétyl-α-D-mannopyranosyle suivie d'une hydrolyse alcaline du groupement protecteur acétyle.

Une solution du composé no. 1 (25 mg, 14 μmol) dans l'eau (2 mL) est additionnée d'hydrogénocarbonate de sodium solide jusqu'à ce que le pH atteigne des valeurs entre 8 et 9. Après 20 min, une solution d'isothiocyanate de 2,3,4,6-tétra-*O*-acétyl-α-D-mannopyranosyle (42 mg, 0,11 mmol, 1,1 équiv) dans l'acétone (3 mL) est ajoutée et le mélange réactionnel est agité à température ambiante pendant 1 h. On évapore l'acétone dans le rotavapor et la solution aqueuse résultante est lyophilisée. Le résidu est repris par le méthanol, filtré et concentré. Le solide résultant est dissous dans le méthanol (6 mL), additionné d'une solution 1 N de méthylate de sodium dans le méthanol (196 μL, 0,5 équiv) et la solution est agitée à 0°C pendant 5 min. On observe la précipitation d'un solide blanc qui est redissous dans l'eau (8 mL). La solution aqueuse est agitée à 0°C pendant 15 min, neutralisée et déminéralisée par traitement, successivement, avec la résine échangeuse ionique Amberlite IR-120 ($H^+$) et la résine mixte Duolite MB-6331 ($H^+$,$OH^-$), filtrée et lyophilisée. Après purification par chromatographie de filtration sur gel (Sephadex G-25), on obtient le composé no. 2 (30 mg, 71 %) ayant les caractéristiques suivantes :

- $[\alpha]_D$ + 50,3° (c 1,0, eau)
- spectre de masse (MALDITOF) : m/z 3103,9 $[M + H]^+$
- solubilité dans l'eau: 780 $g.L^{-1}$ (251 $mmol.L^{-1}$)
- données de $^1H$ RMN (500 MHz, 333 K, $D_2O$) : δ 5,85 (7 H, bs, H-1') ; 5,43 (7 H, bs, H-1) ; 4,34 (7 H, m, H-2') ; 4,30 (7 H, m, H-6a') ; 4,28 (7 H, m, H-5) ; 4,22 (7 H, m, H-3) ; 4,15 (7 H, d, $J_{6a',6b'}$ 12,0 Hz, H-6b') ; 4,01 (7 H, m, H-3') ; 3,96 (7 H, m, H-2) ; 3,92 (7 H, t, $J_{3',4'} = J_{4',5'}$ 9,0 Hz, H-4') ; 3,88 (7 H, m, H-4) ; 3,80 (14 H, m, $CH_2S$) ; 3,75 (7 H, m, H-5') ; 3,72 (14 H, m, $CH_2N$) ; 3,55 (7 H, m, H-6a) ; 3,53 (7 H, m, H-6b)
- données de $^{13}C$ RMN (125,7 MHz, 333 K, $D_2O$): δ 181,7 (CS) ; 101,7 (C-1) ; 82,5 (C-1') ; 84,1 (C-4) ; 73,5 (C-5') ; 73,0 (C-3) ; 72,0 (C-2, C-5) ; 70,5 (C-3') ; 69,5 (C-2') ; 66,6 (C-4') ; 60,7 (C-6') ; 44,2 ($CH_2N$) ; 33,2 (C-6) ; 31,6 ($CH_2S$).

Exemple 3 : Préparation de l'heptakis[6-S-[2-*N'*-tris(α-D-mannopyranosyl oxyméthyl)méthylthiouréido]éthyl-6-thio]cyclomaltoheptaose (composé no. 3).

Ce composé répond à la formule (II) donnée ci-dessus avec n = 1, m = 6, X réprésentant un atome d'hydrogène et R répondant à la formule : '

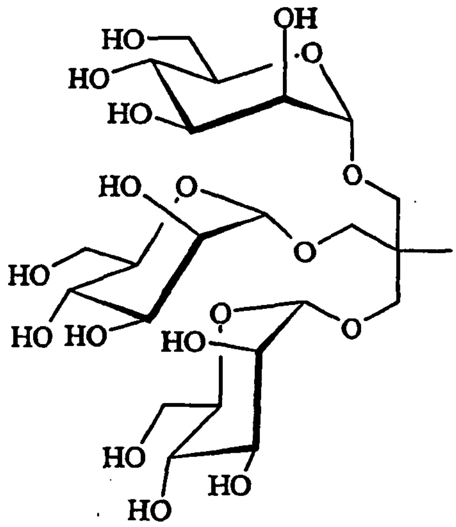

(IV)

Ce composé est obtenu par condensation du composé no. 1 (voir exemple 1) avec le tris(2,3,4,6-tetra-*O*-acétyl-α-D-mannopyranosyloxyméthyl)méthyl isothiocyanate suivi d'une hydrolyse alcaline du groupement protecteur acétyle.

A une solution du composé no. 1 (10 mg, 5.5 μmol), dans l'eau (1 mL), on ajoute l'hydrogénocarbonate de sodium solide jusqu'à que le pH atteigne des valeurs entre 8 et 9. Après 20 min, une solution de tris(2,3,4,6-tétra-*O*-acétyl-α-D-manno pyranosyloxyméthyl)méthyl isothiocyanate (45 mg; 40 μmol ; 1,04 équiv) dans l'acétone (1 mL) est ajoutée et le mélange réactionnel est agité à température ambiante pendant 4 h. On évapore l'acétone dans le rotavapor et la solution aqueuse résultante est extraite par le dichlorométhane (3 x 5 mL). Le solvant organique est évaporé, le résidu est dissous dans le méthanol (3 mL) et additionné d'une solution 1 N de méthylate de sodium dans le méthanol jusqu'à pH 8-9 et la solution est agitée à température ambiante pendant 5 min. On observe la précipitation d'un solide blanc qui est redissous par addition d'eau (1 mL). La solution aqueuse est agitée à température ambiante pendant 15 min, neutralisée et déminéralisée par traitement, successivement, avec la résine échangeuse ionique Amberlite IR-120 ($H^+$) et la résine mixte Duolite MB-6331 ($H^+$, OH), filtrée et lyophilisée. Après purification par chromatographie de filtration sur gel (Sephadex G-25) on obtient le composé no. 3 (15 mg, 45%) ayant les caractéristiques suivantes :

- $[\alpha]_D$ + 130,0° (c 0,5, eau)
- spectre de masse (MALDITOF) ; *m*/*z* 6108,53 ($[M + H]^+$
- solubilité dans l'eau : 800 $g.L^{-1}$ (131 $mmol.L^{-1}$)
- données de $^1H$ RMN (500 MHz, 343 K, $D_2O$) : δ 5,53 (7 H, bs, H-1) ; 5,31 (21 H, s, H-1') ; 4,49 (21 H, d, $^2J$H,H 10,5 Hz, $OCH_2a$) ; 4,41 (3 H, s, H-2') ; 4,40 (7 H, m, H-5) ; 4,36 (21 H, d, $OCH_2b$) ; 4,32 (7 H, m, H-3) ; 4,29 (21 H, d, $J_{6a'6b'}$ 12,0 Hz, H-6a') ; 4,21 (14 H, m, $CH_2N$); 4,20 (21 H, dd, $J_{2',3'}$ 4,0 Hz, $J_{3',4'}$ 10.0 Hz, H-3') ; 4,18 (21 H, m, H-6b') ; 4,12 (21 H, t, $J_{4',5'}$ 10,0 Hz, H-4') ; 4,09 (7 H, m, H-2) ; 4,02 (7 H, m, H-5') ; 3,78 (7 H, m, H-4) ; 3,26 (7 H, m, H-6a) ; 3,47 (7 H, m, $J_{5,66}$ 8,5 Hz, H-6b) ; 3,22 (2 H, m, $CH_2S$).
- données de $^{13}C$ RMN (125,7 MHz, 323 K, $D_2O$) : δ 181,0 (CS) ; 102,6 (C-1) ; 100,9 (C-1') ; 84,3 (C-4) ; 73,7 (C-5') ; 73,5 (C-3) ; 72,6 (C-2, C-5) ; 71,3 (C-3') ; 70,5 (C-2') ; 67,3 (C-4', $OCH_2$) ; 61,4 (C-6') ; 44,8 (Cq, $CH_2N$) ; 34,2 (C-6) ; 32,8 ($CH_2S$).

Les exemples qui suivent se rapportent à des thiouréidocystéaminyl-cyclodextrines hyperramifiées qui comportent un élément multiplicateur dérivé du pentaérythritol répondant à la formule suivante :

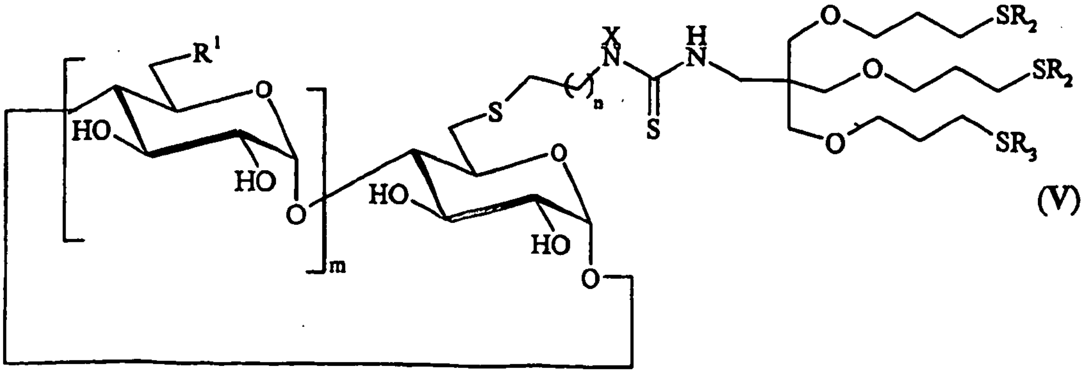

(V)

Le précurseur universel des glycodendrons utilisés par la synthèse de ces dérivés est le 2,2,2-tris(2-oxapent-4-enyl)éthanol (tri-*O*-allylpentaérythritol), obtenu à partir du pentaérythritol commercial.

Une suspension de pentaérythritol (1,36 g, 10 mmol) dans l'hydroxyde de sodium à 40% aqueux est agitée vigoureusement à 70-75°C pendant 15 min. On ajoute le bromure d'allyle (3,2 mL, 40 mmol, 4 équiv) goutte à goutte et on continue à agiter pendant 1 h. Le mélange réactionnel est extrait par le dichlorométhane (3 x 20 mL) et la phase organique est lavée par l'eau, séchée (sulfate de sodium anhydre) et concentrée. Le résidu est purifié par chromatographie sur colonne de gel de silice avec l'acétate d'éthyle-éther de pétrole 1:4. On obtient ainsi 1,28 g (rendement 50%) d'une huile incolore, présentant les caractéristiques suivantes :

- spectre de masse ($FAB^+$) : *m/z* 257 (100%, $[M+H]^+$)

**Exemple 4 : Préparation de l'heptakis(6-*S*-[2-*N'*-[2,2,2-tris[5-(1-thio-α-D-mannopyranosyl)-2-oxapentyl]éthyl] thiouréido]étlhyl-6-thio]cyclomaltoheptaose (composé no. 4).**

Ce composé répond à la formule (V) donnée ci-dessus avec n = 1, m = 6, dans laquelle tous les $R^1$ sont identiques et représentent $CH_2\text{-}C[(CH_2OCH_2CH_2CH_2R_2)_2(CH_2OCH_2CH_2CH_2R_3)]$, X représente un atome d'hydrogène, $R_2$ et $R_3$ répondant à la formule III (voir exemple no. 2).

Ce composé est obtenu par condensation du composé no. 1 (voir exemple 1) avec l'isothiocyanate de 2,2,2-tris[5-(2,3,4,6-tétra-*O*-acétyl-1-thio-α-D-mannopyranosyl)-2-oxapentyl]éthyle suivie d'une hydrolyse alcaline du groupement protecteur acétyle.

**1. Préparation du 2,2,2-tris[5-(2,3,4,6-tétra-*O*-acétyl-1-thio-α-D-manno pyranosyl)-2-oxapentyl]éthyl isothiocyanate.**

Ce composé est préparé en effectuant les étapes suivantes :

- **a) Préparation du 2,2,2-tris[5-(2,3,4,6-tétra-*O*-acétyl-1-thio-α-D-manno pyranosyl)-2-oxapentyl]éthanol.** Une solution de tri-*O*-allylpentaérythritol (0,32g ; 1,3 mmol) et de 2,3,4,6-tétra-*O*-acétyl-1-thio-α-D-mannopyranose (2,12 g; 5,85 mmol; 1,5 équiv) dans le méthanol anhydre dégazé et sous argon est irradiée par la lumière ultraviolette à température ambiante pendant 6 h. Le solvant est évaporé et le résidu est purifié par chromatographie sur colonne de gel de silice avec l'acétate d'éthyle-éther de pétrole 1:2. On obtient ainsi 0,81 g (rendement 46%) d'un solide blanc, présentant les caractéristiques suivantes :

    - $[\alpha]_D$ + 84,6° (*c* 1,0 ; dichlorométhane)
    - spectre de masse ($FAB^+$) : *m/z* 1371 (100%, $[M + Na]^+$)

- **b) Préparation du 2,2,2-tris[5-(2,3,4,6-tétra-*O*-acétyl-1-thio-α-D-manno pyranosyl)-2-oxapentyl]éthyl azide.** A une solution du 2,2,2-tris[5-(2,3,4,6-tétra-*O*-acétyl-1 -thio-α-D-mannoppanosyl) -2-oxapentyl]éthanol (1,15 g ; 0,85 mmol) dans le dichlorométhane (5,5 mL) on ajoute la pyridine (402 μL) et l'anhydride trifluorométhanesulfonique (177 μL). Le mélange réactionnel est agité pendant 20 min à -25°C, lavé par une solution saturée froide d'hydrogénocarbonate de sodium, séché par le sulfate de magnésium anhydre et concentré. Le résidu est repris par la *N,N*-diméthylformamide et additionné d'azidure de sodium (166 mg; 2,55 mmol ; 3 équiv). La suspension est agitée à la température ambiante pendant 3 heures et concentrée. Le résidu est repris par le dichlorométhane, lavé par l'eau, séché par le sulfate de magnésium anhydre, concentré et purifié par chromatographie sur colonne de gel de

silice avec l'acétate d'éthyle-éther de pétrole 1:2. On obtient ainsi 1,0 g d'un solide blanc (rendement 86%) ayant les caractéristiques suivantes :

- $[\alpha]_D$+ 83,2° (*c* 0,7 ; dichlorométhane)
- spectre de masse ($FAB^+$) : *m*/*z* 1396 (100%, $[M + Na]^+$)

- **c) Préparation du 2,2,2-tris[5-(2,3,4,6-tétra-*O*-acétyl-1-thio-α-D-manno pyranosyl)-2-oxapentyl]éthyl isothiocyanate.**
A une solution de 2,2,2-tris[5-(2,3,4,6-tétra-*O*-acétyl-1-thio-α-D-mannopyranosyl) -2-oxapentyl]éthyl azide (0,15 g ; 0,11 mmol) dans le dioxane (10 mL), on ajoute la triphénylphosphine (32 mg ; 0,12 mmol ; 1,1 équiv) et le disulfure de carbone (0,1 mL; 1,1 mmol, 10 équiv) et le mélange réactionnel est agité à température ambiante pendant 24 h. Le solvant est alors évaporé et le résidu est purifié par chromatographie sur colonne de gel de silice avec l'acétate d'éthyle-éther de pétrole 1:1. On obtient ainsi 0,13 g (rendement 85%) d'un solide blanc ayant les caractéristiques suivantes :

- $[\alpha]_D$+61,9° (*c* 0,4 ; dichlorométhane)
- spectre de masse ($FAB^+$) : *m*/*z* 1412 (100%, $[M + Na]^+$)

**2. Préparation du composé no. 4.**

Ce composé est obtenu par condensation du composé no. 1 (20 mg, 11,1 μmol) avec le 2,2,2-tris[5-(2,3,4,6-tétra-*O*-acétyl-1-thio-α-D-mannopyranosyl)-2-oxapentyl] éthyl isothiocyanate (160 mg, 115 μmol, 1,5 équiv) dans l'eau-acétone (1:2, 3 mL) à pH 8-9 (hydrogènocarbonate de sodium solide) comme décrit ci-dessus pour la préparation du composé n° 3. Le mélange réactionnel est agité pendant 48 h à température ambiante, l'acétone est évaporée et la phase aqueuse est extraite par l'acétate d'éthyle (3 x 5 mL). La phase organique est alors séchée par le sulfate de magnésium anhydre et concentrée. Le résidu est purifié par chromatographie sur colonne de gel de silice avec un gradient acétonitrile-eau 20:1 à 10:1. Le produit résultant est désacétylé et déminéralisé comme décrit ci-dessus pour la préparation du composé no. 3. Après lyophilisation, on obtient le composé no. 4 (50 mg, 60%) ayant les caractéristiques suivantes :

- $[\alpha]_D$+ 104,0° (*c* 1,0 ; eau)
- spectre de masse (MALDITOF) : *m*/*z* 7751,88 ($[M + H]^+$)
- solubilité dans l'eau : 800 $g.L^{-1}$ (103 $mmol.L^{-1}$)
- données de $^1H$ RMN (500 MHz, 353 K, $D_2O$) : δ 5,82 (21 H, s, H-1') ; 5,61 (7 H, bs, H-1) ; 4,57 (21 H, m, H-2') ; 4,46 (7 H, m, H-5) ; 4,43 (28 H, m, H-3, H-5') ; 4,36 (63 H, m, H-3', H-6", H-6b') ; 4,29 (21 H, m, H-4') ; 4,23 (14 H, m, $CH_2N_{cyst}$) ; 4,19 (7 H, m, H-2) ; 4,09 (49 H, m, H-4, H-$3_{pent}$) ; 3,96 (56 H, m, $CH_2N_{branche}$, H-$1_{Pent)}$; 3,82 (7 H, m, H-6a) ; 3,50 (7 H, m, H-6b) ; 3,47 (14 H, m, $CH_2S_{cyst}$); 3,26 (42 H, bt, $J_{5a,5b}$ 11,5 Hz, H-$5_{Pent}$ ; 2,46 (42 H, m, H-$4p_{ent}$)
- données de $^{13}C$ NMR (125,7 MHz, 343 K, $D_2O$) : δ181,9 (CS) ; 102,7 (C-1) ; 85,3 (C-1') ; 85,1 (C-4) ; 73,9 (C-5') ; 73,4 (C-3) ; 72,9 (C-2, C-5) ; 72,6 (C-2') ; 72,1 (C-3'); 71,1 (C-$1_{pent}$ ; 70,9 (C-$3_{pent}$) ; 67,7 (C-4') ; 61,6 (C-6'); 46,6 ($CH_2N_{cyst}$, CH2$N_{branche}$) ; 45,0 (Cq) ; 34,1 (C-6) ; 33,2 ($CH_2S_{cyst}$) ; 29,8 (C-$4_{pent}$) ; 28,5 (C-$5_{pent}$)

**Exemple 5: Préparation de l'heptakis[6-*S*-[2-*N*'-[2,22-tris[5-(1-thio-β-D-glucopyranosyl)-2-oxapentyl]éthyl] thiouréido]ethyl-6-thio]cyclomaltoheptaose (composé no. 5)**

Ce composé répond à la formule (V) donnée ci-dessus avec n = 1, m = 6, dans laquelle tous les $R^1$ sont identiques et représentent $CH_2$-C[$(CH_2OCH_2CH_2CH_2R_2)_2$($CH_2OCH_2CH_2CH_2R_2$)], X représente un atome d'hydrogène, $R_2$ et $R_3$ répondent à la formule:

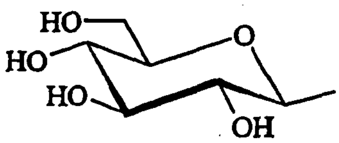

(VI)

Ce composé est obtenu par condensation du composé no. 1 (voir Exemple 1) avec le 2,2,2-tris[5-(2,3,4,6-tétra-*O*-acétyl-1-thio-β-D-glucopyranosyl)-2-oxapentyl]éthyl isothiocyanate suivi d'une hydrolyse alcaline du groupement protecteur acétyle.

**1. Préparation du 2,2,2-tris[5-(2,3,4,6-tetra-*O*-acetyl-1-thio-α-D-manno pyranosyl)-2-oxapentyl]éthyl isothiocyanate.**

Ce composé est préparé par les étapes suivantes :

- **a) Préparation du 2,2,2-tris[5-(2,3,4,6-tetra-*O*-acetyl-1-thio-β-D-glucopyranosyl)-2-oxapentyl]éthanol.** Ce composé est obtenu à partir du tri-*O*-allylpentaérythritol (0,32g ; 1,3 mmol) et du 2,3,4,6-tétra-*O*-acétyl-1-thio-β-D-glucopyranose (2,12 g ; 5,85 mmol ; 1,5 équiv) par irradiation avec la lumière ultraviolette (250 nm) comme décrit ci-dessus pour la préparation du 2,2,2-tris [5-(2,3,4,6-tétra-*O*-acétyl-1-thio-α-D-mannopyranosyl)-2-oxapentyl) éthanol. On obtient ainsi 1,23 g (rendement 70%) d'un solide blanc, présentant les caractéristiques suivantes :

  - $[\alpha]_D$-22,8° (*c* 0,8 ; dichlorométhane)
  - spectre de masse ($FAB^+$) : *m*/*z* 1371 (100%, $[M + Na]^+$)

- **b) Préparation du 2,2,2-tris[5-(2,3,4,6-tétra-*O*-acétyl-1-thio-β-D-gluco pyranosyl)-2-oxapentyl]éthyl azide.** Ce composé est obtenu à partir du 2,2,2-tris[5-(2,3,4,6-tétra-*O*-acétyl-1-thio-β-D-glucopyranosyl)-2-oxapentyl]éthanol (1,15 g ; 0,85 mmol) par réaction avec l'anhydride trifluorométhanesulfonique suivi de traitement par l'azidure de sodium comme décrit ci-dessus pour la préparation de la 2,2,2-tris[5-(2,3,4,6-tétra-*O*-acétyl-1-thio-α-D-mannopyranosyl)-2-oxapentyl]éthyl azide. On obtient ainsi 0,73 g d'un solide blanc (rendement 60%) ayant les caractéristiques suivantes :

  - $[\alpha]_D$+78,9°(*c* 0,9 ; dichlorométhane)
  - spectre de masse ($FAB^+$) :*m*/*z* 1396 (100%, $[M + Na]^+$)

- **c) Préparation du 2,2,2-tris[5-(2,3,4,6-tétra-*O*-acétyl-1-thio-β-D-gluopyranosyl)-2-oxapentyl]éthyl isothiocyanate.** Ce composé est obtenu par isothiocyanation de la 2,2,2-tris[5-(2,3,4,6-tétra-*O*-acétyl-1-β-D-glucopyranosyl)-2-oxapentyl]éthyl azide (0,32 g ; 0,23 mmol) par traitement avec la triphénylphosphine (67,1 mg ; 0,26 mmol ; 1,1 équiv) et le disulfure de carbone (0,15 mL ; 2,33 mmol ; 10 équiv) dans le dioxane (10 mL), comme décrit ci-dessus pour la préparation du 2,2,2-tris[5-(2,3,4,6-tétra-*O*-acétyl-1-thio-β-D-mannopyranosyl)-2-oxapentyl]éthyl isothiocyanate. On obtient ainsi 0,26 g (rendement 80%) d'un solide blanc ayant les caractéristiques suivantes :

  - $[\alpha]_D$+ 61,9° (*c* 0,4 ; dichlorométhane)
  - spectre de masse ($FAB^+$): *m*/*z* 1412 (100%, $[M + Na]^+$)

**2. Préparation du composé n° 5.**

Ce composé est obtenu par condensation du composé no. 1 (20 mg ; 11,1 μmol) et du 2,2,2-tris[5-(2,3,4,6-tétra-*O*-acétyl-1-thio-β-D-glucopyranosyl)-2-oxapentyl]éthyl isothiocyanate (160 mg; 115 μmol ; 1,5 équiv) dans l'eau-acétone (1:2, 3 mL) à pH 8-9 (hydrogénocarbonate de sodium solide) suivi de désacétylation comme décrit ci-dessus pour la préparation du composé no. 4. Après lyophilisation, on obtient le composé no. 5 (56 mg, 68%) ayant les caractéristiques suivantes :

- [α] -16.2° (*c* 1,0 ; eau)
- spectre de masse (MALDITOF) : *m*/*z* 7751,88 ($[M + H]^+$)
- solubilité dans l'eau: 670 $g.L^{-1}$(87 $mmol.L^{-1}$)
- données de $^1H$ RMN (500 MHz, 343 K, $D_2O$): δ 5,46 (7 H, bd, $J_{1,2}$ 3,0 Hz, H-1) ; 4,86 (21 H, d, $V_{1,2}$ 9,5 Hz, H-1') ; 4,29 (7 H, m, H-5) ; 4,28 (7 H, m, H-3) ; 4,27 (21 H, d, $J_{6a,6b'}$ 12,0 Hz, H-6a') ; 4,13 (21 H, d, H-6b') ; 4,09 (14 H, m, $CH_2$Ncyst) ; 4,07 (7 H, m, H-2) ; 3,98 (42 H, t, $^3J_{H,H}$ 6,5 Hz, H-$3_{pent}$); 3,97 (7 H, m, H-4) ; 3,89 (21 H, t, $J_{2',3}$ = $J_{3',4'}$ = 9,3 Hz, H-3') ; 3,84 (98 H, m, H-4', H-5', $CH_2N_{branche}$, H-$1_{pent}$) ; 3,73 (21 H, t, H-2') ; 3,65 (7 H, m, H-6a) ; 3,38 (7 H, m, H-6b) ; 3,32 (14 H, bt, $^3J_{H,H}$ = 6,5 Hz, $CH_2S_{cysc}$) ; 3,22 ; 3,18 (42 H, 2 dt, $J_{5a,5b}$ 13,0 Hz, $J_{4,5}$ 5,5 Hz, H-$5_{pent}$) ; 2,33 (42 H, m, H-$4_{pent}$
- données de $^{13}C$ RMN (125,7 MHz ; 343 K ; $D_2O$) : δ 181,4 (CS), 102,6 (C-1) ; 86,3 (C-1') ; 85,1 (C-4) ; 80,5 (C-5') ; 78,0 (C-3'); 73,4 (C-3) ; 73,2 (C-2') ; 72,8 (C-2, C-5); 71,2 (C-$1_{pent}$); 70,4 (C-$3_{Pent)}$ ; 70,3 (C-4'); 61,8 (C-6'); 46,5 ($CH_2N_{cyst}$, $CH_2N_{branche}$) ; 45,0 ($C_q$) ; 34,2 (C-6) ; 33,1 ($CH_2S_{cyst}$) ; 30,0 (C-$4_{Pent}$) ; 27,4 (C-$5_{Pent}$)

**Exemple 6: Préparation de l'heptakis[6-S-[2-*N'*-[2-[5-(1-thio-α-D-manno pyranosyl)-2-oxapentyl]-2,2-bis [5-(1-thio-β-D-glucopyranosyl)-2-oxapentyl]éthyl] thiouréido]éthyl-6-thio]cyclomaltoheptaose (composé no. 6)**

Ce composé répond à la formule (V) donnée ci-dessus avec n = 1, m = 6, dans laquelle tous les $R_1$ sont identiques et représentent $CH_2-C[(CH_2OCH_2CH_2CH_2R_2)_2(CH_2OCH_2CH_2CH_2R_3)]$, X représente un atome d'hydrogène, $R_2$ et $R_3$ répondant aux formules (VI) et (III), respectivement.

Ce composé est obtenu par condensation du composé no. 1 (voir Exemple 1) avec le 2-[5-(2,3,4,6-tétra-*O*-acétyl-1-thio-α-D-mannopyranosyl)-2-oxapentyl]-2,2-bis[5-(2,3,4,6-tétra-*O*-acetyl-1-thio-β-D-glucopyranosyl)-2-oxapentyl]éthyl isothiocyanate suivi d'une hydrolyse basique du groupement protecteur acétyle.

**1. Préparation du 2-[5-(2,3,4,-tétra.*O*-acétyl-1-thio-α-D-mannopyranosyl)-2-oxapentyl]-2,2-bis[5-(2,3,4,6-tétra-*O*-acétyl-1-thio-β-D-glucopyranosyl)-2-oxa pentyl]éthyl isothiocyanate.**

Ce composé est préparé en effectuant les étapes suivantes :

- **a) Préparation du 2-(2-oxapent-4-enyl)-2,2-bis[5-(2,3,4,6-tétra-*O*-acétyl-1-thio-β-D-glucopyranoyl)-2-oxapentyl]éthanol**
  Une solution de tri-*O*-allylpentaérythritol (0,20g ; 0,78 mmol) et de 2,3,4,6-tétra-*O*-acétyl-1-thio-β-D-glucopyranose (0,85 g ; 2,34 mmol ; 1 équiv), dans le méthanol anhydre (15 mL) dégazé et sous argon, est irradiée par la lumière ultraviolette (250 nm) à température ambiante pendant 6 h. Le solvant est évaporé et le résidu est purifié par chromatographie sur colonne de gel de silice avec l'acétate d'éthyle-éther de pétrole 3:2. On obtient ainsi 0,58 g (rendement 76%) d'un solide blanc, présentant les caractéristiques suivantes :

  - $[\alpha]_D$ - 26,6° (*c* 0,8 ; dichlorométhane)
  - spectre de masse ($FAB^+$) : *m/z* 1007 (100%, $[M + Na]^+$)

- **b) Préparation du 2-[5-(2,3,4,6-tétra-O-acétyl-1.thio-α-D-manno pyranosyl)-2-oxapentyl]-2,2-bis [5-(2,3,4,6-tétra-O-acetyl-1-thio-β-D-gluco pyranosyl)-2-oxapentyl]éthanol**
  Une solution de 2-(2-oxapent-4-ényl)-2,2-bis[5-(2,3,4,6-tétra-*O*-acétyl-1-thio-β-D-glucopyranosyl)-2-oxapentyl] éthanol (0,53 g; 0,54 mmol) et de 2,3,4,6-tétra-*O*-acétyl-1-thio-α-D-mannopyranose (0,29 g ; 0,81 mmol ; 1,5 équiv), dans le méthanol anhydre (35 mL) dégazé et sous argon, est irradiée par la lumière ultraviolette (250 nm) à température ambiante pendant 6 h. Le solvant est évaporé et le résidu est purifié par chromatographie sur colonne de gel de silice avec l'acétate d'éthyle-éther de pétrole 3:1. On obtient ainsi 0,55 g (rendement 76%) d'un solide blanc, présentant les caractéristiques suivantes :

  - $[\alpha]_D$ +15,5° (*c* 1,0 ; dichlorométhane)
  - spectre de masse ($FAB^+$) : *m/z* 1371 (100%, $[M + Na]^+$)

- **c) Préparation du 2-[5-(2,3,4,6-tétra-O-acétyl-1-thio-α-D-manno pyranosyl)-2-oxapentyl]-2,2-bis[5-(2,3,4,6-tétra-O-acétyl-1-thio-β-D-gluco pyranosyl)-2-oxapentyl]éthyl azide**
  Ce composé est obtenu à partir de 2-[5-(2,3,4,6-tétra-*O*-acétyl-1-thio-α-D-mannopyranosyl)-2-oxapentyl]-2,2-bis [5-(2,3,4,6-tétra-*O*-acétyl-1-thio-β-D-gluco pyranosyl)-2-oxapentyl]éthanol (0,43 g ; 0,32 mmol) par réaction avec l'anhydride trifluorométhanesulfonique suivie de traitement par l'azidure de sodium comme décrit ci-dessus pour la préparation de la 2,2,2-tris[5-(2,3,4,6-tétra-*O*-acétyl-1-thio-α-D-mannopyranosyl)-2-oxapentyl]éthyl azide. On obtient ainsi 0,33 g d'un solide blanc (rendement 75%) ayant les caractéristiques suivantes :

  - $[\alpha]_D$+ 8,2° (*c* 1,0 ; dichlorométhane)
  - spectre de masse ($FAB^+$) : *m/z* 1396 (100%, $[M + Na]^+$)

- **d) Préparation du 2-[5-(2,3,4,6-tétra-O-acétyl-1-thio-α-D-manno pyranosyl)-2-oxapentyl]-2,2-bis[5-(2,3,4,6-tétra-O-acétyl-1-thio-β-D-gluco pyranosyl)-2-oxapentyl]éthyl isothiocyanate.**
  Ce composé est obtenu par isothiocyanation de la 2-[5-(2,3,4,6-tétra-*O*-acétyl-1-thio-α-D-mannopyranosyl)-2-oxapentyl]-2,2-bis[5-(2,3,4,6-tétra-*O*-acetyl-1-thio-β-D-glucopyranosyl)-2-oxapentyl]éthyl azide (0,25 g ; 0,18 mmol) par traitement avec la triphénylphosphine (52 mg; 0,20 mmol ; 1,1 équiv) et le disulfure de carbone (0,11 mL ; 1,80 mmol ; 10 équiv) dans le dioxane (8 mL), comme décrit ci-dessus pour la préparation du 2,2,2-tris[5-(2,3,4,6-tétra-*O*-acétyl-1-thio-β-D-mannopyranosyl)-2-oxapentyl]éthyl isothiocyanate. On obtient ainsi 0,20 g (rendement 80%) d'un solide blanc ayant les caractéristiques suivantes :

- $[\alpha]_D$+ 9,7° (*c* 1,0 ; dichlorométhane)
- spectre de masse ($FAB^+$) : *m*/*z* 1412 (100%, $[M + Na]^+$)

**2. Préparation du composé no. 6.**

Ce composé est obtenu par condensation du composé no. 1 (20 mg; 11,1 μmol) avec le 2-[5-(2,3,4,6-tétra-*O*-acétyl-1-thio-α-D-mannopyranosyl)-2-oxapentyl]-2,2-bis[5-(2,3,4,6-tétra-*O*-acétyl-1-thio-p-D-glucopyranosyl)-2-oxapentyl]éthyl isothiocyanate (160 mg ; 115 μmol ; 1,5 équiv) dans l'eau-acétone (1:2, 3 mL) à pH 8-9 (hydrogénocarbonate de sodium solide) suivi de désacétylation comme décrit ci-dessus pour la préparation du composé no. 4. Après lyophilisation, on obtient le composé no. 6 (48 mg, 60%) ayant les caractéristiques suivantes :

- $[\alpha]_D$+ 25,0° (*c* 1,0 ; eau)
- spectre de masse (MALDTTO) : *m*/*z* 7751,88 ($[M + H]^+$)
- solubilité dans l'eau : 700 $g.L^{-1}$ (91 $mmol.L^{-1}$)
- données de $^1H$ RMN (500 MHz, 353 K, $D_2O$) : δ 5,87 (7 H, s, H-$1'_{man}$); 5,60 (7 H, bd, $J_{1,2}$ 3,5 Hz, H-1) ; 5,00 (14 H, d, $J_{1',2'}$ 10,0 Hz, H-$1'_{Glc}$) ; 4,57 (7 H, m, H-$2'_{man}$) ; 4,42 (7 H, m, H-$5'_{Man)}$ ; 4,41 (7 H, m, H-5) ; 4,39 (14 H, d, $J_{6a',6b'}$ 12,0 Hz, H-$6a'_{Glc}$) ; 4,38 (7 H, m, H-3) ; 4,36 (21 H, m, H-$3'_{Man,}$ H-$6a'_{Man}$, H-$6b'_{Man}$) ; 4,29 (7 H, m,' H-$4'_{man}$) ; 4,25 (14 H, dd, $J_{5',6b'}$ 4,5 Hz, H-$6b'_{Glc}$) ; 4,22 (14 H, m, $CH_2N_{cyst}$) ; 4,17 (7 H, d, H-2) ; 4,09 (42 H, t, $J_{3,4}$ 5,5 Hz, H-$3_{pent}$) ; 4,08 (7 H, m, H-4) ; 4,02 (14 H, t, $J_{2',3'} = J_{3',4'}$ 9,4 Hz, H-$3'_{Glc}$) 3,96 (84 H, m, H-$4'_{Glc}$, H-$5'_{Glc,}$ $CH_2NH_{branche,}$ H-$1_{Pent}$) 3,85 (14 H, t, $J_{1',2'}$ 9,4 Hz, H-$2'_{Glc}$) ; 3,78 (7 H, m, H-6a) ; 3,51 (7 H, m, H-6b) ; 3,46 (14 H, bt, $^3J_{H,H}$ 6,5 Hz, $CH_2S_{cyst}$) ; 3,34 ; 3,32 (42 H, 2 dt, $J_{5a,5b}$13,0 Hz, $J_{4,5}$ 6,0 Hz, H-$5_{pent}$) ; 2,45 (42 H, m, H-$4_{Pent}$)
- données de $^{13}C$ RMN (125,7 MHz ; 353 K ; $D_2O$) : δ 181,9 (CS) ; 102,8 (C-1) ; 86,3 (C-1 $'_{Glc}$) ; 86,0 (C-$1'_{Man}$) ; 85,3 (C-4) ; 80,7 (C-$5'_{Glc}$) ; 78,3 (C-$3'_{Glc}$); 74,1 (C-$5'_{Man}$) ; 73,6 (C-3) ; 73,4 (C-$2'_{Glc}$) ; 73,0 (C-2, C-5) ; 72,8 (C-$2'_{Man}$) ; 72,3 (C-3' $_{Man}$) ; 71,4 (C-$1_{Pent}$) ; 71,0 (C-$3p_{ent}$) ; 70,6 (C-$4'_{Glc}$) ; 67,9 (C-$4'_{Man}$) ; 62,0 (C-$6'_{Glc}$) ; 61,7 (C-$6'_{Man}$) ; 45,1 (Cq) ; 45,0 ($CH_2N_{cyst,}$ $CH_2N_{branche}$) ; 34,5 (C-6) ; 33,3 ($CH_2S_{cyst}$ ; 30,4 ; 30,0 (C-$4_{pent}$) ; 28,7 ; 27,6 (C-$5_{Pent}$)

**Exemple 7: Préparation de l'heptakis[6-S-[2-*N'*-[2,2-bis[5-(1-thio-α-D-mannopyranosyl)-2-oxapentyl]-2-[5-(1-thio-β-D-glucopyranosyl)-2-oxapentyl]éthyl] tbiouréido]étbylthio]cyclomaltoheptaose (composé no. 7)**

Ce composé répond à la formule (V) donnée ci-dessus avec n = 1, m = 6, dans laquelle tous les $R_1$ sont identiques et représentent $CH_2-C[(CH_2OCH_2CH_2CH_2R_2)_2(CH_2OCH_2CH_2CH_2R_3)]$, X représente un atome d'hydrogène, $R_2$ et $R_3$ répondant aux formules (III) et (VI), respectivement.

Ce composé est obtenu par condensation du composé no. 1 (voir Exemple 1) avec le 2,2-bis[5-(2,3,4,6-tétra-*O*-acétyl-1-thio-α-D-mannopyranosyl)-2-oxapentyl]-2-[5-(2,3,4,6-tétra-*O*-acétyl-1-thio-β-D-glucopyranosyl)-2-oxapentyl]étyl isothiocyanate suivie d'une hydrolyse alcaline du groupement protecteur acétyle.

**1. Préparation du 2,2-bis[5-(2,3,4,6-tétra-O-acétyl-1-thio-α-D-manno pyranosyl)-2-oxapentyl]-2-[5-(2,3,4,6-tétra-O-acétyl-1-thio-β-D-gluco pyranosyl)-2-oxapentyl]éthyl isothiocyanate.**

Ce composé est préparé par les étapes suivantes :

- **a) Préparation du 2-(2-oxapent-4-ényl)-2,2-bis[5-(2,3,4,6-tétra-O-acétyl-1-thio-α-D-mannopyranosyo-2-oxapentyl]éthanol**

  Une solution de tri-*O*-allylpentaérythritol (0.20 g, 0.78 mmol) et 2,3,4,6-tétra-*O*-acétyl-1-thio-α-D-mannopyranose (0,85 g; 2,34 mmol; 1 équiv) dans le méthanol anhydre (15 mL), dégazé et sous argon, est irradiée par la lumière ultraviolette (250 nm) à température ambiante pendant 6 h. Le solvant est évaporé et le résidu est purifié par chromatographie sur colonne de gel de silice avec l'acétate d'éthyle-éther de pétrole 1:1. On obtient ainsi 0,55 g (rendement 72%) d'un solide blanc, présentant les caractéristiques suivantes :

  - $[\alpha]_D$+ 20,2° (*c* 0,8 ; dichlorométhane)
  - spectre de masse (FAB+): *m*/*z* 1007 (100%, $[M + Na]^+$)

- **b) Préparation du 2,2-bis[5-(2,3,4,6-tétra-O-acétyl-1-thio-α-D-manno pyranosyl)-2-oxapentyl]-2-[5-(2,3,4,6-tétra-O-acetyl-1-thio-β-D-gluco pyranosyl)-2-oxapentyl]éthanol**

  Une solution de 2-(2-oxapent-4-enyl)-2,2-bis[5-(2,3,4,6-tétra-*O*-acetyl-1-thio-α-D-mannopyranosyl)-2-oxapentyl] éthanol (0,49 g; 0,49 mmol) et de 2,3,4,6-tétra-*O*-acétyl-1-thio-β-D-glucopyranose (0,27 g ; 0,66 mmol ; 1,5 équiv), dans le méthanol anhydre (25 mL) dégazé et sous argon, est irradiée par la lumière ultraviolette (250 nm) à température ambiante pendant 6 h. Le solvant est évaporé et le résidu est purifié par chromatographie sur colonne de

gel de silice avec l'acétate d'éthyle-éther de pétrole 3:1. On obtient ainsi 0,42 g (rendement 64%) d'un solide blanc, présentant les caractéristiques suivantes :

- $[\alpha]_D$+ 29,4° (*c* 0,6 ; dichlorométhane)
- spectre de masse ($FAB^+$) : *m/z* 1371 (100%, $[M + Na]^+$)

- **c) Préparation du 2,2-bis[5-(2,3,4,6-tétra-O-acetyl-1-thio-α-D-manno pyranosyl)-2-oxapentyl]-2-[5-(2,3,4,6-tétra-O-acetyl-1-thio-β-D-gluco pyranosyl)-2-oxapentyl]éthyl azide**
Ce composé est obtenu à partir du 2,2-bis[5-(2,3,4,6-tétra-*O*-acétyl-1-thio-α-D-mannopyranosyl)-2-oxapentyl]-2-[5-(2,3,4,6-tétra-*O*-acétyl-1-thio-β-D-glucopyranosyl)-2-oxapentyl]éthanol (0,688 g; 0,51 mmol), par réaction avec l'anhydride trifluorométhanesulfonique suivi de traitement par l'azidure de sodium comme décrit ci-dessus pour la préparation de la 2,2,2-tris[5-(2,3,4,6-tétra-*O*-acétyl-1-thio-α-D-mannopyranosyl)-2-oxapentyl]éthyl azide. On obtient ainsi 0,554 g d'un solide blanc (rendement 79%) ayant les caractéristiques suivantes :

- $[\alpha]_D$+ 45,3° (*c* 1,0 ; dichlorométhane)
- spectre de masse ($FAB^+$) : *m/z* 1396 (100%, $[M + Na]^+$)

- **d) Préparation du 2,2-bis[5-(2,3,4,6-tétra-O-acétyl-1-thio-α-D-manno pyranosyl)-2-oxapentyl]-2-[5-(2,3,4,6-tétra-O-acétyl-1-thio-β-D-gluco pyranosyl)-2-oxapentyl]éthyl isothiocyanate**
Ce composé est obtenu par isothiocyanation du 2,2-bis[5-(2,3,4,6-tétra-*O*-acétyl-1-thio-α-D-mannopyranosyl)-2-oxapentyl]-2,2-bis[5-(2,3,4,6-tétra-*O*-acétyl-1-thio-β-D-glucopyranosyl)-2-oxapentyl]éthyl azide (131 mg; 0,095 mol) par traitement avec la triphénylphosphine (28 mg ; 0,10 mmol ; 1,1 équiv) et le disulfure de carbone (60 μL ; 0,95 mmol ; 10 équiv) dans le dioxane (5 mL), comme décrit ci-dessus pour la préparation du 2,2,2-tris[5-(2,3,4,6-tétra-*O*-acétyl-1-thio-β-D-marmopyranosyl)-2-oxapentyl]éthyl isothiocyanate. On obtient ainsi 95 mg (rendement 72%) d'un solide blanc ayant les caractéristiques suivantes :

- $[\alpha]_D$+ 40,8° (*c* 1,0 ; dichlorométhane)
- spectre de masse ($FAB^+$) : *m/z* 1412 (100%, $[M + Na]^+$

**2. Préparation du composé n° 7.**

Ce composé est obtenu par condensation du composé no. 1 (20 mg ; 11,1 μmol) dans l'eau-acétone (1:2; 3 mL) avec le 2,2-bis[5-(2,3,4,6-tétra-*O*-acétyl-1-thio-α-D-mannopyranosyl)-2-oxapentyl]-2-[5-(2,3,4,6.tétra-*O*-acetyl-1-thio-β-D-glucopyranosyl)-2-oxapentyl]éthyl isothiocyanate (160 mg; 115 μmol ; 1,5 équiv), dans l'eau-acétone (1:2, 3 mL) à pH 8-9 (hydrogénocarbonate de sodium solide), suivie de désacétylation comme décrit ci-dessus pour la préparation du composé no. 4. Après lyophilisation, on obtient le composé no. 7 (40 mg, 52%) ayant les caractéristiques suivantes :

- $[\alpha]_D$+ 66,0° (*c* 1,0 ; eau)
- spectre de masse (MALDITOF) : *m/z* 7751,88 ($[M + H]^+$)
- solubilité dans l'eau : 800 $g.L^{-1}$ (103 $mmol.L^{-1}$)
- données de $^1H$ RMN (500 MHz, 353 K, $D_2O$): δ 5,67 (14 H, s, H-1'$_{Man}$) ; 5,47 (7 H, bs, H-1) ; 4,86 (7 H, d, $J_{1',2}$ 10,0 Hz, H-1'$_{Glc}$) ; 4,43 (14 H, m, H-2'$_{Man}$) ; 4,29 (21 H, m, H-5'$_{Man}$, H-5) ; 4,26 (7 H, m, H-3) ; 4,25 (7 H, d, $J_{6a'6b'}$ 12,0 Hz, H-6a'$_{Glc}$) ; 4,14 (42 H, m, H-3'$_{Man}$, H-6a'$_{Man}$, H-6b'$_{Man}$) ; 4,14 (14 H, m, H-4'$_{Man}$) ; 4,09 (7 H, d, H-6b'$_{Glc}$) ; 4,08 (14 H, m, $CH_2N_{cyst}$) ; 4,04 (7 H, d, H-2) ; 3,95 (49 H, m, H-3$_{pent}$, H-4) ; 3,85 (7 H, t, $J_{2'3'}$ = $J_{3',4'}$ 8,9 Hz, H-3' $_{Glc}$) ; 3,80 (70 H, m, H-4'$_{Glc}$, H-5'$_{Glc}$, $CH_2NH_{brancheb}$, H-1$_{pent}$) ; 3,71 (7 H, t, H-2'$_{Glc}$) ; 3,61 (7 H, m, H-6a) ; 3,37 (7 H, m, H-6b) ; 3,32 (14 H, m, $CH_2S_{cyst}$) ; 3,17 ; 3,12 (42 H, 2 dt, $J_{5a,5b}$ 13,5 Hz, $J_{4,5}$ 6,5 Hz, H-5$_{Pent}$) ; 2,30 (42 H, t, $J_{3,4}$ 6,5 Hz, H-4$_{pent}$)
- données de $^{13}C$ RMN (125,7 MHz, 353 K, $D_2O$) : δ 182,0 (CS) ; 102,7 (C-1) ; 86,1 (C-1'$_{Glc}$) ; 85,7 (C-1'$_{Man}$) 85,2 (C-4) 80,9 (C-5'$_{Glc}$) 78,0 (C-3'Glc) ; 73,9 (C-5'$_{Man}$) ; 73,1 (C-3) ; 73,0 (C-2'$_{Glc}$); 72,8 (C-2, C-5) ; 72,6 (C-2'$_{Man}$) ; 72,0 (C-3'$_{Man}$); 71,1 (C-1$_{pent}$); 70,8 (C-3$_{pent}$) 70,3 (C-4'$_{Glc}$) ; 67,7 (C-4'$_{Man}$) ; 61,8 (C-6'$_{Glc}$); 61,5 (C-6'$_{Man}$) ; 45,1 ($CH_2N_{cyst,}$ $CH_2N_{branche}$) ; 45,0 ($C_q$) ; 34,2 (C-6) ; 33,1 ($CH_2S$) ; 30,2 ; 29,3 (C-4$_{pent}$) ; 28,5 ; 27,5 (C-5$_{Pent}$)

**Exemple 8 : Evaluation de l'affinité des thiouréidocystéaminyl-cyclodextrines composés no. 2 à 7 pour la lectine spécifique de mannose concanavaline A (ConA)**

On a évalué l'affinité des composés no. 2 à 7 pour la lectine spécifique de, mannose concanavaline A (ConA) suivant le protocole ELLA. On obtient ainsi la concentration des composés no. 2 à 7 nécessaire pour inhiber à 50%

l'association de la ConA à un ligand de référence (le mannane de levure dans notre cas) fixé sur la cellule d'une plaque de microtitration ($IC_{50}$). Les valeurs d'$IC_{50}$ sont inversement proportionnelles aux affinités respectives.

Les cellules d'une plaque de microtitration (Nunc-Inmuno™ plates, MaxiSorp™) sont chargées avec 100 μL d'une solution mère de mannane de levure (Sigma, 10 μg.mL$^{-1}$) dans une solution tampon phosphate saline (PBS ; pH 7,3 contenant $Ca^{2+}$ 0;1 mM et $Mn^{2+}$ 0,1 mM) pendant une nuit à température ambiante. Les cellules sont lavées (3 x 300 μL) par une solution tampon contenant 0,05% (v/v) de Tween 20 (PBST). Ce protocole de lavage est répété après chaque incubation pendant l'essai. Les cellules sont alors additionnées d'une solution d'albumine du sérum bovin (BSA, 1%) dans le PBS (150 μL/cellule) suivi d'incubation pendant 1 h à 37°C, puis lavées.

Pour déterminer la concentration de lectine optimale pour les études d'inhibition, on ajoute dans les cellules chargées avec le mannane, et traitées comme indiqué ci-dessus 100 μL d'une série de solutions de concanavaline A marquée avec la peroxydase de radis de $10^{-1}$ à $10^{-5}$ mg.mL$^{-1}$ dans le PBS. Après incubation à 37°C pendant 1 h, les plaques sont lavées (PBST) et additionnées d'une solution (50 μL) du sel de diammonium de l'acide 2,2'-azinobis (3-éthylbenzothiazoline-6-sulfonique) (ABTS, 1 mg dans 4 mL) dans le tampon citrate (0,2 M, pH 4,0 avec 0,015% d'eau oxygénée). La réaction est arrêtée après 20 min par addition de 50 μL/cellule d'acide sulfurique 1 M et les absorbances sont mesurées à 415 nm utilisant un lecteur ELISA. Les cellules témoin contenaient le tampon citrate-phosphate. La concentration de lectine marquée avec la peroxydase donnant une valeur d'absorbance entre 0,8 et 1,0 (typiquement entre $10^{-2}$ et $10^{-3}$ mg.mL$^{-1}$) a été utilisée dans les essais d'inhibition.

Pour les essais d'inhibition, on a utilisé des solutions mères des composés no. 2 à 7 à une concentration de 5 à 7 mg.mL$^{-1}$ dans le PBS. Dans une série d'expériences, les solutions de chaque composé (60 μL/cellule) dans le PBS, diluées séquentiellement au double, sont additionnées de ConA marquée à la peroxydase de radis de concentration appropriée comme indiqué ci-dessus (60 μL/cellule) dans une plaque de microtitration Nunclon™ (Delta) qui est incubée à 37°C pendant 1 h. Les solutions (100 μL/cellule) sont alors transférées sur une plaque de microtitration chargée avec le mannane et traitée comme indiqué ci-dessus, laquelle est ensuite incubée à 37°C pendant 1 h. Les cellules sont lavées (PBST) et additionnées de la solution de l'ABTS (50 μL/cellule). Après 20 min, la réaction est arrêtée (acide sulfurique) et les absorbances sont mesurées. Le pourcentage d'inhibition est calculé par la formule :

$$\%\ \text{d'inhibition} = \frac{A_{\text{en absence d'inhibiteur}}}{A_{\text{en présence d'inhibiteur}}} \times 100$$

Dans le Tableau 1 qui suit, on donne les valeurs d'$IC_{50}$ pour les composés n° 2 à 7 (moyenne de trois expériences indépendantes) en comparaison avec la valeur correspondante pour le méthyl-α-D-glucopyranoside, utilisé comme ligand monovalent de référence. On observe une augmentation importante de l'affinité pour la lectine dans le cas des dérivés hyperramifiés comportant des substituants mannopyranosyle.

**Tableau 1.** Donnés ELLA pour l'inhibition de l'association entre le mannane de levure et la lectine ConA marquée par la peroxydase de radis.

| | Composé | | | | | | |
|---|---|---|---|---|---|---|---|
| | Me-α-$_{D}$-Glc*p* | No. 2 | No. 3 | No. 4 | No. 5 | No. 6 | No. 7 |
| $IC_{50}$ (μM) | 865 | 175 | 21 | 5 | n.i. [a] | 18 | 13 |
| Affinité relative | 1 | 4,9 | 41 | 173 | --- | 48 | 67 |
| Affinité molaire relative | 1 | 0,7 | 2,0 | 8,2 | --- | 6,9 | 4,8 |

[a] Pas d'inhibition significative détectée à la concentration de 5 mM.

La Figure 1 représente la variation de l'inhibition de l'association entre la lectine ConA et le mannane de levure par les composés n°2, n°3, n°4, n°6 et n°7, du type thiouréidocystéaminyl-cyclodextrine, ainsi que pour l'heptakis(6-désoxy-6-α-D-mannopyranosylthiouréido)cyclomaltoheptaose, un dérivé heptavalent du type thiouréido-cyclodextrine décrit dans le document *ChemBioChem* 2001, en fonction de la concentration du ligand mannosylé. Une comparaison des courbes pour le composé n°2 et pour le dérivé de la per-(C-6)-amine montre une augmentation remarquable de

l'affinité pour la lectine du fait de l'introduction de l'espaceur cystéaminyle. On observe, par ailleurs, une augmentation beaucoup plus importante de l'affinité lorsque les ligands mannopyranosyle sont incorporés dans une structure du type hyperramifié (voir courbes pour les composés n°3, n°4, n°6 et n°7).

Ainsi, la concentration du composé no. 2 nécessaire pour inhiber 50% de l'association entre la concanavaline A et le mannane de levure fixé sur la microplaque ($IC_{50}$), est de 175 μM, alors que pour l'heptakis(6-désoxy-6-α-D-mannopyranosylthiouréido)cyclomaltoheptaose, un dérivé heptavalent du type thiouréido-cyclodextrine, l'inhibition à la même concentration n'atteint que 8% de la valeur précédente. L'efficacité du phénomène de reconnaissance est encore beaucoup plus élevée pour les thiouréidocystéaminyl-cyclodextrines hyperramifiées. Ainsi, les valeurs d'$IC_{50}$ pour les composés n° 3, n° 4, n° 6 et n° 7 sont comprises entre 5 et 21 μM, c'est-à-dire entre un et deux ordres de magnitude plus bas que la valeur trouvée pour le composé no. 2. Dans le même temps, la sélectivité dans la reconnaissance entre le marqueur glucidique placé sur la cyclodextrine et la lectine spécifique reste intacte. Ainsi, le composé no. 6 qui ne comporte que des substituants β-D-glucopyranosyle, n'est pas reconnu par la ConA, une lectine spécifique du ligand α-D-mannopyranosyle. On n'observe pas de phénomènes de reconnaissance non spécifiques imputables à la présence de la cyclodextrine.

**Exemple 9 : Inclusion du Taxotère dans l'heptachlorhydrate d'heptakis[6-*S*-(2-aminoéthyl-6-thio)]cyclomaltobeptaose (composé no. 1)**

On part du Taxotère à l'état pur et on disperse 2,1 mg (2,47 mmol) de ce produit dans 1 mL d'une solution contenant 50 mmo1.$L^{-1}$ du composé no. 1 dans l'eau stérile, puis on agite la suspension obtenue à 70°C jusqu'à obtention d'une solution claire qui indique la complexation du Taxotère. Une fois formé, le complexe reste en solution à température ambiante. On obtient ainsi une augmentation de la solubilité du Taxotère (2,1 g.$L^{-1}$) de l'ordre de 525 fois par rapport à celle du Taxotère en l'absence de cyclodextrine (0,004 g.$L^{-1}$).

**Exemple 10 : Inclusion du Taxotère dans l'heptakis[6-*S*-(2-*N'*-[2,2,2-tris[5-(1-thio-α-D-mannopyranosyl)-2-oxapentyl]éthyl]thiouréido]éthyl-6-thio]cyclomalto heptaose (composé no. 4)**

On part du Taxotère à l'état pur et on disperse 0,2 mg (235 μmol) de ce produit dans 0,1 mL d'une solution contenant 50 mmol.$L^{-1}$ du composé no. 4 dans l'eau stérile, puis on agite la suspension obtenue à 70°C jusqu'à obtention d'une solution claire qui indique l'encapsulation du Taxotère. Une fois formé, le complexe reste en solution à température ambiante. On obtient ainsi une augmentation de la solubilité du Taxotère (2,0 g.$L^{-1}$) de l'ordre de 500 fois celle du Taxotère en l'absence de cyclodextrine.

## Revendications

1. Composé répondant à la formule générale suivante :

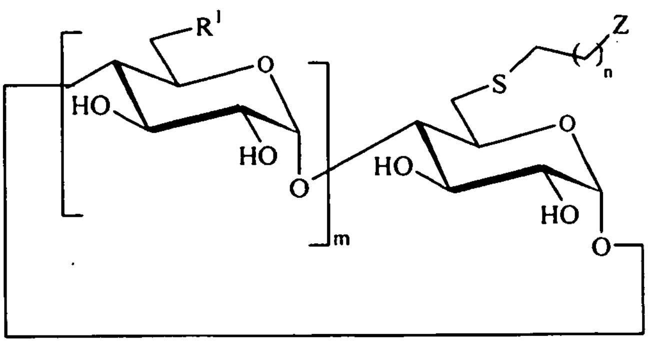

(I)

dans laquelle :

- n représente un nombre entier compris de 1 à 6 ;
- m représente un nombre entier égal à 5, 6 ou 7 ;
- $R^1$ représente soit un groupe OH soit un groupe $-S-CH_2-(CH_2)_n-Z$, tous les $R^1$ étant identiques ;
- Z représente soit :

* un groupe NHX,
* un groupe ammonium quaternaire de la forme $^+NX_3$,
* un groupe

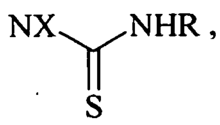

X représentant un atome d'hydrogene ou un groupe alKyle comprenant de 1 à 6 atomes de carbone, et étant notamment un groupe méthyle, éthyle, propyle ou butyle, et
R représentant un atome d'hydrogène, un substituant alkyle de 1 à 12 atomes de carbone linéaire ou ramifié, ou un groupe aromatique tel que le groupe phényle, benzyle ou naphtyle, ou des dérivés de ces groupements portant des substituants sur le cycle aromatique tels que les substituants méthyle, éthyle, chlore, brome, iode, nitro, hydroxyle, méthoxyle ou acétamido,

ou R représentant un élément de bioreconnaissance tel qu'un dérivé d'acide aminé, un peptide, un monosaccharide, un oligosaccharide, un élément de multiplication à plusieurs ramifications comportant des groupements glucidiques qui peuvent être identiques ou différents. ou un
sonde de visualisation ou de détection fluorescente ou radioactive. sous réserve que le composé dans lequel n = 1, m = 6, Z = $NH_2$ et $R_1$ = OH soit exclu.

2. Composé selon la revendication 1, **caractérisé en ce que** $R^1$. représente OH. et répondant à la formule générale suivante :

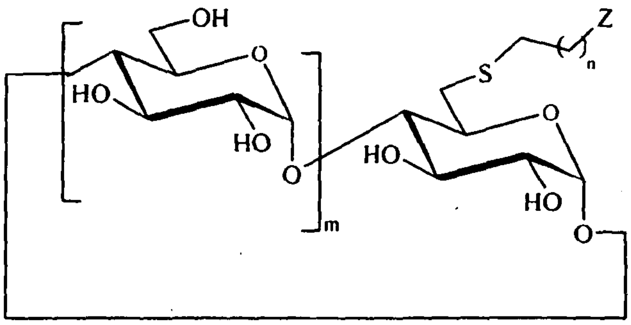

**(I-a)**

dans laquelle :

- m, n et Z sont tels que définis dans la revendication 1.

3. Composé selon la revendication 2, répondant à la formule (I-a) et **caractérisé en ce que** Z représente un groupe NHX, X étant tel que défini dans la revendication 1, et étant notamment un atome d'hydrogène.

4. Composé selon la revendication 2, répondant à la formule (I-a) et **caractérisé en ce que** Z représente un groupe

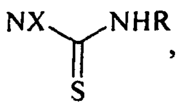

R étant tel que défini dans la revendication 1, et X étant tel que défini dans la revendication 1, et étant notamment un atome d'hydrogène.

5. Composé selon la revendication 1, **caractérisé en ce que** $R^1$ représente un groupe $-S-CH_{2-}(CH_2)_n-Z$, et répondant à la formule générale suivante :

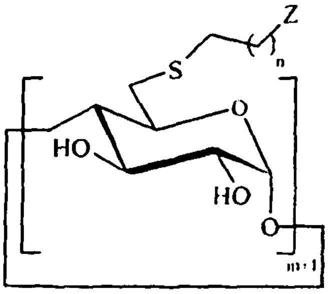

(I-b)

dans laquelle m. n et Z sont tels que définis dans la revendication 1.

6. Composé selon la revendication 5, répondant à la formule suivante :

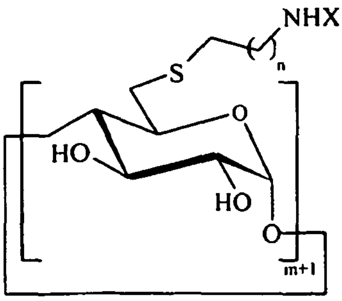

(I-c)

X, n et m étant tels que définis dans la revendication 1.

7. Composé selon la revendication 6, **caractérisé en ce que** X représente un atome d'hydrogène et **en ce que** n est égal à 1, et répondant à la formule suivante :

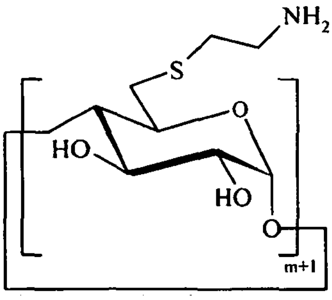

(I-d)

m étant tel que défini dans la revendication 1.

8. Composé selon la revendication 6, répondant à la formule suivante :

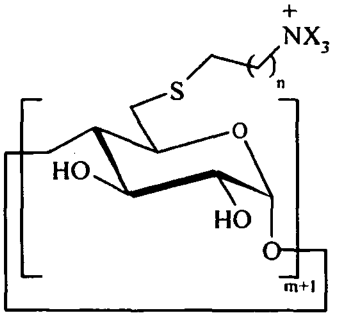

(I-e)

X, n et m étant tels que définis dans la revendication 1.

**9.** Composé selon la revendication 6, répondant à la formule suivante :

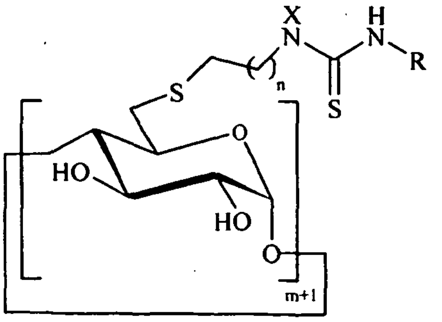

**(II)**

X. n. m et R étant tels que définis dans la revendication 1, et R étant identique pour chaque groupe

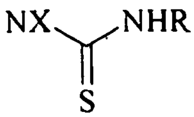

tel que défini dans la revendication 1.

**10.** Composé selon la revendication 9, **caractérisé en ce que** X représente un atome d'hydrogène et **en ce que** n est égal à 1, et répondant à la formule suivante :

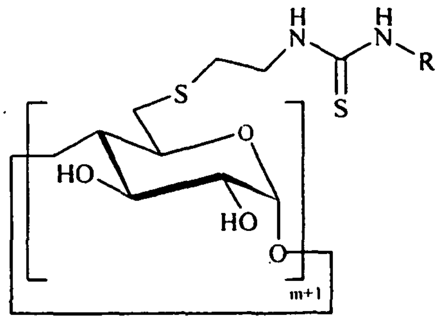

**(II-a)**

R et m étant tels que définis dans la revendication 1.

**11.** Composé selon l'une quelconque des revendications 1 à 10, **caractérisé en ce que** l'un au moins des groupes NHX tels que définis ci-dessus est protoné et associé à un anion monovalent choisi notamment parmi l'ion chlorure, bromure ou iodure.

**12.** Composé selon la revendication 6, **caractérisé en ce que** n est égal à 1 et **en ce que** le groupe Z représente le groupe ammonium quaternaire $^{+}NX_3$, et **en ce qu'**il peut être associé à un anion monovalent choisi notamment parmi l'ion chlorure, bromure ou iodure, et répondant à la formula suivante :

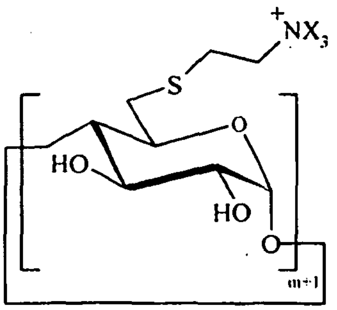

(I-e-bis)

**13.** Composé selon la revendication 10, **caractérisé en ce que** le groupement R est choisi parmi les groupes suivantes :

- le groupe α-D-mannopyranosyle, de formule suivante (III) :

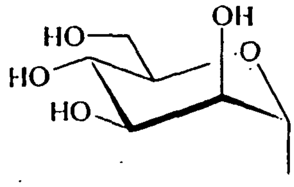

(III)

- le groupe β-lactosyle, de formule suivante (III-a) :

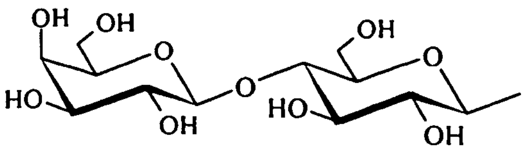

(III-a)

- le groupe dérivé du trisaccharide Lewis X ou du tétrasaccharide sialyl Lewis X, respectivement de formule suivante (III-b) et (III-c) :

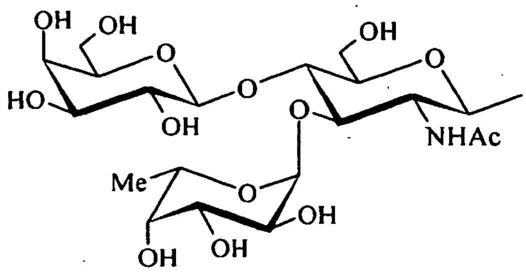

(III-b)

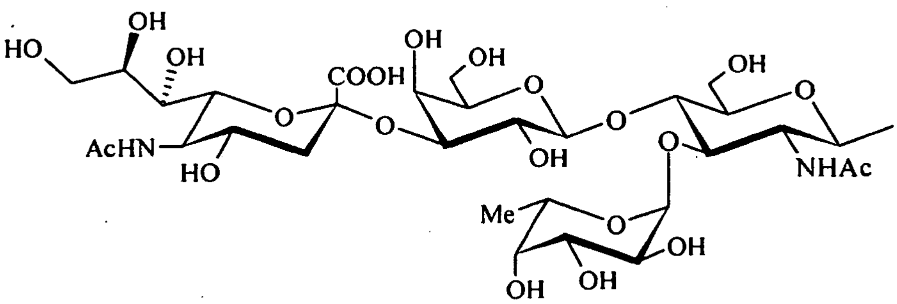

(III-c)

- un oligosaccharide dérivé de l'héparine, de formule suivante (III-d) :

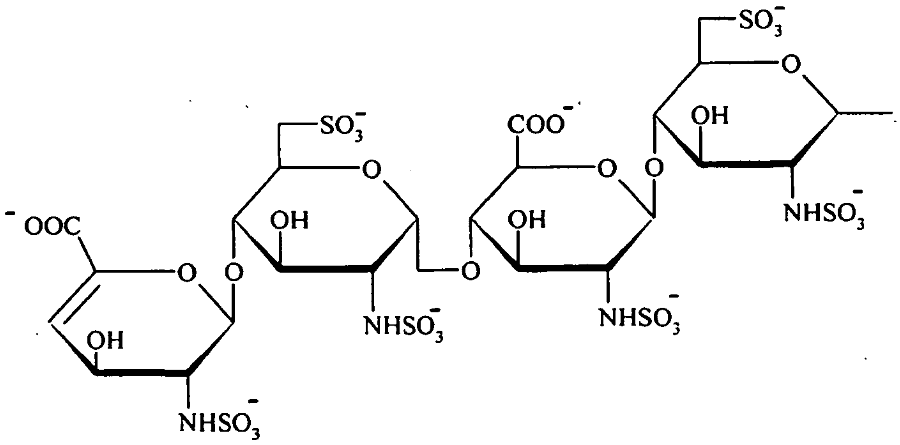

(III-d)

**14.** Composé selon la revendication 10, **caractérisé en ce que** :

R comporte un élément de ramification dérivé du tris(2-hydroxyméthyl)méthylamine, ou R représente l'un des groupes suivants :

- le groupe tris(α-D-mannopyranosyloxyméthyl)méthyle, de formule suivante (IV) :

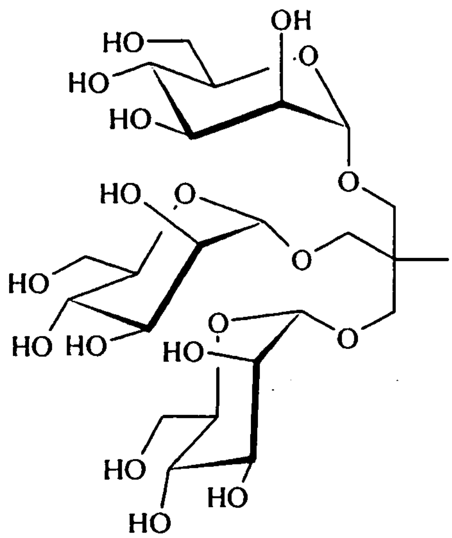

(IV)

- le groupe tris(β-lactosyloxyméthyl)méthyle, de formule suivante (IV-a) :

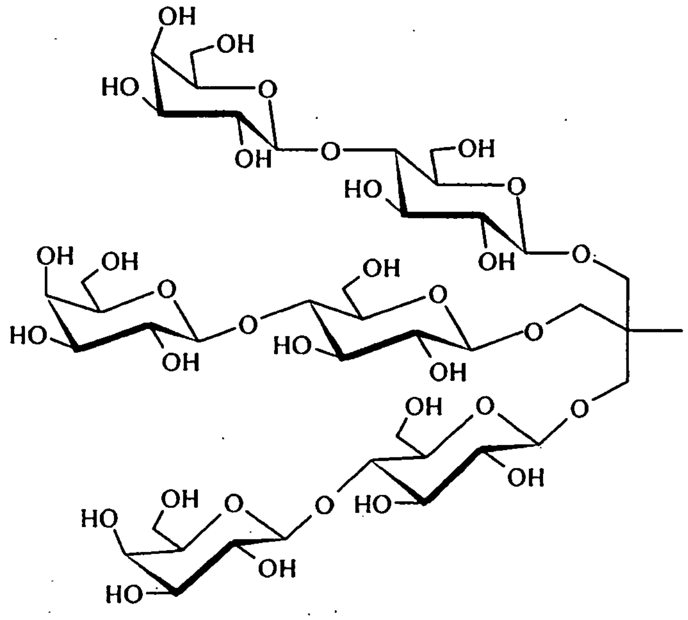

(IV-a)

**15.** Composé selon la revendication 4, **caractérisé en ce que** R comporte un élément de ramification dérivé du pentaérythritol, ledit composé répondant à la formule

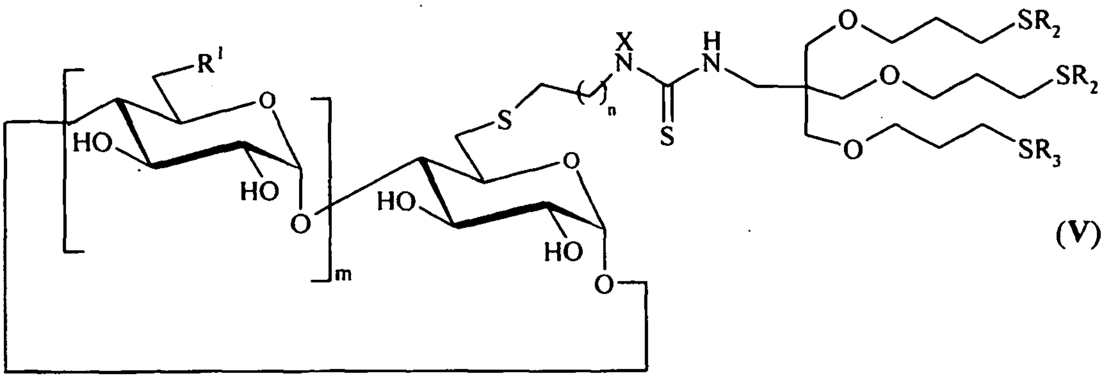

(V)

dans laquelle m, n, $R^1$ et X sont tels que définis dans la revendication 1, et
$R^2$ et $R^3$ représentent des dérivés glucidiques qui peuvent être différents ou identiques ou encore une sonde fluorescente ou radioactive.

**16.** Composé selon la revendication 15, **caractérisé en ce que** $R^1$ représente OH.

**17.** Composé selon la revendication 15, **caractérisé en ce que** $R^1$ représente le groupe de formule :

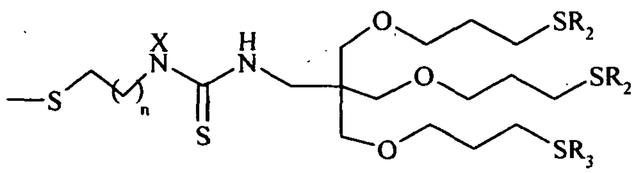

**18.** Composé selon l'une quelconque des revendications 15 à 17, **caractérisé en ce que** n est égal à 1, **en ce que** X

représente un atome d'hydrogène et **en ce que** $R^2$ et $R^3$ représentent l'un des groupes suivants :

- le groupe α-D-mannopyranosyle, de formule (III) tel que défini dans la revendication 12, ou
- le groupe β-lactosyle, de formule (III-a) tel que défini dans la revendication 13, ou
- le groupe β-D-glucopyranosyle, de formule (VI) suivante :

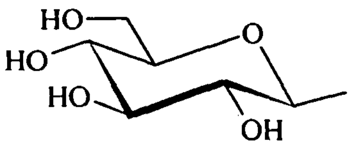

(VI)

$R^2$ et $R^3$ pouvant être identiques ou différents.

**19.** Composé selon l'une quelconque des revendications 1 à 18. **caractérisé en ce que** m est égal à 6.

**20.** Complexe d'inclusion d'un composé selon l'une quelconque des revendications 1 à 19, avec une molécule pharmacologiquement active, le rapport molaire entre le composé selon l'une des revendications 1 à 19 et la molécule pharmacologiquement active étant avantageusement d'environ 50:1 à environ 1:1.

**21.** Complexe selon la revendication 20, **caractérisé en ce que** la molécule pharmacologiquement active est un agent antitumoral, appartenant notamment à la famille du Taxol.

**22.** Composition pharmaceutique comprenant un composé selon l'une quelconque des revendications 1 à 19, ou un complexe d'inclusion selon la revendication 20 ou 21, avec un véhicule pharmacologiquement acceptable.

**23.** Composition pharmaceutique selon la revendication 22, sous forme de solution aqueuse.

**24.** Composition pharmaceutique selon l'une quelconque des revendications 22 ou 23, **caractérisée en ce qu'**elle contient par dose unitaire d'environ 50 mg à environ 500 mg de l'un des composés selon l'une quelconque des revendications 1 à 19, ou **en ce qu'**elle contient par dose unitaire d'environ 100 mg à environ 750 mg de l'un des complexes selon l'une des revendications 20 ou 21.

**25.** Procédé de préparation d'un composé de formule (I) selon la revendication 1, comprenant les étapes suivantes :

- la réaction d'un composé sélectivement ou totalement halogéné en position alcool primaire, de formule (VII) suivante :

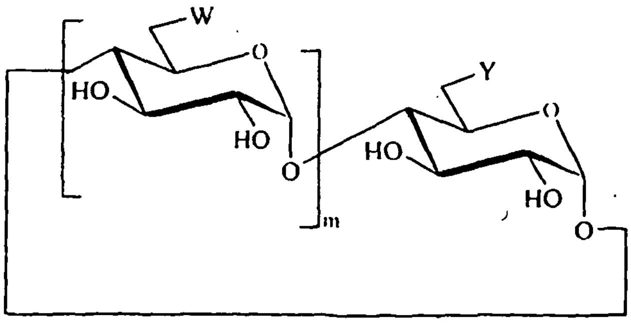

(VII)

m étant tel que défini dans la revendication 1 dans la formule (I),
W représentant un groupe OH ou un groupe Y, les groupes W étant tous identiques,
et Y représentant un atome d'halogène choisi dans le groupe constitué du chlore, du brome, de l'iode, et étant de préférence le brome ou l'iode,
avec un ω-aminoalcanethiol de formule (VIII) suivante :

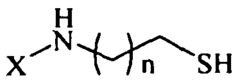

(VIII)

ledit ω-aminoalcanethiol étant éventuellement N-alkylé,

ou le sel correspondant de formule (VIII-a) suivante :

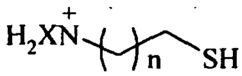

(VIII-a)

ou un sel de tétraalkylammonium de formule (VIII-b) suivante :

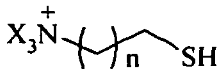

(VIII-b)

ledit sel étant associé à un contre-ion halogénure, de préférence l'ion chlorure,
n et X étant tels que définis ci-dessus, et X étant de préférence un atome d'hydrogène,
le composé de formule (VIII) étant de préférence la cystéamine de formule $H_2N-CH_2-CH_2-SH$,
afin d'obtenir un composé de formule (I) tel que défini dans la revendication 1 et répondant aux formules suivantes (A-a) ou (A-b) :

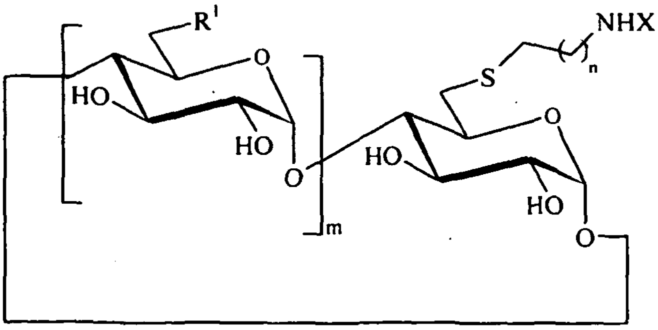

(A-a)

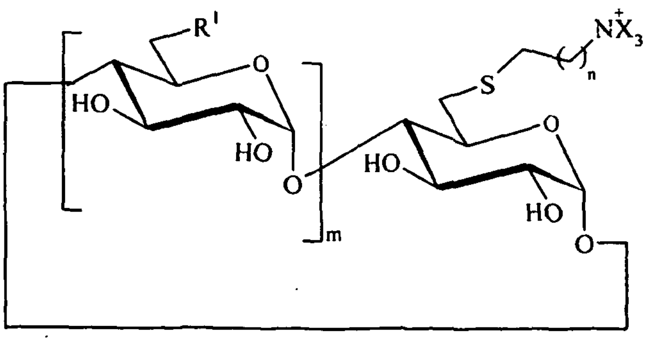

(A-b)

et éventuellement

- la réaction du composé de formule (A-a) tel qu'obtenu à l'étape précédente avec un isothiocyanate de formule (IX) suivante :

R-N=C=S **(IX)**

dans laquelle R est tel que défini dans la revendication 1,
afin d'obtenir un composé de formule (I) telle que définie ci-dessus, et répondant à la formule suivante :

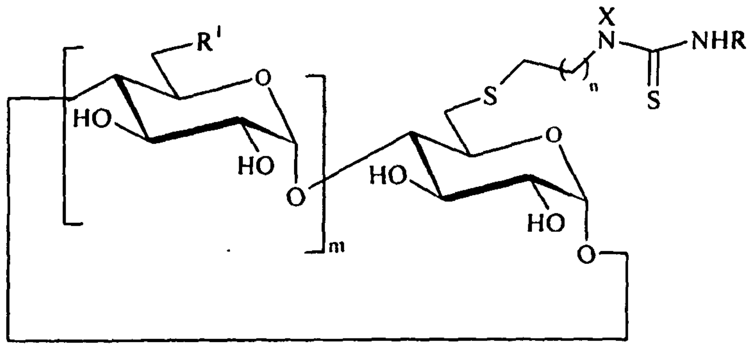

**(B)**

**26.** Procédé de préparation selon la revendication 25, d'un composé répondant à la formule générale (I-b) suivante :

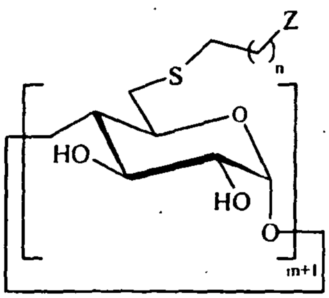

**(I-b)**

ledit procédé étant **caractérisé en ce qu'**il comprend les étapes suivantes :

- la réaction d'un composé per(6-désoxy-6-halo) cyclodextrine, de formule (VII-a) suivante :

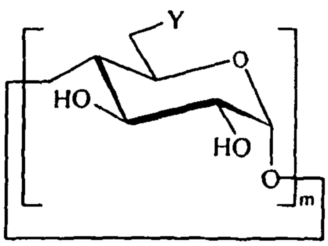

**(VII-a)**

m et Y étant tels que définis dans la revendication 25.
avec un ω-aminoalcanethiol de formule (VIII) suivante :

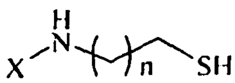

**(VIII)**

ledit ω-aminoaleanethiol étant éventuellement N-alkylé,

ou le sel correspondant de formule (VIII-a) suivante :

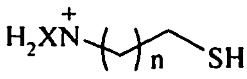

**(VIII-a)**

ou un sel de tétraalkylammonium de formule (VIII-b) suivante :

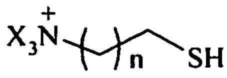

**(VIII-b)**

ledit sel étant associé à un contre-ion halogénure, de préférence l'ion chlorure,
n et X étant tels que définis dans la revendication 1, et X étant de préférence un atome d'hydrogène,
le composé de formule (VIII) étant de préférence la cystéamine de formule $H_2N$-$CH_2$-$CH_2$-SH,
afin d'obtenir un composé de formules (I-c), (I-d) ou (I-e) suivantes

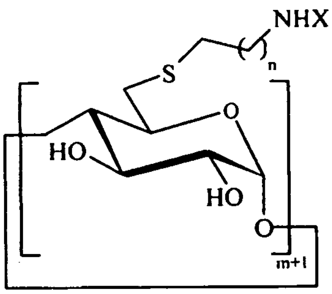

**(I-c)**

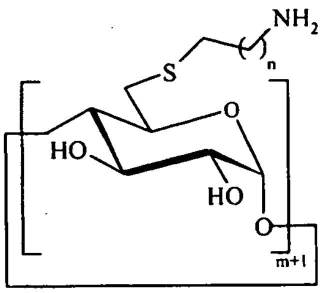

**(I-d)**

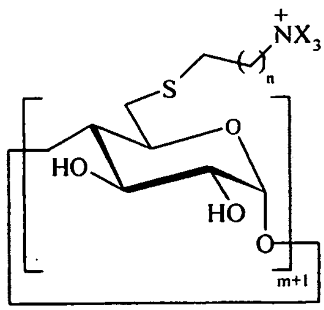

**(I-e)**

et éventuellement

- la réaction du composé de formule (I-c) tel qu'obtenu à l'étape précédente avec un isothiocyanate de formule (IX) suivante :

R-N=C=S **(IX)**

dans laquelle R est tel que défini dans la revendication 1,
afin d'obtenir un composé de formule (II) ou (II-a) suivante

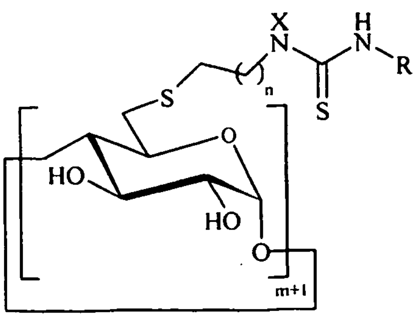

**(II)**

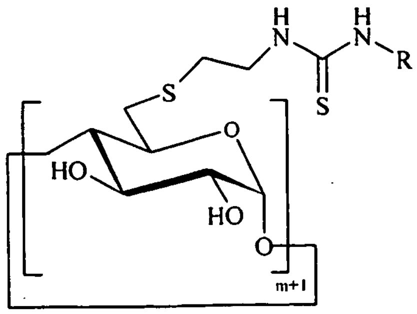

**(II-a)**

**27.** Procédé de préparation selon la revendication 25. de composés répondant à la formule suivante :

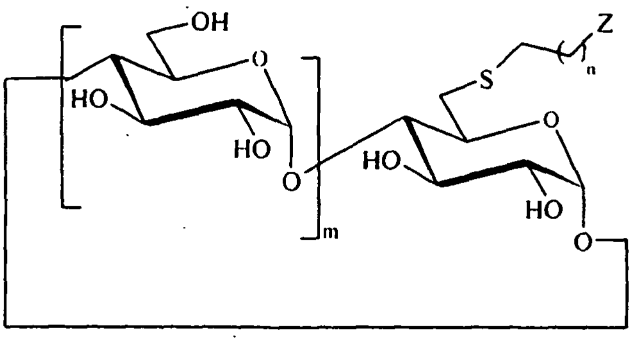

(I-a)

ledit procédé étant **caractérisé en ce qu'**il comprend les étapes suivantes :

- la réaction d'un composé sélectivement halogéné en position alcool primaire, de formule (VII) suivante :

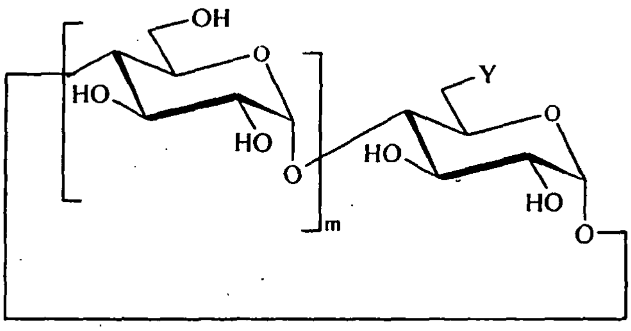

(VII)

m et Y étant tels que définis dans la revendication 25,
avec un ω-aminoalcanethiol de formule (VIII) suivante :

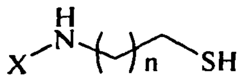

(VIII)

ledit ω-aminoalcanethiol étant éventuellement N-alkylé,

ou le sel correspondant de formule (VIII-a) suivante :

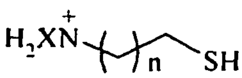

(VIII-a)

ou un sel de tétraalkylammonium de formule (VIII-b) suivante :

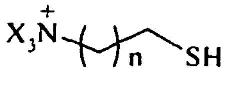

(VIII-b)

ledit sel étant associé à halogénure comme contre-ion, et étant de préférence l'ion chlorure,
n et X étant tels que définis dans la revendication 1. et X étant de préférence un atome d'hydrogène.
le composé de formule (VIII) étant de préférence la cystéamine de formule $H_2N-CH_2-CH_2-SH$,
afin d'obtenir un composé de formule (I-f) ou (I-g), de formule suivante :

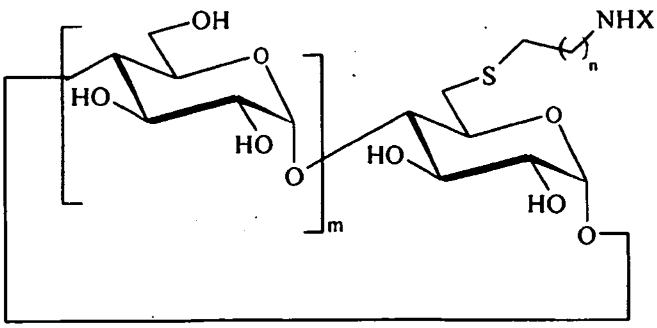

**(I-f)**

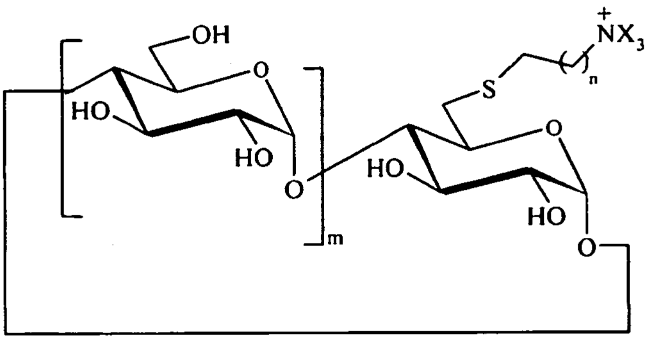

**(I-g)**

dans lesquelles m, n et X sont tels que définis dans la revendication 1, et éventuellement

- la réaction du composé de formule (I-f) tel qu'obtenu à l'étape précédente avec un isothiocyanate de formule (IX) suivante :

R-N=C=S **(IX)**

dans laquelle R est tel que défini dans la revendication 1,
afin d'obtenir un composé de formule (I-h) :

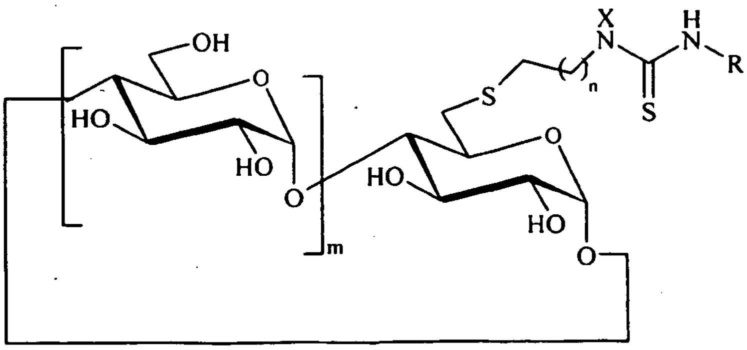

**(I-h)**

**28.** Procédé de préparation d'un composé de formule (I-f-bis)

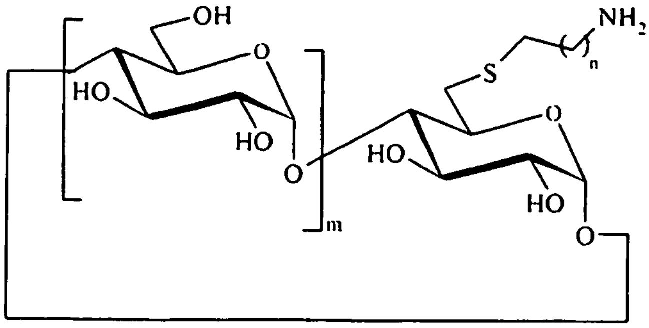

(I-f-bis)

dans laquelle m et n sont tels que définis dans la revendication 1, n étant de préférence égal à 1, ledit procédé étant **caractérisé en ce qu'**il comprend la réaction d'un composé sélectivement halogéné en position alcool primaire, de formule (VII) suivante :

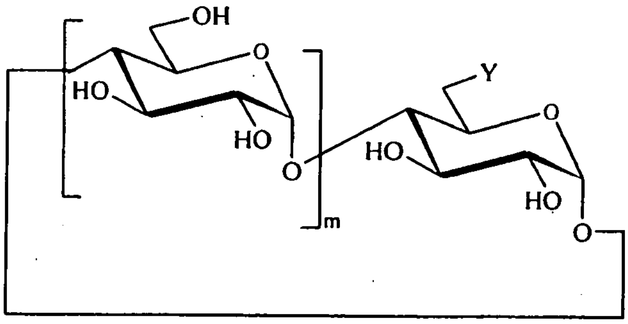

(VII)

m étant tel que défini ci-dessus, et
Y représentant un atome d'halogène choisi dans le groupe constitué du chlore, du brome, de l'iode, et étant de préférence le brome ou l'iode,
avec un ω-aminoalcanethiol de formule suivante :

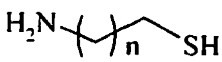

n étant tel que définis ci-dessus,
ou de préférence avec la cystéamine de formule $H_2N-CH_2-CH_2-SH$.

## Claims

**1.** A compound of the following general formula:

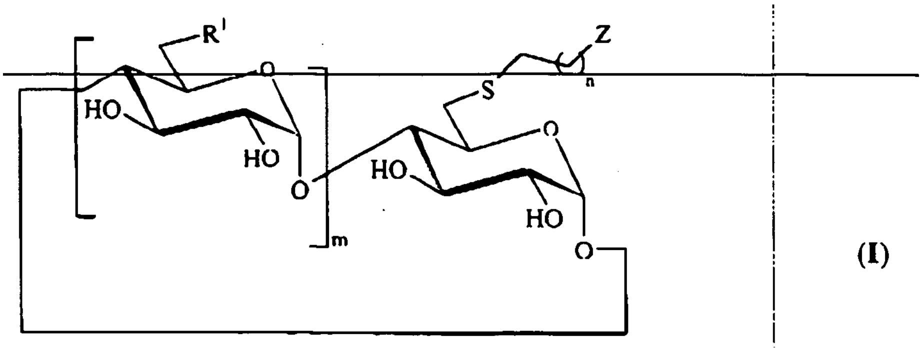

(I)

in which:

- n represents an integer from 1 to 6;
- m represents an integer equal to 5, 6 or 7;
- $R^1$ represents either an OH group or an $-S-CH_2-(CH_2)_n-Z$ group, the $R^1$ groups all being identical;
- Z represents either:

  * an NHX group,
  * a quaternary ammonium group of the $^+NX_3$ form, !
  * a

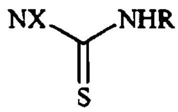

group,
X representing a hydrogen atom or an alkyl group comprising from 1 to 6 carbon atoms, and being in particular a methyl, ethyl, propyl or butyl group, and
R representing a hydrogen atom, a linear or branched alkyl substituent with 1 to 12 carbon atoms, or an aromatic group such as the phenyl, benzyl or naphthyl group, or derivatives of these groups carrying substituents on the aromatic ring such as methyl, ethyl, chlorine, bromine, iodine, nitro, hydroxyl, methoxyl or acetamido substituents,
or R representing a biorecognition element such as an amino acid derivative, a peptide, a monosaccharide, an oligosaccharide, a multiplication element with several branchings comprising glucidic groups which can be identical or different, or a visualization probe or fluorescent on radioactive detection probe,

provided that the compound in which n = 1, m = 6, Z = $NH_2$ and $R_1$ = OH is excluded.

**2.** The compound of claim 1, **characterized in that** $R^1$ represents OH, and having the following general formula:

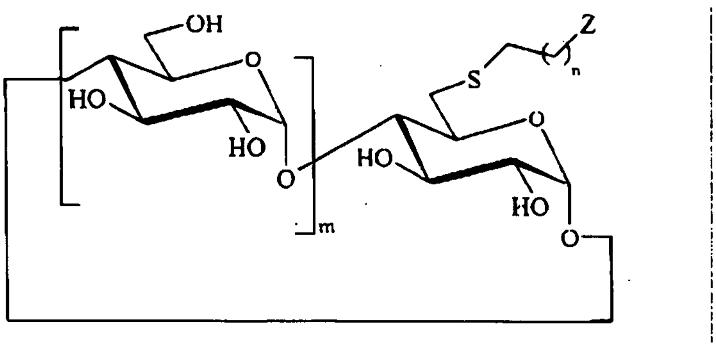

(I-a)

in which:

- m, n and Z are as defined in claim 1.

**3.** The compound of claim 2, having the formula (I-a) and **characterized in that** Z represents an NHX group, X being as defined in claim 1, and in particular being a hydrogen atom.

**4.** The compound of claim 2, having the formula (I-a) and **characterized in that** Z represents a

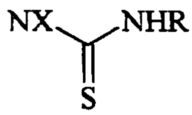

group, R being as defined in claim 1, and X being as defined in claim 1, and being in particular a hydrogen atom.

**5.** The compound of claim 1, **characterized in that** $R^1$ at $-S-CH_2-(CH_2)_n-Z$ group, and having the following general formula:

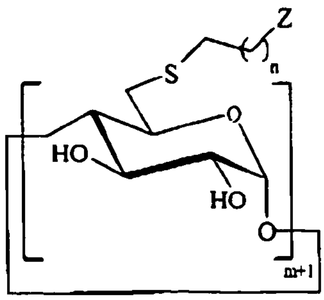

**(I-b)**

in which m, n and Z are as defined in claim 1.

**6.** The compound of claim 5, having the following formula:

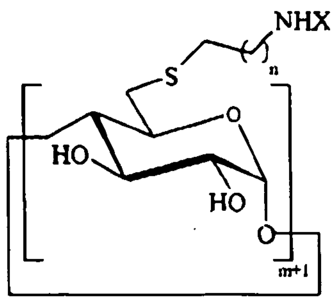

**(I-c)**

X, n and m being as defined in claim 1.

**7.** The compound of claim 6, **characterized in that** X represents a hydrogen atom and **in that** n is equal to 1, and having the following formula:

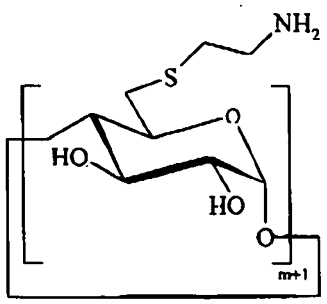

(I-d)

m being as defined in claim 6.

**8.** The compound of claim 5, corresponding to the following formula:

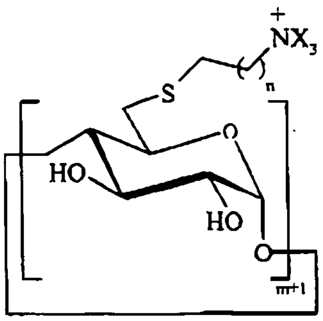

(I-e)

X, n and m being as defined in claim 1.

**9.** The compound of claim 5, corresponding to the following formula:

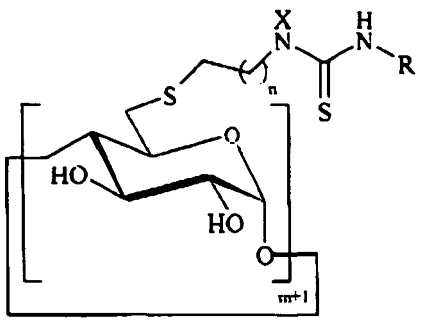

(II)

X, n, m and R being as defined in claim 1, and R being identical for each

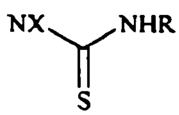

group as defined in claim 1.

**10.** The compound of claim 9, **characterized in that** X represents a hydrogen atom and **in that** n is equal to 1, and having the following formula:

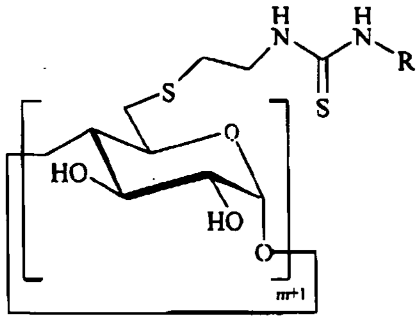

(II-a)

R and m being as defined in claim 1.

**11.** The compound according to any one of claims 1 to 10, **characterized in that** at least one of the NHX groups as defined above is protonated and associated with a monovalent anion chosen in particular from the chloride, bromide or iodide ion.

**12.** The compound of claim 6, **characterized in that** n is equal to 11 and **in that** the Z group represents the quaternary ammonium $+NX_3$ group, and **in that** it can be associated with a monovalent anion chosen in particular from the chloride, bromide or iodide ion, and having the following formula:

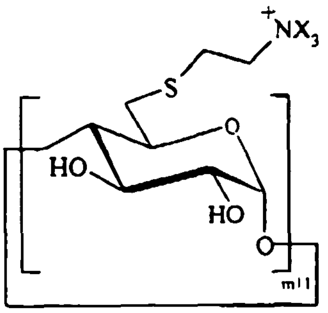

(I-e-bis)

**13.** The compound according to claim 10, **characterized in that** the R group is chosen from the following groups:

- the α-D-mannopyranosyl group, of the following formula (III):

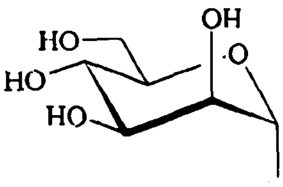

(III)

- the β-lactosyl group, of the following formula (III-a):

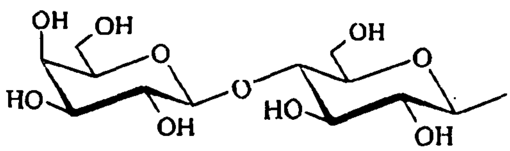

(III-a)

- the group derived from Lewis X trisaccharide or from sialyl Lewis X tetrasaccharide, of the following formulae

(III-b) and (III-c) respectively:

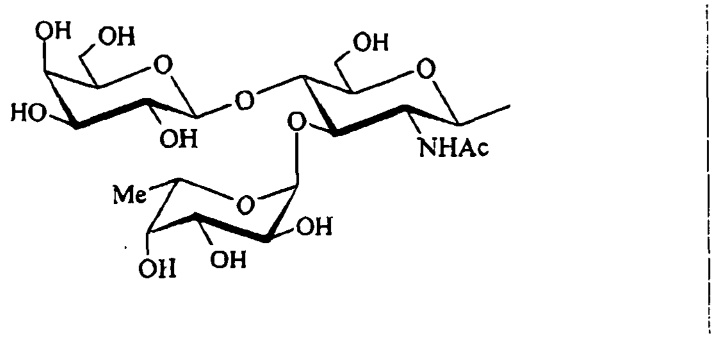

(III-b)

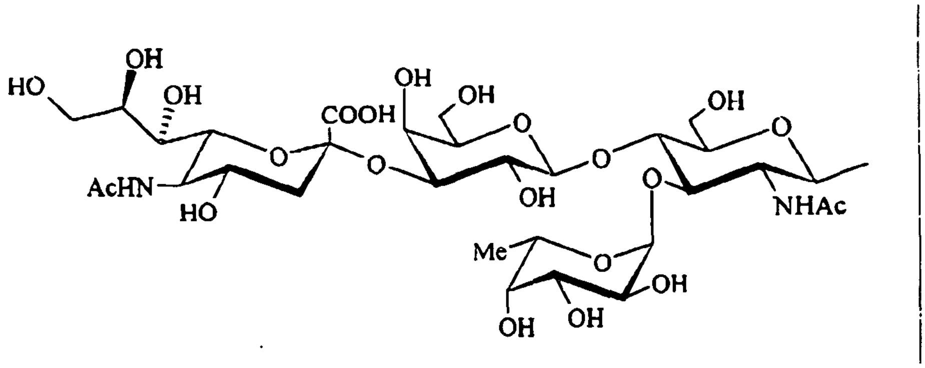

(III-c)

- an oligosaccharide derived from heparin, of the following formula (III-d):

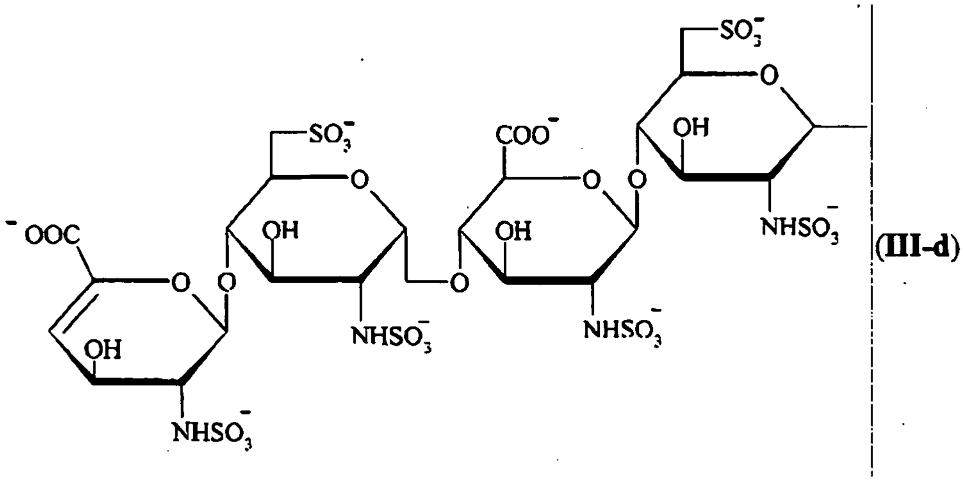

(III-d)

**14.** The compound of claim 10, **characterized in that**:

R comprises a branching element derived from tris(2-hydroxymethyl)methylamine, or R represents one of the following groups:

- the tris(α-D-mannopyranosyloxymethyl)methyl group, of the following formula (IV):

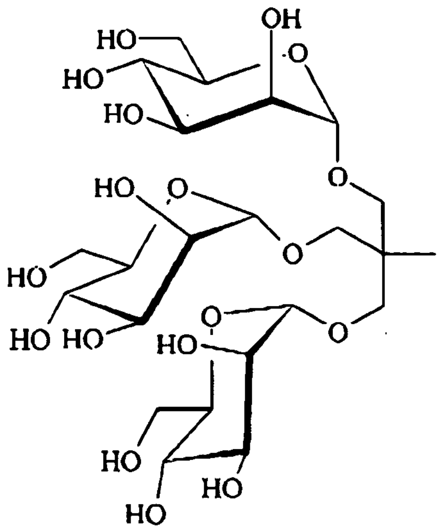

(IV)

- the tris(β-lactosyloxymethyl)methyl groups, of the following formula (IV-a):

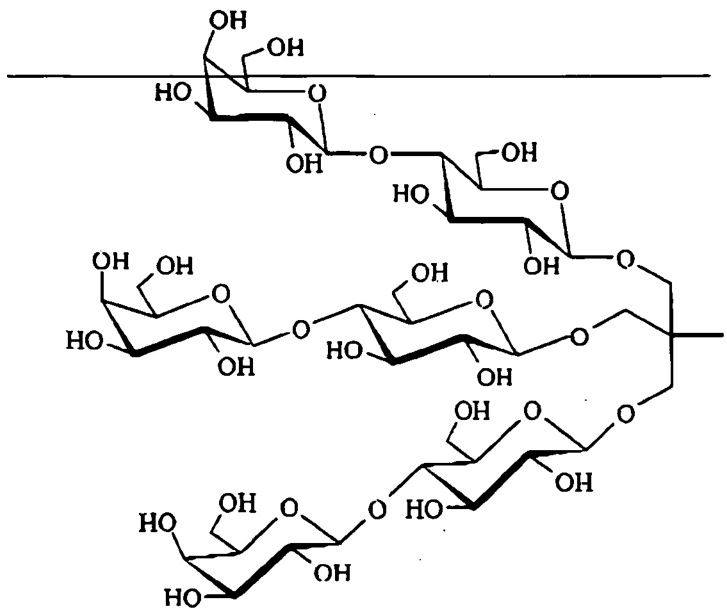

(IV-a)

**15.** The compound of claim 4, **characterized in that** R comprises a branching element derived from pentaerythritol, said compound having the following formula:

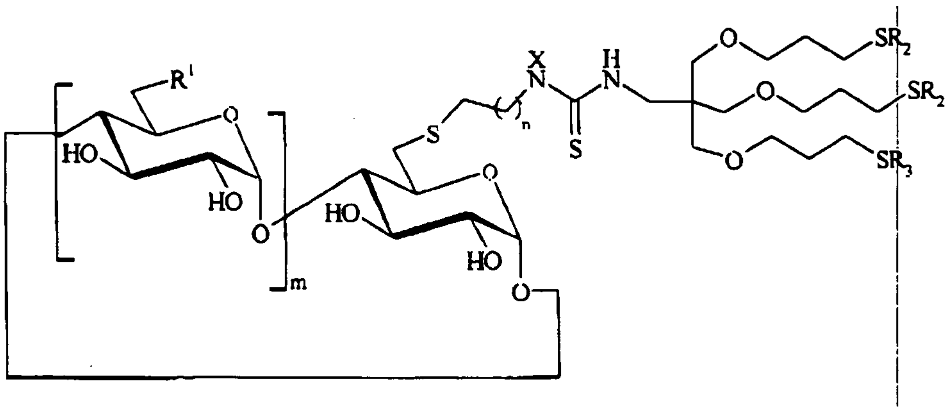

(V)

in which m, n, $R^1$ and X are as defined in claim 1, and
$R^2$ and $R^3$ represent glucidic derivatives which can be different or identical or also a fluorescent or radioactive probe.

**16.** The compound of claim 15, **characterized in that** $R^1$ represents OH.

**17.** The compound of claim 15, **characterized in that** $R^1$ represents the group of formula:

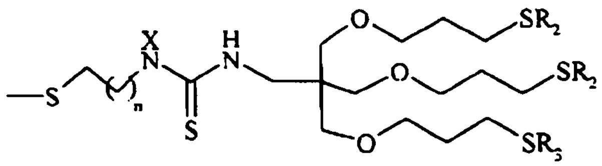

**18.** The compound of any one of claims 15 to 17, **characterized in that** n is equal to 1, **in that** X represents a hydrogen atom and **in that** $R^2$ and $R^3$ represent one of the following groups:

- the $\alpha$-D-mannopyranosyl group, of formula (III) as defined in claim 12, or
- the $\beta$-lactosyl group, of formula (III-a) as defined in claim 13, or
- the $\beta$-D-glucopyranosyl group, of the following formula (VI):

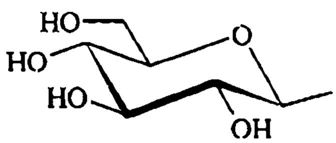

(VI)

$R^2$ and $R^3$ being able to be identical or different.

**19.** The compound according to any one of claims 1 to 18, **characterized in that** m is equal to 6.

**20.** An inclusion complex of a compound according to any one of claims 1 to 19, with a pharmacologically active molecule, the molar ratio between compound according to one of claims 1 to 19 and the pharmacologically active molecule advantageously being approximately 50:1 to approximately 1:1.

**21.** The complex of claim 20, **characterized in that** the pharmacologically active molecule is an antineoplastic agent, in particular belonging to the taxol family.

**22.** A pharmaceutical composition comprising a compound according to any one of claims 1 to 19, or an inclusion complex according to claim 20 or 21, with a pharmacologically acceptable vehicle.

**23.** The pharmaceutical composition of claim 22, in the form of an aqueous solution.

**24.** The pharmaceutical composition of any one of claims 22 or 23, **characterized in that** it contains per single dose approximately 50 mg to approximately 500 mg of one of the compounds according to any one of claims 1 to 19 or **in that** it contains per single dose approximately 100 mg to approximately 750 mg of one of the complexes according to one of claims 20 or 21.

**25.** A process for the preparation of a compound of formula (I), according to claim 1, said process being **characterized in that** it comprises the following stages:

- the reaction of a compound selectively or totally halogenated in primary alcohol position, of the following formula (VII):

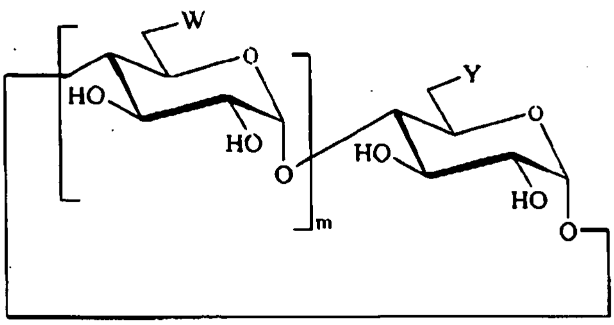

(VII)

m being as defined in claim 1 formula (I),
W representing an OH group or a Y group, the W groups all being identical,
and Y representing a halogen atom chosen from the group constituted by chlorine, bromine, iodine, and preferably being bromine or iodine,
with an ω-aminoalkanethiol of the following formula (VIII):

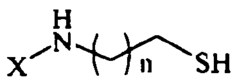

(VIII)

said ω-aminoalkanethiol optionally being N-alkylated,
or the corresponding salt of the following formula (VIII-a):

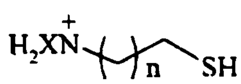

(VIII-a)

or a tetraalkylammonium salt of the following formula (VIII-b):

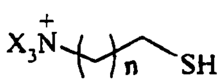

(VIII-b)

said salt being associated with a halide counter ion, preferably the chloride ion,
n and X being as defined above, and X preferably being a hydrogen om,
the compound of formula (VIII) preferably being cysteamine of formula $H_2N\text{-}CH_2\text{-}CH_2\text{-}SH$,
in order to obtain a compound of formula (I) as defined in claim 1 and having the following formulae (A-a) or (A-b):

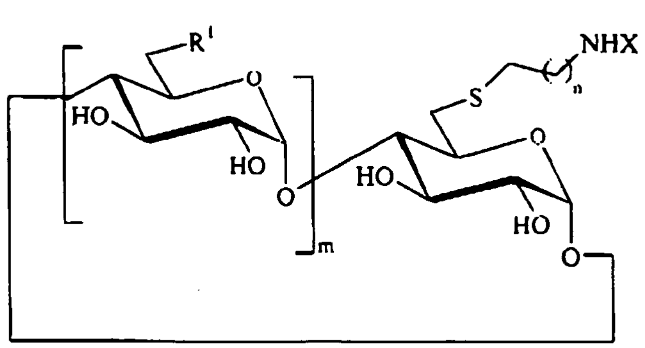

(A-a)

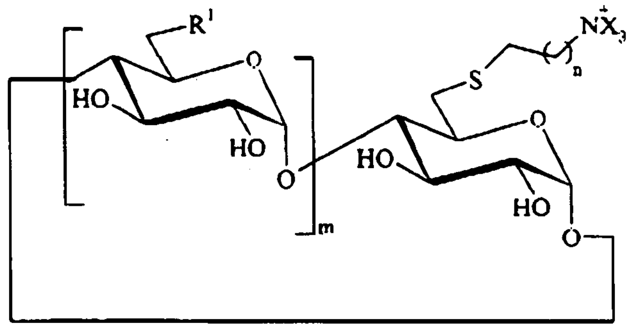

(A-b)

and optionally

- the reaction of the compound of formula (A-a) as obtained in the preceding stage with an isothiocyanate of the following formula (IX):

R-N=C=S **(IX)**

in which R is as defined un claim 1,

in order to obtain a compound of formula (1) as defined above, and corresponding to the following formula:

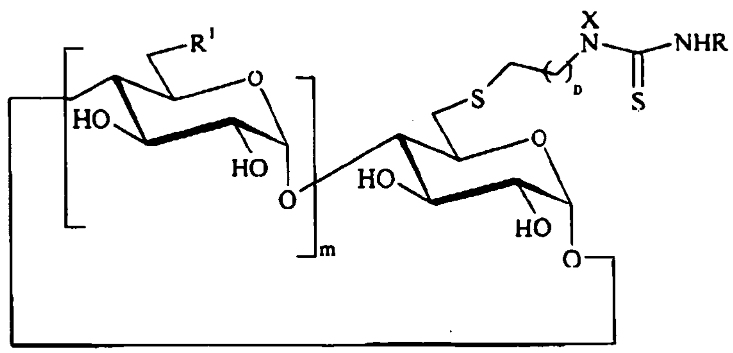

(B)

**26.** The preparation process according to claim 25, of a compound having the following general formula (I-b):

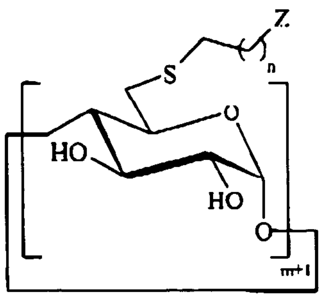

(I-b)

said process being **characterized in that** it comprises the following stages:

- the reaction of a per(6-deoxy-6-halo) cyclodextrin compound, of the following formula (VII-a):

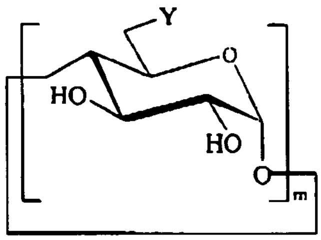

(VII-a)

m and Y being as defined in claim 25,
with an ω-aminoalkanethiol of the following formula (VIII):

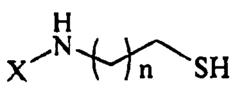

(VIII)

said ω-aminoalkanethiol optionally being N-alkylated,
or the corresponding salt of the following formula (VIII-a):

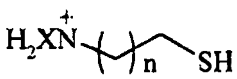

(VIII-a)

or a tetraalkylammonium salt of the following formula (VIII-b):

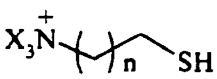

(VIII-b)

said salt being associated with a halide counter ion, preferably the chloride ion,
n and X being as defined in claim 1, and X preferably being a hydrogen atom,
the compound of formula (VIII) preferably being cysteamine of formula $H_2N-CH_2-CH_2-SH$,
in order to obtain a compound of the following formulae (I-c), (I-d) or (I-e)

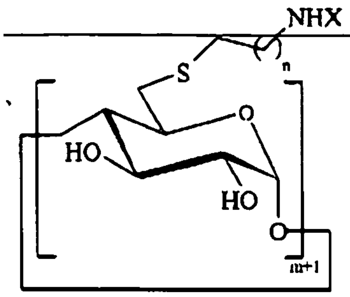

(I-c)

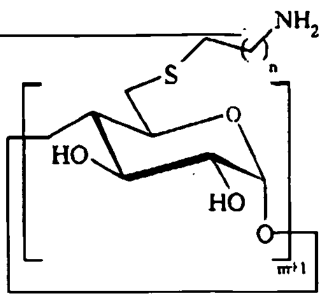

(I-d)

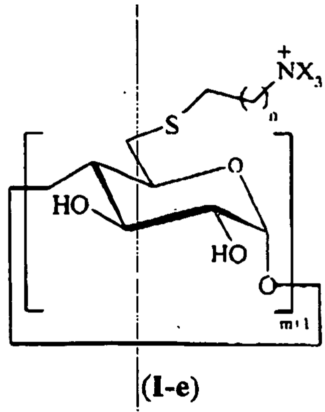

(I-e)

and optionally
- the reaction of the compound of formula (I-c) as obtained in the preceding stage with an isothiocyanate of the following formula (IX):

R-N=C=S **(IX)**

in which R is as defined in claim 1,
in order to obtain a compound of the following formula (II) or (II-a)

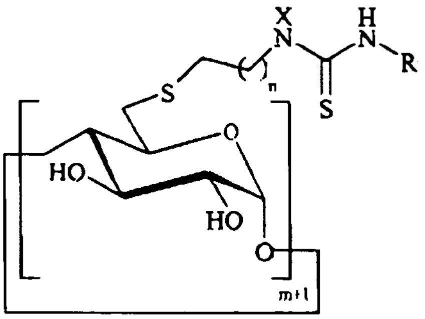

(II)

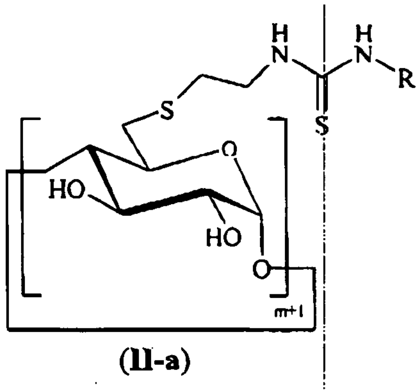

(II-a)

**27.** The preparation process according to claim 25 of compounds heaving the following formula:

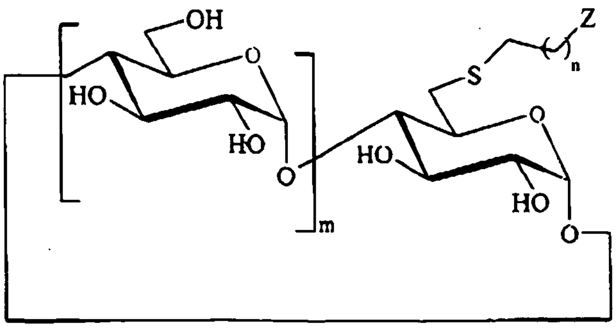

(I-a)

said process being **characterized in that** it comprises the following stages:

- the reaction of a compound selectively halogenated in primary alcohol position, of the following formula (VII):

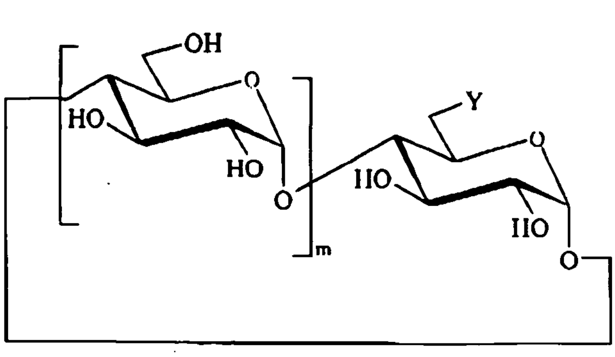

(VII)

m and Y being as defined in claim 25, and
with an ω-aminoalkanethiol of the following formula (VIII):

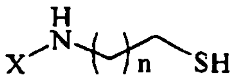

(VIII)

said ω-aminoalkanethiol optionally being N-alkylated,
or the corresponding salt of the following formula (VIII-a):

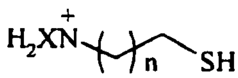

(VIII-a)

or a tetraalkylammonium salt of the following formula (VIII-b):

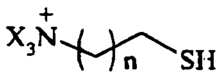

(VIII-b)

said salt being associated with halide as a counter ion, and preferably being the chlorine ion,
n and X being as defined in claim 1, and X preferably being a hydrogen atom,
the compound of formula (VHI) preferably being cysteamine of formula $H_2N-CH_2-CH_2-SH$,
in order to obtain a compound of formula (I- f) or (I- g), of the following formula:

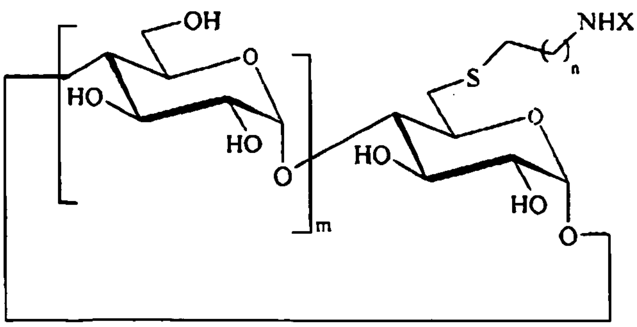

(I-f)

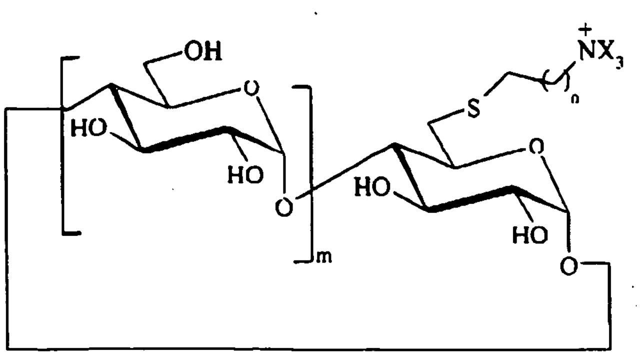

(I-g)

in which m, n and X are as defined in claim 1,
and optionally

- the reaction of the compound of formula (I-f) as obtained in the preceding stage with an isothiocyanate of the following formula (IX):

R-N=C=S **(IX)**

in which R is as defined in claim 1,
in order to obtain a compound of formula (I-h):

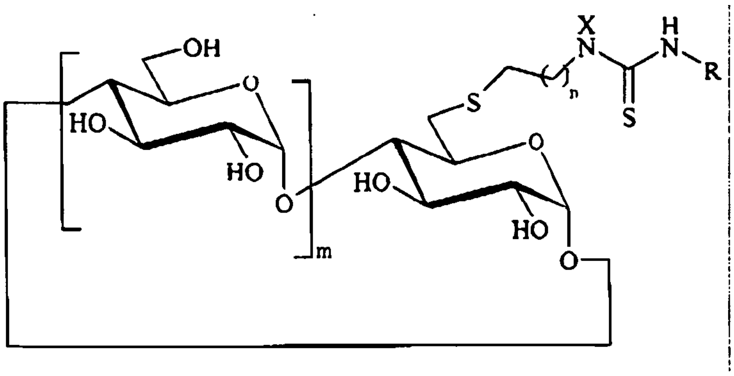

(I-h)

**28.** A process for the preparation of a compound of formula (I-f bis)

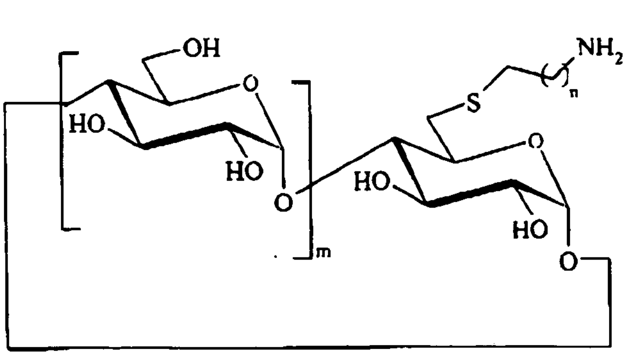

(I-f-bis)

in which m and n are as defined in claim 1, n preferably being equal to 1, said process being **characterized in that** it comprises the reaction of compound selectively halogenated in primary alcohol position, of the following formula (VII):

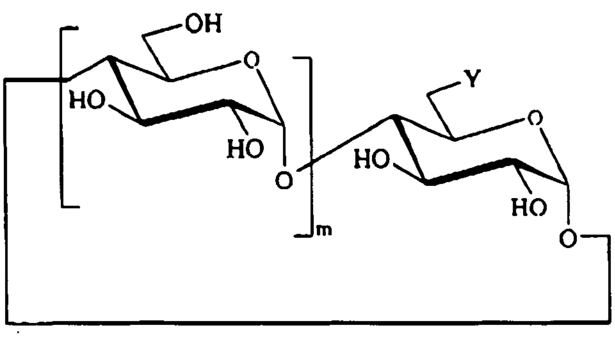

(VII)

m being as defined above, and
Y representing a halogen atom chosen from the group constituted by chlorine, bromine, iodine, and preferably being bromine or iodine,
with an ω-aminoalkanethiol of the following formula:

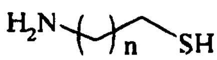

n being as defined above,
or preferably with cysteamine of formula $H_2N-CH_2-CH_2-SH$.

## Patentansprüche

**1.** Verbindung, die der folgenden allgemeinen Formel entspricht:

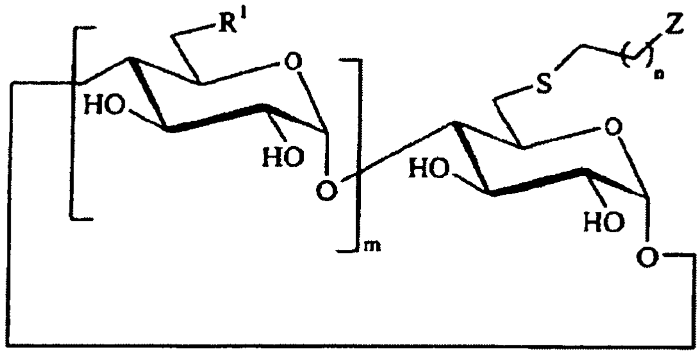

(I)

worin:

- n für eine ganze Zahl im Bereich von 1 bis 6 steht;
- m für eine ganze Zahl gleich 5, 6 oder 7 steht;
- $R^1$ entweder für eine OH-Gruppe oder eine $-S-CH_2-(CH_2)_n-Z$-Gruppe steht, wobei alle $R^1$ gleich sind;
- Z entweder:

  * für eine NHX-Gruppe,
  * eine quartäre Ammoniumgruppe mit der Form $^+NX_3$,
  * eine Gruppe

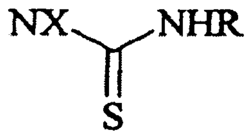

steht,

X für ein Wasserstoffatom oder eine Alkylgruppe steht, die 1 bis 6 Kohlenstoffatome umfasst, und insbesondere eine Methyl-, Ethyl-, Propyl- oder Butylgruppe ist, und

R für ein Wasserstoffatom, einen Alkylsubstituenten, linear oder verzweigt, mit 1 bis 12 Kohlenstoffatomen, oder eine aromatische Gruppe, wie etwa die Phenyl-, Benzyl- oder Naphtylgruppe, oder Derivate dieser Gruppen steht, die Substituenten auf dem aromatischen Ring tragen, wie etwa die Methyl-, Ethyl-, Chlor-, Brom-, Iod-, Nitro-, Hydroxyl-, Methoxyl- oder Acetamidosubstituenten,

oder R für ein Bioerkennungselement, wie etwa ein Aminosäurederivat, ein Peptid, ein Monosaccharid, ein Oligosaccharid, ein Multiplikationselement mit mehreren Verzweigungen, die Kohlenhydratgruppen umfassen, die gleich oder verschieden sein können, oder eine Sonde zur Visualisierung oder zum Fluoreszenz- oder Radioaktivitätsnachweis steht.

unter der Voraussetzung, dass die Verbindung, worin n = 1, m = 6, Z = $NH_2$ und $R^1$ = OH, ausgeschlossen ist.

2. Verbindung nach Anspruch 1, **dadurch gekennzeichnet, dass** $R^1$ für OH steht, und der folgenden allgemeinen Formel entspricht:

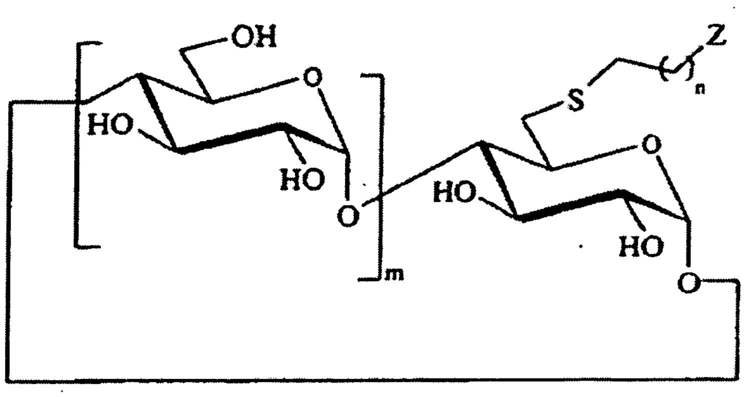

(I-a)

worin:

- m, n und Z so sind, wie in Anspruch 1 definiert.

3. Verbindung nach Anspruch 2, die der Formel (I-a) entspricht und **dadurch gekennzeichnet ist, dass** Z für eine NHX-Gruppe steht, wobei X so ist, wie in Anspruch 1 definiert, und insbesondere ein Wasserstoffatom ist.

4. Verbindung nach Anspruch 2, die der Formel (I-a) entspricht und **dadurch gekennzeichnet ist, dass** Z für eine Gruppe

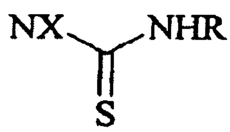

steht, wobei R so ist, wie in Anspruch 1 definiert, und X so ist, wie in Anspruch 1 definiert, und insbesondere ein

Wasserstoffatom ist.

5. Verbindung nach Anspruch 1, **dadurch gekennzeichnet, dass** $R^1$ für eine $-S-CH_2-(CH_2)_n-Z$-Gruppe steht, und der folgenden allgemeinen Formel entspricht:

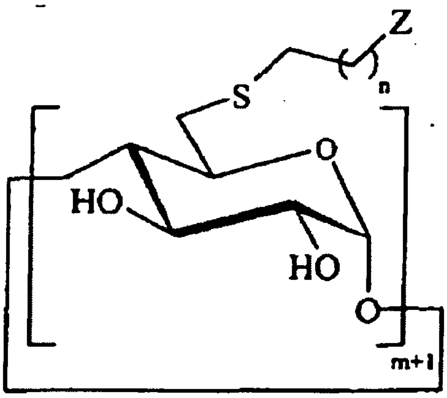

(I-b)

worin m, n und Z so sind, wie in Anspruch 1 definiert.

6. Verbindung nach Anspruch 5, die der folgenden Formel entspricht:

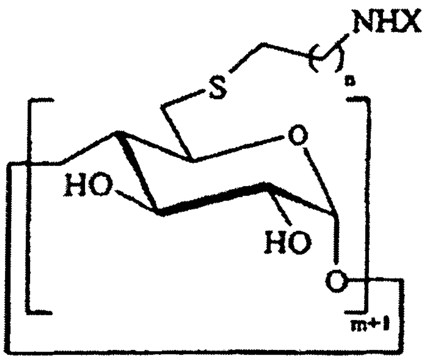

(I-c)

wobei X, n und m so sind, wie in Anspruch 1 definiert.

7. Verbindung nach Anspruch 6, **dadurch gekennzeichnet, dass** X für ein Wasserstoffatom steht und dadurch, dass n gleich 1 ist, und dass sie der folgenden Formel entspricht:

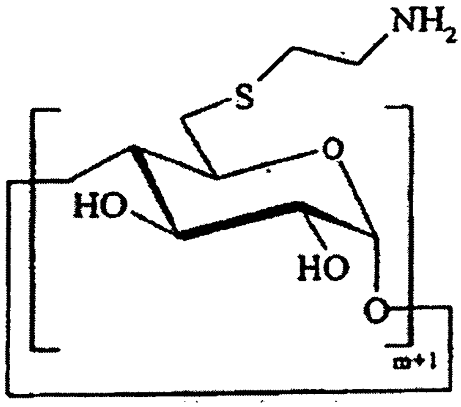

(I-d)

wobei m so ist, wie in Anspruch 1 definiert.

**8.** Verbindung nach Anspruch 5, die der folgenden Formel entspricht:

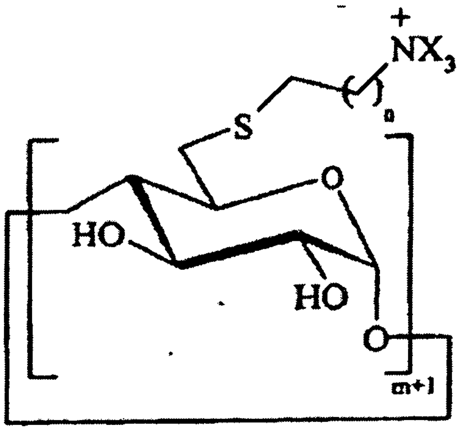

(I-e)

wobei X, n und m so sind, wie in Anspruch 1 definiert.

**9.** Verbindung nach Anspruch 5, die der folgenden Formel entspricht:

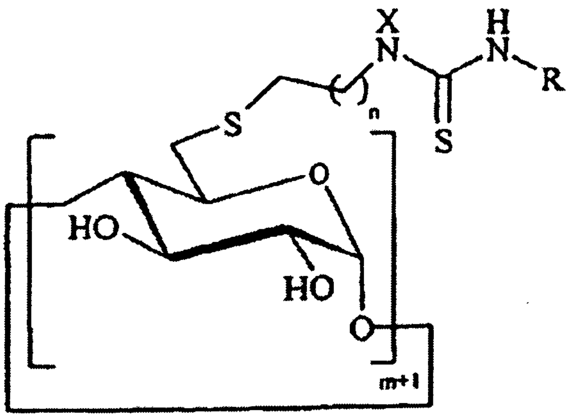

(II)

wobei X, n, m und R so sind, wie in Anspruch 1 definiert, und R für jede Gruppe

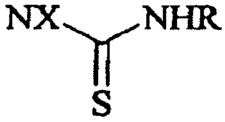

gleich oder verschieden ist, wie in Anspruch 1 definiert.

**10.** Verbindung nach Anspruch 9, **dadurch gekennzeichnet, dass** X für ein Wasserstoffatom steht und dadurch, dass n gleich 1 ist, und dass sie der folgenden Formel entspricht:

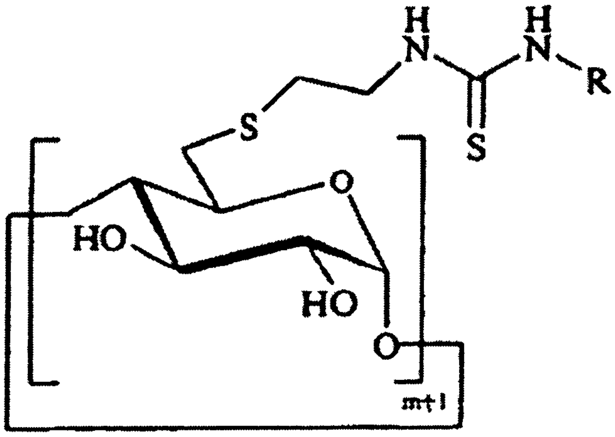

(II-a)

wobei R und m so sind, wie in Anspruch 1 definiert.

**11.** Verbindung nach einem der Ansprüche 1 bis 10, **dadurch gekennzeichnet, dass** mindestens eine der NHX-Gruppen, wie sie oben definiert sind, protoniert und mit einem monovalenten Anion assoziiert ist, das insbesondere aus dem Chlorid-, Bromid- oder Iodidion ausgewählt ist.

**12.** Verbindung nach Anspruch 6, **dadurch gekennzeichnet, dass** n gleich 1 ist, und dadurch, dass die Gruppe Z für eine quartäre Ammoniumgruppe $^{+}NX_3$ steht, und dadurch, dass sie mit einem monovalenten Anion assoziiert werden kann, das insbesondere aus dem Chlorid-, Bromid- oder Iodidion ausgewählt ist, und das der folgenden Formel entspricht:

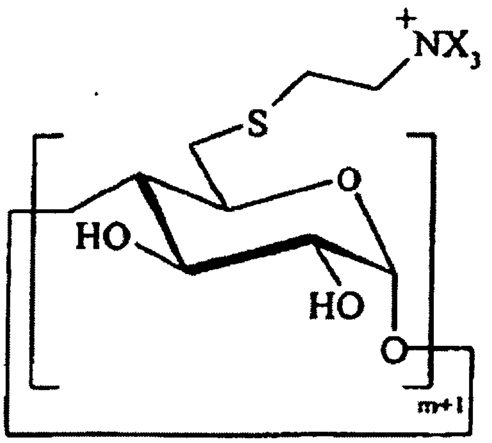

(I-e-bis)

**13.** Verbindung nach Anspruch 10, **dadurch gekennzeichnet, dass** die Gruppe R aus den folgenden Gruppen ausgewählt ist:

- der α-D-Mannopyranosylgruppe mit der folgenden Formel (III):

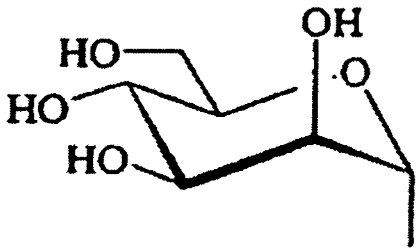

(III)

- der β-Lactosylgruppe mit der folgenden Formel (III-a):

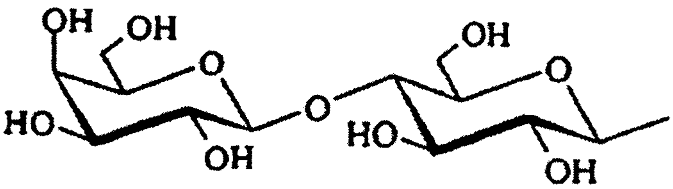

(III-a)

- der Gruppe, die von dem Trisaccharid Lewis-X oder dem Tetrasaccharid Sialyl-Lewis-X abgeleitet ist, mit der folgenden Formel (III-b) bzw. (III-c):

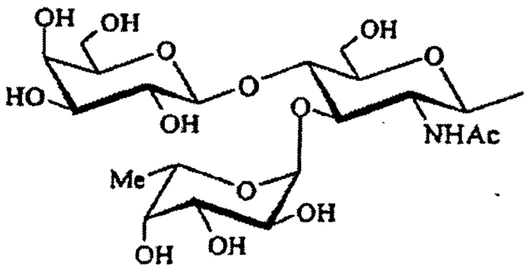

(III-b)

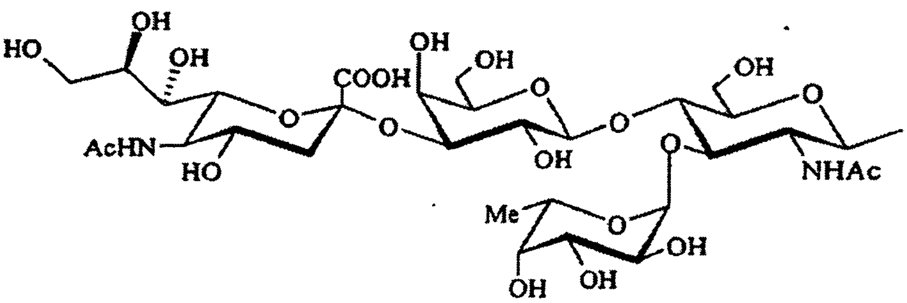

(III-c)

- einem Oligosaccharid, das von Heparin abgeleitet ist, mit der folgenden Formel (III-d):

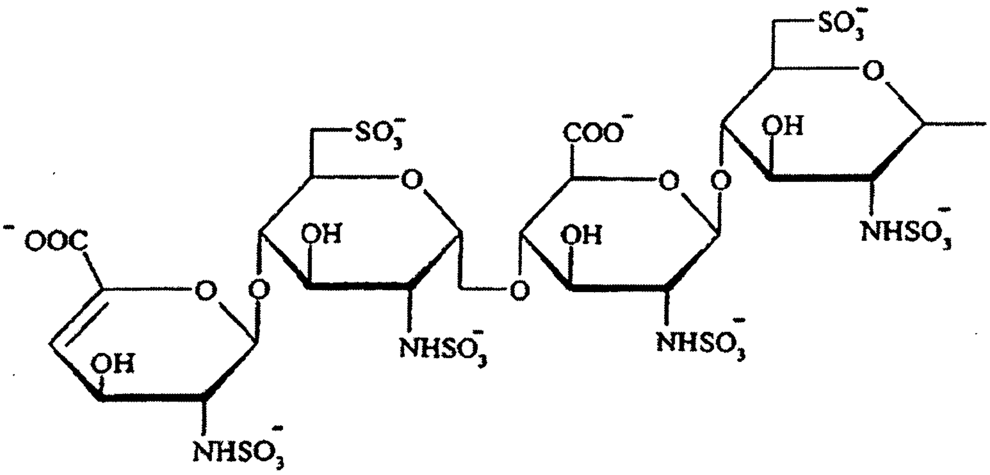

(III-d)

**14.** Verbindung nach Anspruch 10, **dadurch gekennzeichnet, dass**:

R ein Verzweigungselement umfasst, das von Tris(2-hydroxymethyl)methylamin abgeleitet ist, oder R für eine der folgenden Gruppen steht:

- der Tris-α-D-mannopyranosyloxymethyl)methylgruppe mit der folgenden Formel (IV):

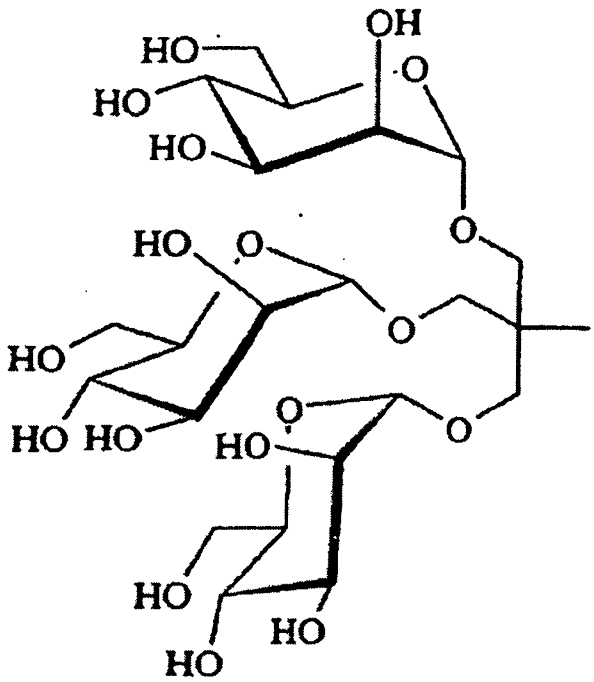

(IV)

- der Tris(β-lactosyloxymethyl)methylgruppe mit der folgenden Formel (IV-a):

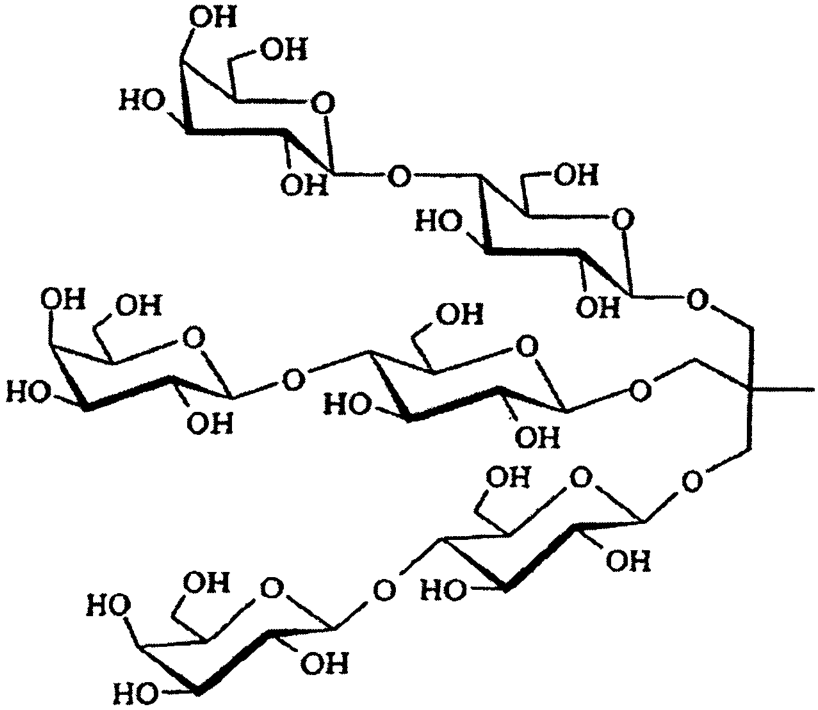

**(IV-a)**

**15.** Verbindung nach Anspruch 4, **dadurch gekennzeichnet, dass** R ein Verzweigungselement umfasst, das von Pentaerythritol abgeleitet ist, wobei die Verbindung der folgenden Formel entspricht:

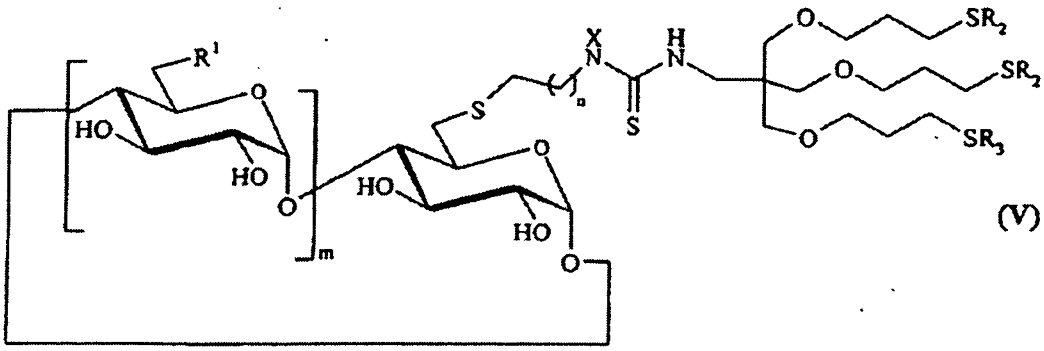

**(V)**

worin m, n und $R^1$ und X so sind, wie in Anspruch 1 definiert, und
$R^2$ und $R^3$ für Kohlenhydratderivate, die verschieden oder gleich sein können, oder auch eine fluoreszierende oder radioaktive Sonde stehen.

**16.** Verbindung nach Anspruch 15, **dadurch gekennzeichnet, dass** $R^1$ für OH steht.

**17.** Verbindung nach Anspruch 15, **dadurch gekennzeichnet, dass** $R^1$ für die Gruppe mit der folgenden Formel steht:

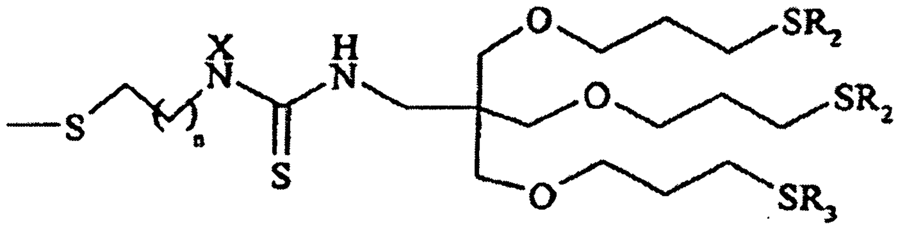

**18.** Verbindung nach irgendeinem der Ansprüche 15 bis 17, **dadurch gekennzeichnet, dass** n gleich 1 ist, dadurch, dass X für ein Wasserstoffatom steht und dadurch, dass $R^2$ und $R^3$ für eine der folgenden Gruppen stehen:

- die $\alpha$-D-Mannopyranosylgruppe mit der Formel (III), wie in Anspruch 12 definiert, oder
- die $\beta$-Lactosylgruppe mit der Formel (III-a), wie in Anspruch 13 definiert, oder
- die $\beta$-D-Glucopyranosylgruppe mit der folgenden Formel (VI):

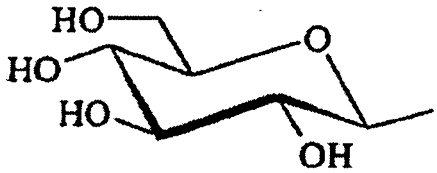

(VI)

wobei $R^2$ und $R^3$ gleich oder verschieden sein können.

**19.** Verbindung nach irgendeinem der Ansprüche 1 bis 18, **dadurch gekennzeichnet, dass** m gleich 6 ist.

**20.** Einschlusskomplex einer Verbindung nach irgendeinem der Ansprüche 1 bis 19 mit einem pharmakologisch aktiven Molekül, wobei das Molverhältnis zwischen der Verbindung nach einem der Ansprüche 1 bis 19 und dem pharmakologisch aktiven Molekül vorteilhafterweise etwa 50:1 bis etwa 1:1 beträgt.

**21.** Komplex nach Anspruch 20, **dadurch gekennzeichnet, dass** das pharmakologisch aktive Molekül ein antitumorales Mittel ist, das insbesondere zur Taxolfamilie gehört.

**22.** Pharmazeutische Zusammensetzung, die eine Verbindung nach einem der Ansprüche 1 bis 19 oder einen Einschlusskomplex nach Anspruch 20 oder 21 mit einem pharmakologisch verträglichen Vehikel umfasst.

**23.** Pharmazeutische Zusammensetzung nach Anspruch 22 in Form einer wässrigen Lösung.

**24.** Pharmazeutische Zusammensetzung nach irgendeinem der Ansprüche 22 oder 23, **dadurch gekennzeichnet, dass** sie als Dosiseinheit etwa 50 mg bis etwa 500 mg einer der Verbindungen nach einem der Ansprüche 1 bis 19 enthält, oder dadurch, dass sie als Dosiseinheit etwa 100 mg bis etwa 750 mg einer der Komplexe nach einem der Ansprüche 20 oder 21 enthält.

**25.** Verfahren zur Herstellung einer Verbindung mit der Formel (I) nach Anspruch 1, das die folgenden Schritte umfasst:

- das Umsetzen einer Verbindung, die an der Primäralkoholposition selektiv oder vollständig halogeniert ist, mit der folgenden Formel (VII):

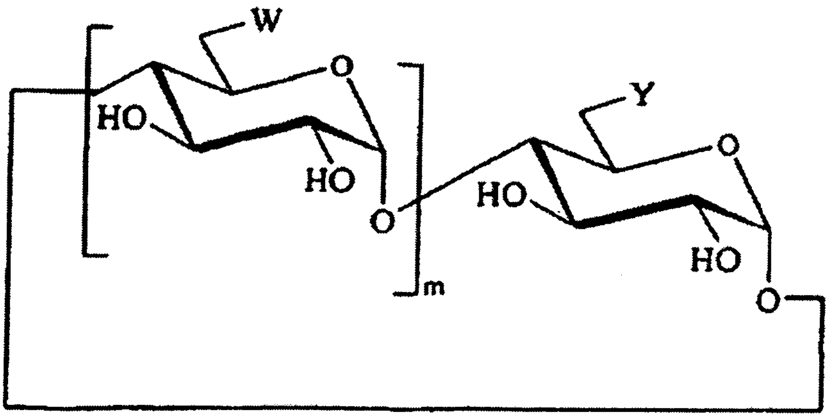

(VII)

wobei m so ist, wie in Anspruch 1 in der Formel (I) definiert.
W für eine OH-Gruppe oder eine Y-Gruppe steht, wobei die Gruppen W alle gleich sind,
und Y für ein Halogenatom steht, das aus der Gruppe bestehend aus Chlor, Brom, Iod ausgewählt ist und das bevorzugt Brom oder Iod ist,
mit einem ω-Aminoalkanthiol mit der folgenden Formel (VIII):

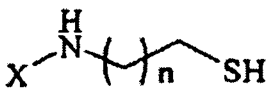

(VIII)

wobei das ω-Aminoalkanthiol gegebenenfalls N-alkyliert ist,
oder dem entsprechenden Salz der folgenden Formel (VIII-a):

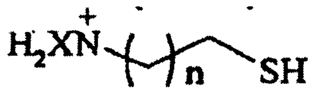

(VIII-a)

oder einem Tetraalkylammoniumsalz der folgenden Formel (VIII-b):

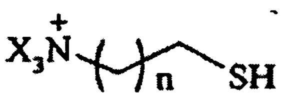

(VIII-b)

wobei das Salz mit einem Halogenid-Gegenion, bevorzugt dem Chloridion, assoziiert ist,
n und X so sind, wie oben definiert, und X bevorzugt ein Wasserstoffatom ist,
wobei die Verbindung mit der Formel (VIII) bevorzugt das Cysteamin mit der Formel $H_2N\text{-}CH_2\text{-}CH_2\text{-}SH$ ist,
um eine Verbindung mit der Formel (I), wie in Anspruch 1 definiert, zu erhalten, die den folgenden Formeln (A-a) oder (A-b) entspricht:

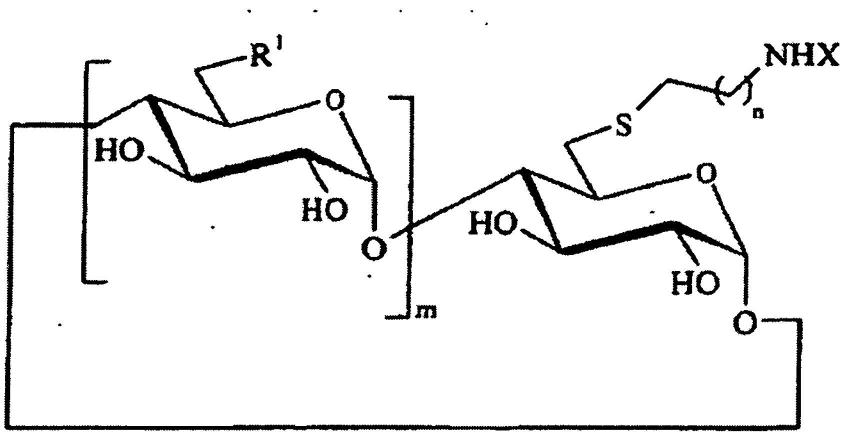

(A-a)

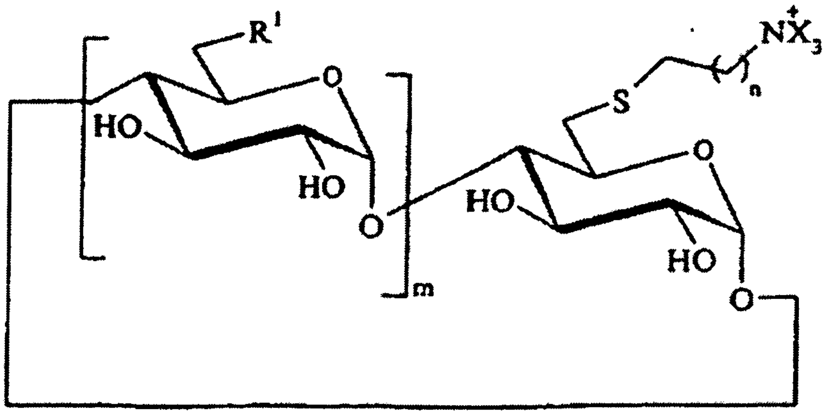

(A-b)

und gegebenenfalls

- das Umsetzen der Verbindung mit der Formel (A-a), wie im vorhergehenden Schritt erhalten, mit einem Isothiocyanat mit der folgenden Formel (IX):

R-N=C=S (IX)

worin R so ist, wie in Anspruch 1 definiert.
um eine Verbindung mit der Formel (I), wie oben definiert, zu erhalten, die der folgenden Formel entspricht:

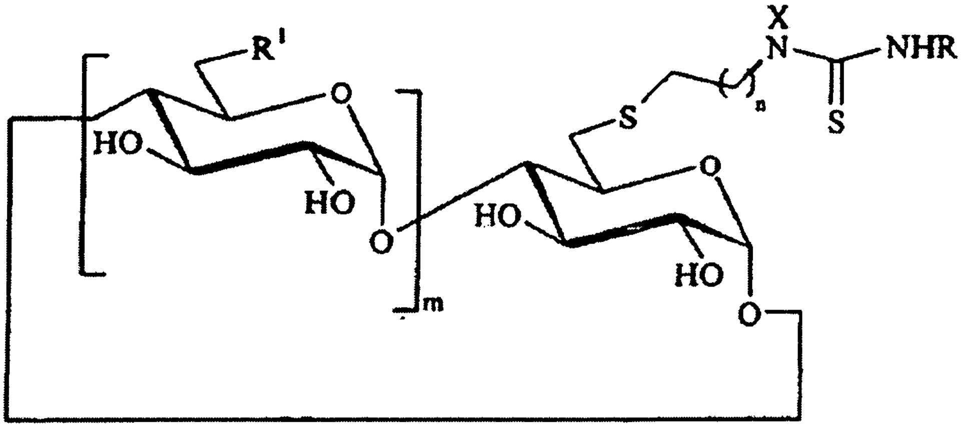

(B)

**26.** Verfahren zur Herstellung nach Anspruch 25 einer Verbindung, die der folgenden allgemeinen Formel (I-b) entspricht:

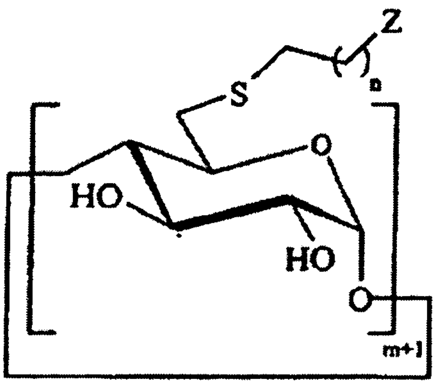

(I-b)

wobei das Verfahren **dadurch gekennzeichnet, dass** es die folgenden Schritte umfasst:

- das Umsetzen einer Per (6- desoxy- 6- halo) cyclodextrinverbindung mit der folgenden Formel (VII- a):

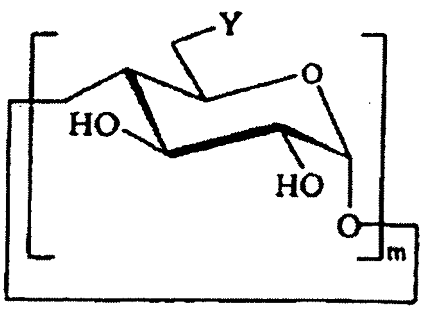

(VII-a)

wobei m und Y so sind, wie in Anspruch 25 definiert,
mit einem ω-Aminoalkanthiol mit der folgenden Formel (VIII):

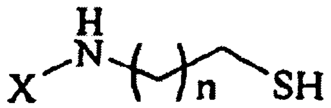

**(VIII)**

wobei das ω-Aminoalkanthiol gegebenenfalls N-alkyliert ist, oder dem entsprechenden Salz der folgenden Formel (VIII-a):

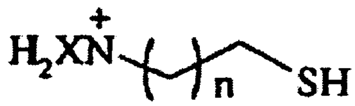

**(VIII-a)**

oder einem Tetraalkylammoniumsalz der folgenden Formel (VIII-b):

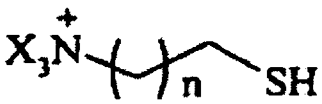

**(VIII-b)**

wobei das Salz mit einem Halogenid-Gegenion, bevorzugt dem Chloridion assoziiert ist, wobei n und X so sind, wie in Anspruch 1 definiert, und X bevorzugt ein Wasserstoffatom ist,
wobei die Verbindung mit der Formel (VIII) bevorzugt das Cysteamin mit der Formel $H_2N-CH_2-CH_2-SH$ ist,
um eine Verbindung mit den folgenden Formeln (I-c), (I-d) oder (I-e) zu erhalten:

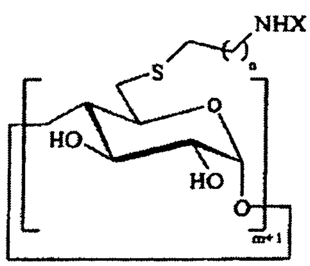

**(I-c)**

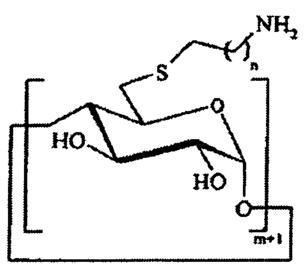

**(I-d)**

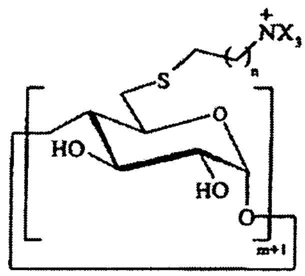

**(I-e)**

und gegebenenfalls

- das Umsetzen der Verbindung mit der Formel (I-c), wie im vorhergehenden Schritt erhalten, mit einem Isothiocyanat mit der folgenden Formel (IX):

R-N=C=S (IX)

worin R so ist, wie in Anspruch 1 definiert.
um eine Verbindung mit der folgenden Formel (II) oder (II-a) zu erhalten:

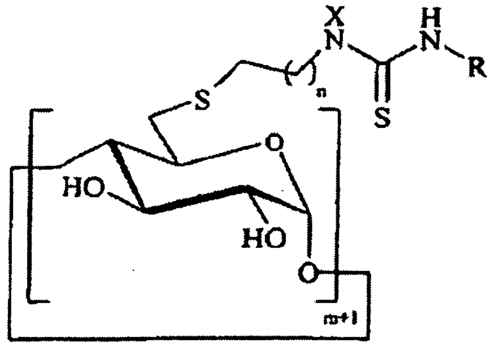

(II)

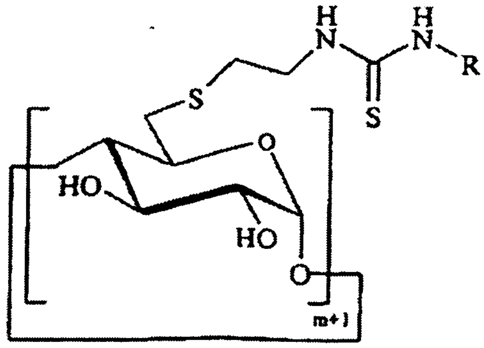

(II-a)

**27.** Verfahren zur Herstellung nach Anspruch 25 von Verbindungen, die der folgenden Formel entsprechen:

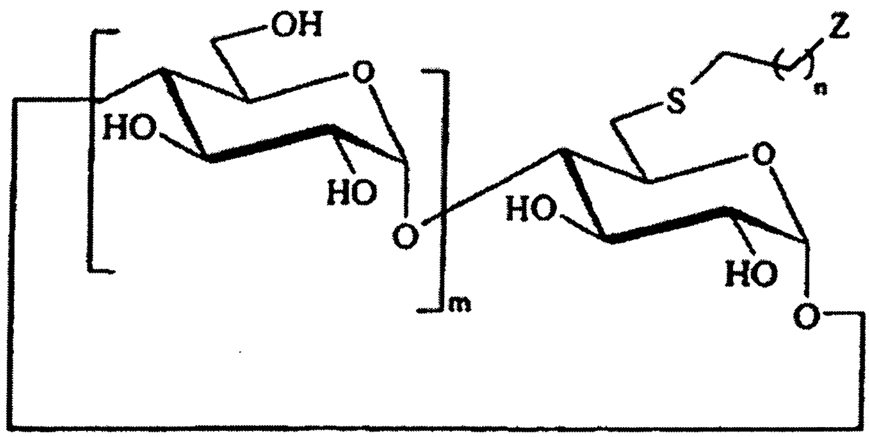

(I-a)

wobei das Verfahren **dadurch gekennzeichnet, dass** es die folgenden Schritte umfasst:

- das Umsetzen einer Verbindung, die an der Primäralkoholposition selektiv halogeniert ist, mit der folgenden Formel (VII):

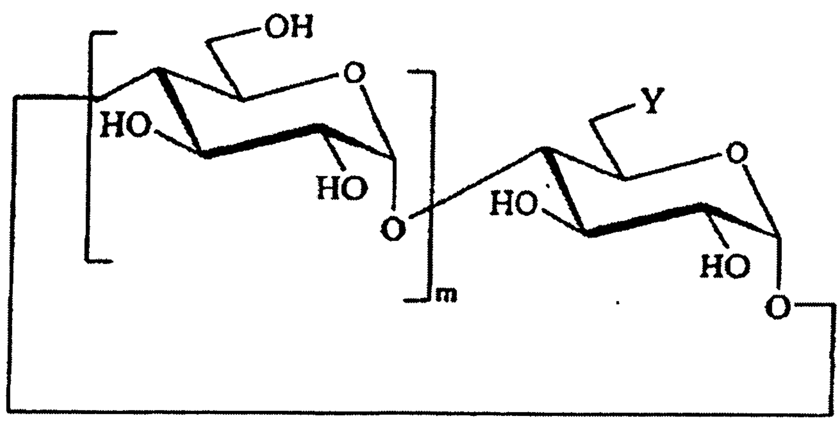

(VII)

wobei m und Y so sind, wie in Anspruch 25 definiert,
mit einem ω-Aminoalkanthiol mit der folgenden Formel (VIII):

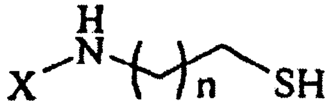

(VIII)

wobei das ω-Aminoalkanthiol gegebenenfalls N-alkyliert ist, oder dem entsprechenden Salz der folgenden Formel (VIII-a):

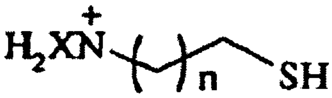

(VIII-a)

oder einem Tetraalkylammoniumsalz der folgenden Formel (VIII-b):

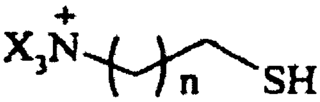

(VIII-b)

wobei das Salz mit einem Halogenid-Gegenion, bevorzugt dem Chloridion, assoziiert ist, n und X so sind, wie in Anspruch 1 definiert, und X bevorzugt ein Wasserstoffatom ist, wobei die Verbindung mit der Formel (VIII) bevorzugt das Cysteamin mit der Formel $H_2N-CH_2-CH_2-SH$ ist, um eine Verbindung mit den folgenden Formel (I-f) oder (I-g) zu erhalten:

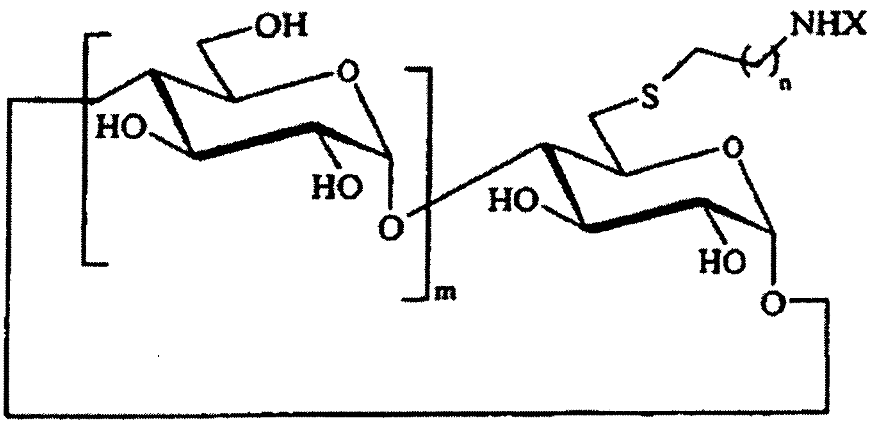

(I-f)

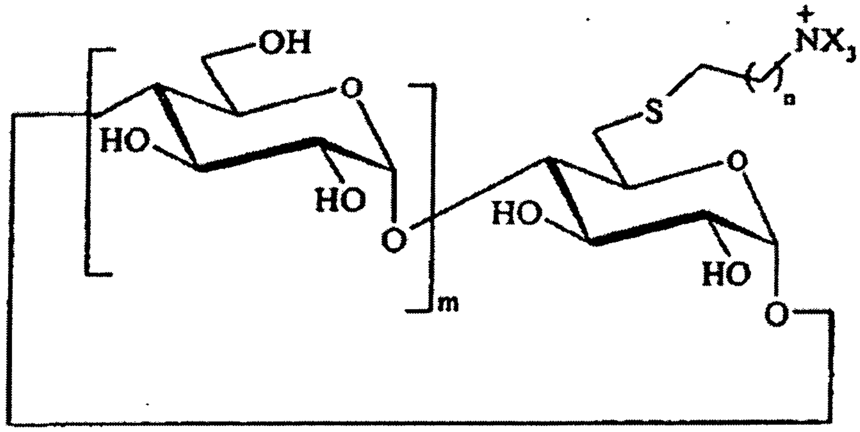

(I-g)

worin m, n und X so sind, wie in Anspruch 1 definiert, und gegebenenfalls

- das Umsetzen der Verbindung mit der Formel (I-f), wie im vorhergehenden Schritt erhalten, mit einem Isothiocyanat mit der folgenden Formel (IX):

R-N=C=S (IX)

worin R so ist, wie in Anspruch 1 definiert. um eine Verbindung mit der folgenden Formel (I-h) zu erhalten:

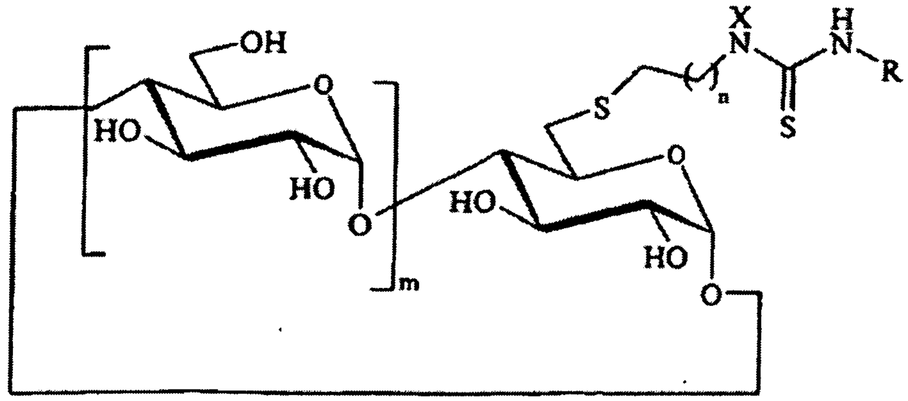

(I-h)

**28.** Verfahren zur Herstellung einer Verbindung mit der Formel (I-f-bis)

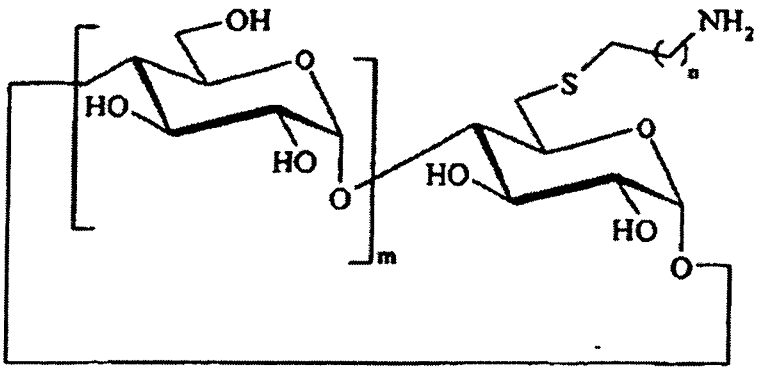

(I-f-bis)

worin m und n so sind, wie in Anspruch 1 definiert, wobei n bevorzugt gleich 1 ist, wobei das Verfahren **dadurch gekennzeichnet ist, dass** es das Umsetzen einer Verbindung, die an der Primäralkoholposition selektiv halogeniert ist, mit der folgenden Formel (VII):

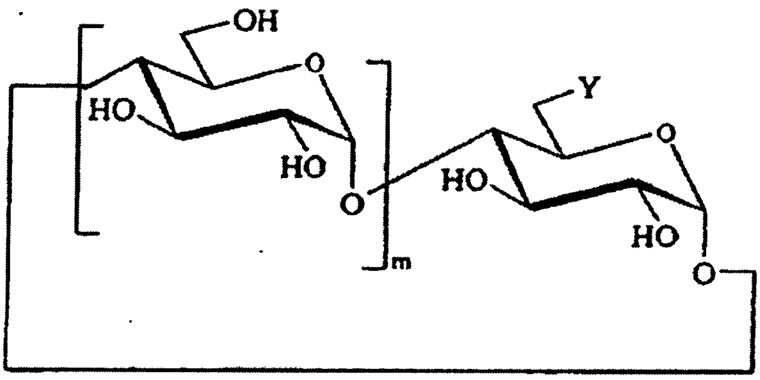

(VII)

wobei m so ist, wie oben definiert, und
und Y für ein Halogenatom steht, das aus der Gruppe bestehend aus Chlor, Brom, Iod ausgewählt ist und das bevorzugt Brom oder Iod ist,
mit einem ω-Aminoalkanthiol mit der folgenden Formel umfasst:

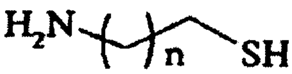

wobei n so ist, wie oben definiert,
oder bevorzugt das Cysteamin mit der Formel $H_2N-CH_2-CH_2-SH$ ist.

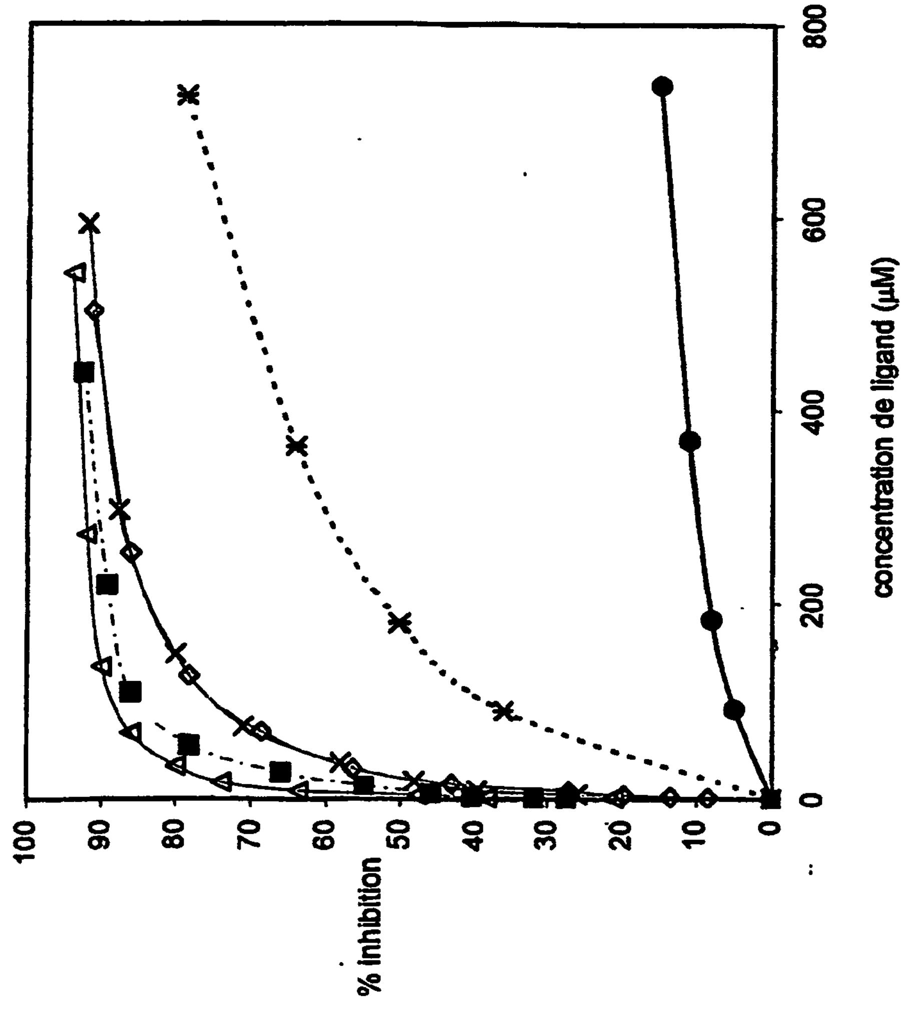
% inhibition
100
90
80
70
60
50
40
30
20
10
0
0
200
400
600
800
concentration de ligand (µM)

FIGURE 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9519994 A
- WO 9521870 A
- WO 9733919 A
- EP 0403366 A
- EP 0605753 A
- WO 9961483 A


### Littérature non-brevet citée dans la description


- **P. Potier.** *Chem. Soc. Rev.,* 1992, vol. 21, 113-119
- **V. Lainé et al.** *J. Chem. Soc., Perkin Trans.,* 1995, vol. 2, 1479-1487
- **I. Baussanne et al.** *Chem. Commun,* 2000, 1489-1490
- **I. Baussanne et al.** *ChemBioChem,* 2001, 777-783
- **Y. Nagata et al.** *Bull Chem. Soc. Jpn.,* 1994, vol. 67, 495-499
- **B. Ekberg et al.** *Carbohydr. Res.,* 1992, vol. 192, 111-117
- **J. Defaye.** *J. Incl. Phen. Mol. Recogn. Chem.,* 1997, vol. 29, 57-63
- *ChemBioChem,* 2001
- **J. Defaye.** *Supramol. Chem,* 2000, vol. 12, 221-224
- *Polish J. Chem.,* 1999, vol. 73, 967-971
- *Tetrahedron Lett.,* 1997, vol. 38, 7365-7368
- *Carbohydr. Res.,* 1992, vol. 228, 307-314
- *Angew. Chem., Int. Ed. Engl.,* 1991, vol. 30, 78-80
- **L. Jicsinszky.** Comprehensive Supramolecular Chemistry. Pergamon, 1996, vol. 3, 57-198
- **L. Jicsinszky.** Comprehensive Supramolecular Chemistry. Pergarnon, 1996, vol. 3, 57-198
- **J. M. Garcia Fernandez ; C. Ortiz Mellet.** *Adv. Carbohydr. Chem. Biochem.,* 1999, vol. 55, 35-135
- *Chem. Commun.,* 2000, 1489-1490
- **P. R. Ashton et al.** *J. Org. Chem.,* 1998, vol. 63, 3429-3437
- **D. A. Fulton ; J. F. Stoddart.** *Org. Lett.,* 2000, vol. 2, 1113-1116
- **X.-B. Meng et al.** *Carbohydr. Res.,* 2002, vol. 337, 977-981
- **J. Defaye ; J. Gelas.** Studies in Natural Products Chemistry. Elsevier, 1991, vol. 8, 315-357
- **H. Driguez.** *Top. Curr. Chem.,* 1997, vol. 187, 85-116
- **J. J. Lundquist ; E. J. Toon.** *Chem. Rev.,* 2002, vol. 102, 555-578